(19) Europäisches Patentamt
European Patent Office
Office européen des brevets

(11) EP 3 545 058 B1

(12) FASCICULE DE BREVET EUROPEEN

(45) Date de publication et mention de la délivrance du brevet:
**13.03.2024 Bulletin 2024/11**

(21) Numéro de dépôt: **17812011.9**

(22) Date de dépôt: **21.11.2017**

(51) Classification Internationale des Brevets (IPC):
***C10M 145/14*** *(2006.01)* ***C08L 43/00*** *(2006.01)*
***C10M 161/00*** *(2006.01)* ***C10M 159/00*** *(2006.01)*
***C10M 157/00*** *(2006.01)* ***C10M 155/00*** *(2006.01)*
***C08F 230/06*** *(2006.01)* ***C08F 220/20*** *(2006.01)*
***C10N 20/04*** *(2006.01)* ***C10N 30/02*** *(2006.01)*
***C10N 30/00*** *(2006.01)* ***C10N 70/00*** *(2006.01)*
***C08F 220/18*** *(2006.01)*

(52) Classification Coopérative des Brevets (CPC):
(C-Sets disponibles)
**C10M 145/14; C08F 220/1812; C08F 230/06; C08L 43/00; C10M 155/00; C10M 159/005; C10M 161/00;** C08F 220/1818; C08K 2201/014; C10M 2203/1025; C10M 2205/04; C10M 2227/061; C10M 2227/062; C10M 2229/00; C10N 2020/04;

(Cont.)

(86) Numéro de dépôt international:
**PCT/FR2017/053189**

(87) Numéro de publication internationale:
**WO 2018/096253 (31.05.2018 Gazette 2018/22)**

(54) **COMPOSITIONS D'ADDITIFS THERMOASSOCIATIFS DONT L'ASSOCIATION EST CONTROLEE ET COMPOSITIONS LUBRIFIANTES LES CONTENANT**

THERMOASSOZIATIVE ADDITIVZUSAMMENSETZUNGEN MIT GESTEUERTER ASSOZIATION UND SCHMIERMITTELZUSAMMENSETZUNGEN DAMIT

THERMOASSOCIATIVE ADDITIVE COMPOSITIONS, THE ASSOCIATION OF WHICH IS CONTROLLED, AND LUBRICATING COMPOSITIONS CONTAINING SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats de validation désignés:
**MA**

(30) Priorité: **23.11.2016 FR 1661403**

(43) Date de publication de la demande:
**02.10.2019 Bulletin 2019/40**

(73) Titulaires:
- **TotalEnergies OneTech 92400 Courbevoie (FR)**
- **Centre National de la Recherche Scientifique 75016 Paris (FR)**
- **Ecole Supérieure de Physique et de Chimie Industrielles de la Ville de Paris 75005 Paris (FR)**

(72) Inventeurs:
- **DEROUINEAU, Thibault 72440 Coudrecieux (FR)**
- **BRIAND, Fanny 69003 Lyon (FR)**
- **DESCROIX, Grégory 69126 Brindas (FR)**
- **NICOLAY, Renaud 91370 Verrières le Buisson (FR)**

(74) Mandataire: **Pact-IP 37, rue Royale 92210 Saint-Cloud (FR)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

(56) Documents cités:
**WO-A1-2016/113229**

(52) Classification Coopérative des Brevets (CPC): (Cont.)
C10N 2030/02; C10N 2030/68; C10N 2070/02

C-Sets
**C08K 5/55, C08L 33/066;**
**C08K 5/55, C08L 33/14;**
C08F 220/1818, C08F 220/1812, C08F 212/08, C08F 220/20;
C10M 2203/1025, C10N 2020/02;
C10M 2205/04, C10M 2209/084, C10M 2209/084

## Description

La présente invention concerne de nouvelles compositions d'additifs qui résultent du mélange d'au moins deux composés thermoassociatifs et échangeables, dont au moins un copolymère, et d'au moins un composé ester boronique permettant de contrôler l'association de ces deux composés thermoassociatifs.

L'invention concerne également une composition lubrifiante qui résulte du mélange d'au moins une huile de base lubrifiante, d'au moins deux composés thermoassociatifs et échangeables, dont au moins un copolymère, et d'au moins un composé ester boronique permettant de contrôler l'association de ces deux copolymères.

La présente invention concerne également un procédé pour moduler la viscosité d'une composition lubrifiante qui résulte du mélange d'au moins une huile de base lubrifiante, d'au moins deux composés thermoassociatifs et échangeables, dont au moins un copolymère ; ainsi que l'utilisation d'un composé ester boronique pour moduler la viscosité d'une composition lubrifiante.

## Etat de la technique antérieure

Les polymères de hautes masses molaires sont largement utilisés pour augmenter la viscosité de solutions dans de nombreux domaines, tels que l'industrie du pétrole, du papier, du traitement de l'eau, l'industrie minière, des cosmétiques, du textile et de manière générale dans toutes les techniques industrielles utilisant des solutions épaissies.

Or, ces polymères de hautes masses molaires présentent l'inconvénient de présenter une dégradation irréversible importante sous contrainte mécanique par rapport aux mêmes polymères de tailles plus faibles. Ces contraintes de cisaillement sur les polymères de hautes masses molaires entraînent des coupures au niveau des chaînes macromoléculaires. Le polymère ainsi dégradé voit ses propriétés épaississantes diminuer ou disparaître, et la viscosité des solutions le contenant chute de manière irréversible. Cette perte de résistance au cisaillement conduit à une dégradation des propriétés des solutions à base de polymères de hautes masses molaires.

On connaît par les demandes WO2015/110642, WO2015/110643 et WO2016113229 une composition résultant du mélange d'au moins un copolymère A1 résultant de la copolymérisation d'au moins un monomère fonctionnalisé par des fonctions diols et d'au moins un composé A2 comprenant au moins deux fonctions esters boroniques. Ces composés peuvent s'associer, pour former éventuellement un gel, et échanger des liens chimiques de manière thermoréversible. Ces additifs présentent l'avantage d'augmenter la viscosité de la solution les comprenant lorsque la température augmente. Ces compositions de polymères présentent des propriétés rhéologiques très variées selon la proportion des composés A1 et A2 utilisée. Elles peuvent en outre comprendre un composé diol qui permet de mieux contrôler l'association des deux copolymères.

En particulier, ces compositions de polymères peuvent être ajoutées dans une huile lubrifiante pour lubrifier une pièce mécanique. Ces copolymères permettent de formuler des compositions lubrifiantes dont la viscosité est mieux contrôlée par rapport aux compositions lubrifiantes de l'art antérieur. En particulier, ces copolymères, lorsqu'ils sont introduits dans une huile de base, tendent à faire diminuer la chute de viscosité du mélange lorsque la température augmente. La présence d'un composé diol dans ces compositions lubrifiantes permet de mieux moduler leur viscosité.

Les compositions lubrifiantes sont des compositions appliquées entre les surfaces, notamment métalliques, de pièces mobiles. Elles permettent de réduire le frottement et l'usure entre deux pièces en contact et en mouvement l'une par rapport à l'autre. Elles servent également à dissiper une partie de l'énergie thermique engendrée par ce frottement. Les compositions lubrifiantes forment un film protecteur entre les surfaces des pièces sur lesquelles elles sont appliquées.

Les compositions utilisées pour la lubrification de pièces mécaniques sont généralement constituées d'une huile de base et d'additifs. L'huile de base, notamment d'origine pétrolière ou synthétique, présente des variations de viscosité lorsqu'on fait varier la température.

En effet, lorsque la température d'une huile de base augmente, sa viscosité diminue et lorsque la température de l'huile de base diminue, sa viscosité augmente. Or, en régime de lubrification hydrodynamique, l'épaisseur du film protecteur est proportionnelle à la viscosité, donc dépend aussi de la température. Une composition présente de bonnes propriétés lubrifiantes si l'épaisseur du film protecteur reste sensiblement constante quelles que soient les conditions et la durée d'utilisation du lubrifiant.

Dans un moteur à combustion interne, une composition lubrifiante peut être soumise à des changements de température externes ou internes. Les changements de température externes sont dus aux variations de température de l'air ambiant, telles que les variations de température entre l'été et l'hiver par exemple. Les changements de température internes résultent de la mise en oeuvre du moteur. La température d'un moteur est plus basse lors de sa phase de démarrage, notamment par temps froid, que lors d'une utilisation prolongée. Par conséquent, l'épaisseur du film protecteur peut varier dans ces différentes situations.

Il existe donc un besoin de disposer d'une composition lubrifiante possédant de bonnes propriétés de lubrification et dont la viscosité est peu sujette aux variations de température.

Il est connu d'ajouter des additifs améliorant la viscosité d'une composition lubrifiante. Ces additifs ont pour fonction de modifier le comportement rhéologique de la composition lubrifiante. Ils permettent de favoriser une plus grande stabilité de la viscosité sur une plage de température à laquelle la composition lubrifiante est utilisée. Par exemple, ces additifs limitent la diminution de la viscosité de la composition lubrifiante lorsque la température s'élève, tout en limitant l'augmentation de la viscosité à froid.

Les additifs améliorant la viscosité (ou additifs améliorant l'indice de viscosité) permettent de garantir une bonne lubrification en limitant l'impact sur la viscosité à froid et en garantissant une épaisseur minimale de film à chaud. Les additifs améliorant la viscosité. Les additifs améliorant la viscosité actuellement utilisés sont des polymères tels que les oléfines copolymères (OCP), et les polyméthacrylates d'alkyles (PMA). Ces polymères sont de hautes masses molaires. En général, la contribution de ces polymères au contrôle de la viscosité est d'autant plus importante que leur poids moléculaire est élevé.

Cependant, les polymères de hautes masses molaires présentent l'inconvénient d'avoir une faible résistance au cisaillement permanent par rapport aux polymères de même nature mais de taille plus faible.

Or, une composition lubrifiante est soumise à des contraintes de cisaillement importantes notamment dans les moteurs à combustion interne, où les surfaces en frottement ont un écartement très faible et les pressions exercées sur les pièces sont élevées. Ces contraintes de cisaillement sur les polymères de hautes masses molaires entraînent des coupures au niveau des chaînes macromoléculaires. Le polymère ainsi dégradé voit ses propriétés épaississantes réduites, et la viscosité chute de manière irréversible. Cette perte de résistance au cisaillement permanent conduit donc à une dégradation des propriétés de lubrification de la composition lubrifiante.

Les compositions décrites dans les demandes WO2015/110642, WO2015/110643 et WO2016113229 présentent des propriétés très intéressantes, du fait de leur capacité à former des associations thermoréversibles, et également du fait de la possibilité de moduler leur viscosité par addition d'un composé diol.

La demanderesse a maintenant découvert que certains composés, les esters boroniques comprenant une seule fonction ester boronique, désignés A5 dans la suite de la description, présentent aussi la propriété de contrôler l'association d'un copolymère A1 résultant de la copolymérisation d'au moins un monomère fonctionnalisé par des fonctions diols et d'au moins un composé A2 comprenant au moins deux fonctions esters boroniques. Les fonctions ester boronique du composé A5 entrent en compétition avec les fonctions ester boronique du composé A2 comprenant au moins deux fonctions esters boroniques. Ainsi, en fonction de la température et des proportions de A1, A2 et A5, des réactions de transestérification réversibles se produisent entre les fonctions diol et les fonctions ester boronique. Ces réactions conduisent à des produits de structure différentes, à nombre de liaisons ester constant dans le mélange. Il est possible de moduler la cinétique et la fenêtre de température de formation de ces associations, donc de moduler le comportement rhéologique de la composition lubrifiante en fonction de l'utilisation désirée.

Il est possible, grâce aux compositions de l'invention de fournir des compositions lubrifiantes qui possèdent de bonnes propriétés de lubrification lors des phases de démarrage d'un moteur (phase à froid) et des bonnes propriétés de lubrification lorsque le moteur fonctionne à sa température de service (phase à chaud).

**Résumé de l'invention**

L'invention concerne une composition résultant du mélange d'au moins :

- un copolymère statistique polydiol A1,
- un composé A2 comprenant au moins deux fonctions esters boroniques,
- un composé exogène A5 choisi parmi ceux répondant à la formule (XI) :

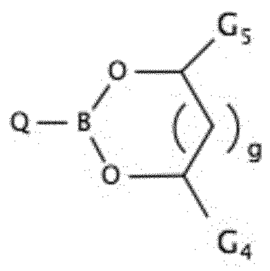

(XI)

dans laquelle :

- Q représente un groupement choisi parmi un groupement hydrocarboné comprenant de 1 à 30 atomes de carbone, éventuellement substituée par un ou plusieurs groupements choisis parmi : un hydroxyle, un groupement -OJ,

-C(O)-O-J avec J un groupement hydrocarboné comprenant de 1 à 24 atomes de carbone,

- $G_4$, $G_5$, identiques ou différents, représentent des groupements choisis parmi un atome d'hydrogène, une chaîne hydrocarbonée comprenant de 1 à 24 atomes de carbone, un hydroxyle, un groupement -OJ, -C(O)-O-J avec J un groupement hydrocarboné comprenant de 1 à 24 atomes de carbone,
- g représente 0 ou 1,

le copolymère statistique polydiol A1 ayant une masse molaire moyenne en nombre allant de 5 000 à 400 000 g/mol, le pourcentage molaire de composé exogène A5 par rapport aux fonctions diol du copolymère statistique A1 allant de 0,025 à 5000%, et le ratio massique entre le copolymère A1 et le composé A2 (ratio A1/A2) allant de 0,1 à 10.

Avantageusement le composé exogène A5 répond à la formule (XIA) :

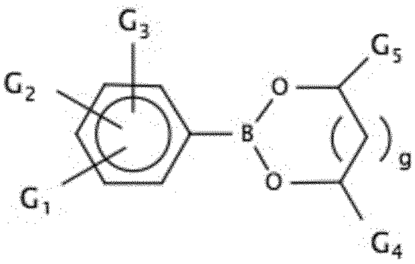

(XIA)

dans laquelle :

- $G_1$, $G_2$, $G_3$, $G_4$, $G_5$, identiques ou différents, représentent des groupements choisis parmi un atome d'hydrogène, une chaîne hydrocarbonée comprenant de 1 à 24 atomes de carbone, un hydroxyle, un groupement -OJ, -C(O)-O-J avec J un groupement hydrocarboné comprenant de 1 à 24 atomes de carbone,
- g représente 0 ou 1.

Selon un mode de réalisation préféré, le pourcentage molaire de composé exogène A5 par rapport aux fonctions diol du copolymère statistique A1 va de 0,1% à 1000%, de manière encore plus préférée de 0,5% à 500%, de manière encore plus préférée de 1% à 150%.

Selon un mode de réalisation préféré, le composé exogène A5 est choisi parmi ceux répondant à la formule (XI B) :

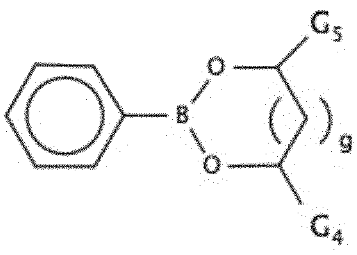

(XI B)

Selon un mode de réalisation encore préféré, le composé exogène A5 est choisi parmi ceux répondant à la formule (XI B) avec g = 0, $G_4$ = H et $G_5$ représente un alkyle en C1-C24.

Selon un mode de réalisation préféré, le copolymère statistique A1 résulte, directement ou indirectement, de la copolymérisation :

- d'au moins un premier monomère M1 de formule générale (I) :

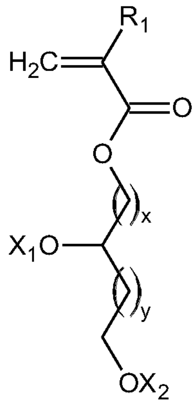

(I)

dans laquelle :

- $R_1$ est choisi parmi le groupe formé par -H, $-CH_3$, et $-CH_2-CH_3$ ;
- x est un nombre entier allant de 1 à 18, de préférence de 2 à 18 ;
- y est un nombre entier égal à 0 ou 1;
- $X_1$ et $X_2$, identiques ou différents, sont choisis parmi le groupe formé par l'hydrogène, le tétrahydropyranyle, le méthyloxyméthyle, le ter-butyle, le benzyle, le triméthylsilyle et le t-butyle diméthylsilyle ;
  ou bien
- $X_1$ et $X_2$ forment avec les atomes d'oxygène un pont de formule suivante

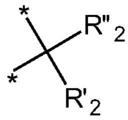

dans laquelle:

- les étoiles (*) symbolisent les liaisons aux atomes d'oxygène,
- $R'_2$ et $R''_2$, identiques ou différents, sont choisis parmi le groupe formé par l'hydrogène et un alkyle en $C_1$-$C_{11}$, de préférence le méthyle ;
  ou bien
- $X_1$ et $X_2$ forment avec les atomes d'oxygène un ester boronique de formule suivante :

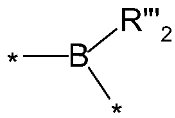

dans laquelle :

- les étoiles (*) symbolisent les liaisons aux atomes d'oxygène,
- $R'''_2$ est choisi parmi le groupe formé par un aryle en $C_6$-$C_{30}$, un aralkyle en $C_7$-$C_{30}$ et un alkyle en $C_2$-$C_{30}$, de préférence un aryle en $C_6$-$C_{18}$;

▪ avec au moins un second monomère M2 de formule générale (II) :

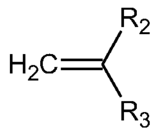

(II)

dans laquelle :

- $R_2$ est choisi parmi le groupe formé par -H, $-CH_3$ et $-CH_2-CH_3$,
- $R_3$ est choisi parmi le groupe formé par un aryle en $C_6$-$C_{18}$, un aryle en $C_6$-$C_{18}$ substitué par un groupement $R'_3$, -C(O)-O-$R'_3$,
- O-$R'_3$, -S-$R'_3$ et -C(O)-N(H)-$R'_3$ avec $R'_3$ un groupe alkyle en $C_1$-$C_{30}$.

Selon un mode de réalisation encore préféré, le copolymère statistique A1 résulte, directement ou indirectement, de la copolymérisation :

- d'au moins un premier monomère M1 de formule générale (I),
- avec au moins un second monomère M2 de formule générale (II) :

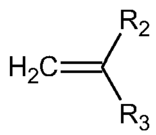

(II)

dans laquelle :

- $R_2$ est choisi parmi le groupe formé par -H, $-CH_3$ et $-CH_2-CH_3$,

- $R_3$ est choisi parmi le groupe formé par -C(O)-O-$R'_3$, -O-$R'_3$, -S-$R'_3$ et -C(O)-N(H)-$R'_3$ avec $R'_3$ un groupe alkyle en $C_1$-$C_{30}$,

et

- avec au moins un troisième monomère M3 de formule générale (X) :

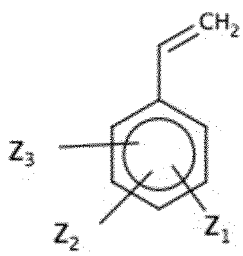

(X)

dans laquelle :

- $Z_1$, $Z_2$, $Z_3$, identiques ou différents, représentent des groupements choisis parmi un atome d'hydrogène, un alkyle en $C_1$-$C_{12}$, un groupement -OZ', -C(O)-O-Z' avec Z' un alkyle en $C_1$-$C_{12}$.

Selon un mode de réalisation encore préféré, le troisième monomère M3 est le styrène.

Selon un mode de réalisation encore préféré, le copolymère statistique A1 résulte, directement ou indirectement, de la copolymérisation d'au moins un monomère M1 avec au moins deux monomères M2 ayant des groupes $R_3$ différents et au moins un monomère M3.

Selon une première variante préférée, les deux monomères M2 du copolymère statistique A1 ont pour formule générale (II-B) :

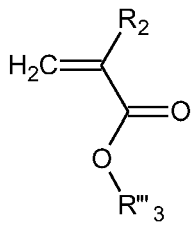

(II-B)

dans laquelle :

- $R_2$ est choisi parmi le groupe formé par -H, $-CH_3$ et-$CH_2$-$CH_3$,
- $R'''_3$ est un groupe alkyle en $C_9$-$C_{30}$.

Selon une autre variante préférée, l'un des monomères M2 du copolymère statistique A1 a pour formule générale (II-A) :

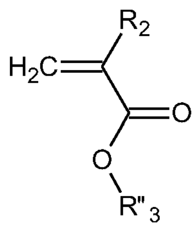

(II-A)

dans laquelle :

- $R_2$ est choisi parmi le groupe formé par -H, $-CH_3$ et-$CH_2$-$CH_3$,
- $R''_3$ est un groupe alkyle en $C_1$-$C_8$

et l'autre monomère M2 du copolymère statistique A1 a pour formule générale (II-B) :

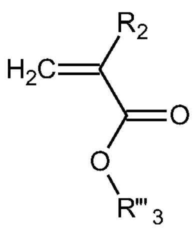

(II-B)

dans laquelle :

- $R_2$ est choisi parmi le groupe formé par -H, $-CH_3$ et-$CH_2$-$CH_3$,
- $R'''_3$ est un groupe alkyle en $C_9$-$C_{30}$.

Selon un mode de réalisation préféré, les chaînes latérales du copolymère statistique A1 ont une longueur moyenne allant de 8 à 20 atomes de carbone, de préférence de 9 à 18 atomes de carbone.

Selon un mode de réalisation préféré, le copolymère statistique A1 comprend un pourcentage molaire de monomère M3 de formule (X) dans ledit copolymère allant de 2 à 50%, de préférence de 3 à 40%, de manière plus préférée allant de 5 à 35%.

Selon un mode de réalisation préféré, le copolymère statistique A1 a un pourcentage molaire de monomère

M1 de formule (I) dans ledit copolymère allant de 1 à 30%, de préférence de 5 à 25%.

Selon un mode de réalisation préféré, le copolymère statistique A1 a un degré de polymérisation moyen en nombre allant de 40 à 2000, de préférence de 40 à 1000.

Selon un mode de réalisation préféré, le copolymère statistique A1 a un indice de polydispersité (Ip) allant de 1,05 à 4,0 ; de préférence allant de 1,10 à 3,8.

Selon un mode de réalisation préféré, le composé A2 est un composé de formule (III) :

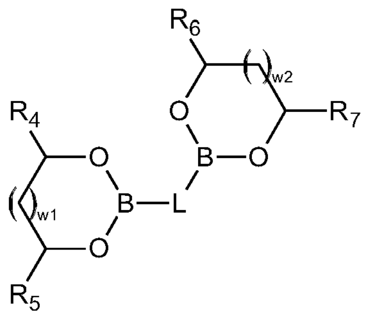

(III)

dans laquelle :

- $w_1$ et $w_2$, identiques ou différents sont des nombres entiers choisis entre 0 et 1 ;
- $R_4$, $R_5$, $R_6$ et $R_7$, identiques ou différents représentent un groupement choisi parmi un atome d'hydrogène, un groupement hydrocarboné comprenant de 1 à 30 atomes de carbone, de préférence entre 4 et 18 atomes de carbone, encore plus préférentiellement entre 6 et 14 atomes de carbone, ledit groupement hydrocarboné étant éventuellement substituée par un ou plusieurs groupements choisis parmi : un hydroxyle, un groupement -OJ, -C(O)-O-J avec J un groupement hydrocarboné comprenant de 1 à 24 atomes de carbone ;
- L est un groupement de liaison divalent et choisi parmi le groupe formé par un aryle en $C_6$-$C_{18}$, un aralkyle en $C_7$-$C_{24}$ et une chaîne hydrocarbonée en $C_2$-$C_{24}$.

Selon un mode de réalisation préféré, le composé A2 est un copolymère statistique résultant de la copolymérisation

- d'au moins un monomère M4 de formule (IV) :

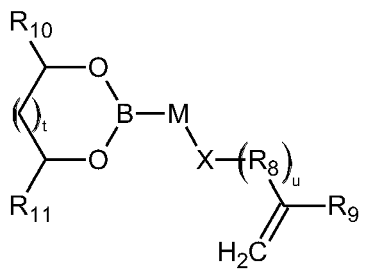

(IV)

dans laquelle :

- t est un nombre entier égal à 0 ou 1 ;
- u est un nombre entier égal à 0 ou 1 ;
- M et $R_8$ sont des groupements de liaison divalents, identiques ou différents, choisis parmi le groupe formé par un aryle en $C_6$-$C_{18}$, un aralkyle en $C_7$-$C_{24}$ et un alkyle en $C_2$-$C_{24}$, de préférence un aryle en $C_6$-$C_{18}$,
- X est une fonction choisie parmi le groupe formé par -O-C(O)-, - C(O)-O-, -C(O)-N(H)-, -N(H)-C(O)-, -S- , -N(H)- , -N(R'$_4$)- et -O- avec R'$_4$ une chaîne hydrocarbonée comprenant de 1 à 15 atomes de carbone;
- $R_9$ est choisi parmi le groupe formé par -H, $-CH_3$ et $-CH_2-CH_3$ ;
- $R_{10}$ et $R_{11}$ identiques ou différents représentent un groupement choisi parmi un atome d'hydrogène, un groupement hydrocarboné comprenant de 1 à 30 atomes de carbone, de préférence entre 4 et 18 atomes de carbone, encore plus préférentiellement entre 6 et 14 atomes de carbone, ledit groupement hydrocarboné étant éventuellement substituée par un ou plusieurs groupements choisis parmi : un hydroxyle, un groupement -OJ, -C(O)-O-J avec J un groupement hydrocarboné comprenant de 1 à 24 atomes de carbone ;

- avec au moins un second monomère M5 de formule générale (V) :

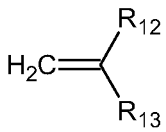

(V)

dans laquelle :

- $R_{12}$ est choisi parmi le groupe formé par -H, $-CH_3$ et $-CH_2-CH_3$,
- $R_{13}$ est choisi parmi le groupe formé par un aryle en $C_6$-$C_{18}$, un aryle en $C_6$-$C_{18}$ substitué par un groupement $R'_{13}$, -C(O)-O-$R'_{13}$ ; -O-$R'_{13}$, -S-$R'_{13}$ et -C(O)-N(H)-$R'_{13}$ avec $R'_{13}$ un groupe alkyle en $C_1$-$C_{30}$.

Selon un mode de réalisation préféré, l'une au moins des trois conditions suivantes est réalisée :

- soit dans la formule (IV) : u = 1, $R_9$ est H et $R_8$ représente un aryle en $C_6$-$C_{18}$ ou un aralkyle en $C_7$-$C_{24}$ et la double liaison du monomère M4 de formule (IV) est directement connectée au groupement aryle ;
- soit dans la formule (V) : $R_{12}$ représente H et $R_{13}$ est choisi parmi le groupe formé par un aryle en $C_6$-$C_{18}$ et un aryle en $C_6$-$C_{18}$ substitué par un groupement $R'_{13}$ avec $R'_{13}$ un groupe alkyle en $C_1$-$C_{25}$ et la double liaison du monomère M5 de formule (V) est directement connectée au groupement aryle ;
- soit le copolymère A2 comprend au moins un troisième monomère M3 de formule (X)

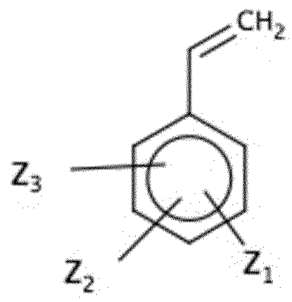

(X)

dans laquelle :

- $Z_1$, $Z_2$, $Z_3$, identiques ou différents, représentent, des groupements choisis parmi un atome d'hydrogène, un alkyle en $C_1$-$C_{12}$, un groupement -OZ', -C(O)-O-Z' avec Z' un alkyle en $C_1$-$C_{12}$.

Avantageusement, lorsque A2 comprend un troisième monomère M3 de formule (X), ce monomère M3 est le styrène.

Avantageusement, le copolymère statistique ester boronique A2 a un pourcentage molaire de monomère(s) styrénique(s), avantageusement du styrène, de formule (IV), (V) et/ou (X), dans ledit copolymère allant de 2 à 50% molaire, préférentiellement de 3 à 40%, de manière plus préférée de 5 à 35% molaire.

Selon un mode de réalisation préféré, la chaîne formée par l'enchaînement des groupes $R_{10}$, M, X et $(R_8)_u$ avec u égal à 0 ou 1 du monomère de formule générale (IV) présente un nombre total d'atomes de carbone allant de 8 à 38, de préférence de 10 à 26.

Selon un mode de réalisation préféré, les chaînes latérales du copolymère A2 ont une longueur moyenne supérieure ou égale à 8 atomes de carbone, de préférence allant de 11 à 16 atomes de carbone.

Selon un mode de réalisation préféré, le copolymère A2 a un pourcentage molaire de monomère de formule

(IV) dans ledit copolymère allant de 0,25 à 30%, de préférence de 1 à 25%.

Selon un mode de réalisation préféré, le copolymère A2 a un degré de polymérisation moyen en nombre allant de 50 à 1500, de préférence de 50 à 800.

Selon un mode de réalisation préféré, le copolymère A2 a un indice de polydispersité (Ip) allant de 1,04 à 3,54 ; de préférence allant de 1,10 à 3,10.

Selon un mode de réalisation préféré, les substituants $R_{10}$, Ru et la valeur de l'indice (t) du monomère de formule (IV) du copolymère statistique A2 sont identiques respectivement aux substituants $G_4$, $G_5$ et à la valeur de l'indice (g), du composé exogène A5 de formule (XI).

Selon un mode de réalisation préféré, au moins un des substituants $R_{10}$, $R_{11}$ ou la valeur de l'indice (t) du monomère de formule (IV) du copolymère statistique A2 est différent respectivement des substituants $G_4$, $G_5$ ou de la valeur de l'indice (g), du composé exogène A5 de formule (XI).

Selon un mode de réalisation préféré, la teneur en copolymère A1 va de 0,1% à 50% en poids par rapport au poids total de la composition.

Selon un mode de réalisation préféré, la teneur en composé A2 va de 0,1% à 50% en poids par rapport au poids total de la composition.

Selon un mode de réalisation préféré, la composition comprend en outre au moins un composé exogène A4 choisi parmi les 1,2-diols et les 1,3-diols.

Selon un mode de réalisation préféré, la composition lubrifiante résultant du mélange d'au moins :

- d'une huile lubrifiante ; et
- d'une composition telle que définie ci-dessus et de façon détaillée ci-dessous.

Selon un mode de réalisation préféré, l'huile lubrifiante est choisie parmi les huiles du groupe I, du groupe II, du groupe III, du groupe IV, du groupe V de la classification API et l'un de leur mélange.

Selon un mode de réalisation préféré, la composition lubrifiante résulte du mélange avec en outre un additif fonctionnel choisi parmi le groupe formé par les antioxydants, les détergents, les additifs anti-usure, les additifs extrême pression, les polymères améliorant l'indice de viscosité, les améliorants de point d'écoulement, les anti-mousses, les additifs anticorrosion, les épaississants, les dispersants, les modificateurs de frottements et leurs mélanges.

**Description détaillée**

➢ <u>Composition d'additifs selon l'invention :</u>

Un premier objet de la présente invention est une composition d'additifs associatifs et échangeables de manière thermoréversible et dont le taux d'association est contrôlé par la présence d'un composé dit exogène, la composition résultant du mélange d'au moins :

- un copolymère statistique polydiol A1,
- un composé A2, notamment un copolymère statistique A2, comprenant au moins deux fonctions esters boroniques et pouvant s'associer avec ledit copolymère statistique polydiol A1 par une réaction de transestérification,
  - un composé exogène A5 choisi parmi ceux répondant à la formule (XI):

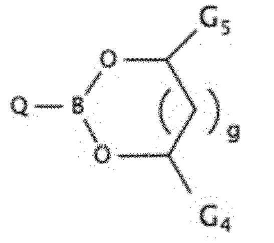

(XI)

dans laquelle :

- Q représente un groupement choisi parmi une chaîne hydrocarbonée comprenant de 1 à 30 atomes de

carbone, éventuellement substituée par un ou plusieurs groupements choisis parmi : un hydroxyle, un groupement -OJ, -C(O)-O-J avec J un groupement hydrocarboné comprenant de 1 à 24 atomes de carbone,
- $G_4$, $G_5$, identiques ou différents, représentent des groupements choisis parmi un atome d'hydrogène, une chaîne hydrocarbonée comprenant de 1 à 24 atomes de carbone, un hydroxyle, un groupement -OJ, -C(O)-O-J avec J un groupement hydrocarboné comprenant de 1 à 24 atomes de carbone,
- g représente 0 ou 1,

le copolymère statistique polydiol A1 ayant une masse molaire moyenne en nombre allant de 5 000 à 400 000 g/mol, le pourcentage molaire de composé exogène A5 par rapport aux fonctions diol du copolymère statistique A1 allant de 0,025 à 5000%, et le ratio massique entre le copolymère A1 et le composé A2 (ratio A1/A2) allant de 0,1 à 10.

Cette composition d'additifs permet de moduler le comportement rhéologique d'un milieu dans lequel elle est ajoutée. Le milieu peut être un milieu hydrophobe, notamment apolaire, tel qu'un solvant, une huile minérale, une huile naturelle, une huile synthétique.

◦ ***Copolymères statistiques polydiol A1***

Le copolymère statistique polydiol A1 résulte, directement ou indirectement, de la copolymérisation d'au moins un premier monomère M1 portant des fonctions diols, d'au moins un second monomère M2, de structure chimique différente de celle du monomère M1, et éventuellement d'au moins un troisième monomère M3, choisi parmi le styrène et les dérivés du styrène.

Par « résulte directement ou indirectement », on entend que le procédé de préparation du copolymère peut comprendre une ou plusieurs étapes distinctes de la copolymérisation, telle qu'une étape de déprotection. Notamment la copolymérisation peut éventuellement être suivie d'une étape de déprotection des fonctions diol.

Dans toute la description, on utilise indifféremment et de façon équivalente les expressions : « le copolymère statistique polydiol A1 résulte, directement ou indirectement, de la copolymérisation » et « le copolymère statistique polydiol A1 résulte de la copolymérisation ».

Par « copolymère », on entend un oligomère ou une macromolécule linéaire ou ramifiée ayant une séquence constituée de plusieurs unités répétitives (ou motif monomère) dont au moins deux unités ont une structure chimique différente.

Par « motif monomère » ou « monomère », on entend une molécule capable d'être convertie en un oligomère ou une macromolécule par combinaison avec luimême ou avec d'autres molécules du même type. Un monomère désigne la plus petite unité constitutive dont la répétition conduit à un oligomère ou à une macromolécule.

Par « copolymère statistique », on entend un oligomère ou une macromolécule dans lequel la distribution séquentielle des motifs monomères obéit à des lois statistiques connues. Par exemple, un copolymère est dit statistique lorsqu'il est constitué par des motifs monomères dont la distribution est une distribution markovienne. Un polymère statistique schématique (P1) est illustré en figure 1. La distribution dans la chaîne polymère des motifs monomères dépend de la réactivité des fonctions polymérisables des monomères et de la concentration relative des monomères. Les copolymères statistiques polydiols de l'invention se distinguent des copolymères à blocs et des copolymères à gradients. Par « bloc » on désigne une partie d'un copolymère comprenant plusieurs motifs monomères identiques ou différents et qui possèdent au moins une particularité de constitution ou de configuration permettant de la distinguer de ses parties adjacentes. Un copolymère à blocs schématique (P3) est illustré en figure 1. Un copolymère à gradient désigne un copolymère d'au moins deux motifs monomères de structures différentes dont la composition en monomère change de façon graduelle le long de la chaîne polymère, passant ainsi de façon progressive d'une extrémité de la chaîne polymère riche en un motif monomère, à l'autre extrémité riche en l'autre comonomère. Un polymère à gradient schématique (P2) est illustré en figure 1.

Par « copolymérisation », on entend un procédé qui permet de convertir un mélange d'au moins deux motifs monomères de structures chimiques différentes en un oligomère ou en un copolymère.

Dans la suite de la présente demande « B » représente un atome de bore.

Par « alkyle en $C_i$-$C_j$ » on entend une chaîne hydrocarbonée saturée, linéaire ou ramifiée, comprenant de i à j atomes de carbone. Par exemple, pour « alkyle en $C_1$-$C_{10}$ », on entend une chaîne hydrocarbonée saturée, linéaire ou ramifiée, comprenant de 1 à 10 atomes de carbone.

Par « aryle en $C_x$-$C_y$», on entend un groupe fonctionnel qui dérive d'un composé hydrocarboné aromatique comprenant de x à y atomes de carbone. Ce groupe fonctionnel peut être monocyclique ou polycyclique. A titre illustratif, un aryle en $C_6$-$C_{18}$ peut être le phényle, le naphtalène, l'anthracène, le phénanthrène et le tétracène.

Par « alcényle en $C_x$-$C_x$», on entend une chaîne hydrocarbonée linéaire ou ramifiée comportant au moins une insaturation, de préférence une double liaison carbone-carbone, et comprenant de x à y atomes de carbone.

Par « aralkyle en $C_x$-$C_y$ », on entend un composé hydrocarboné aromatique, de préférence monocyclique, substitué par au moins une chaîne alkyle linéaire ou ramifiée et dont le nombre total d'atomes de carbone du cycle aromatique et de ses substituants va de x à y atomes de carbone. A titre illustratif un aralkyle en $C_7$-$C_{18}$ peut être choisi dans le groupe formé par le benzyle, le tolyle et le xylyle.

Par groupe « aryle en $C_x$-$C_y$ substitué par un groupement $R'_3$ », on entend un composé hydrocarboné aromatique, de préférence monocyclique, comprenant de x à y atomes de carbone dont au moins un atome de carbone du cycle aromatique est substitué par un groupe $R'_3$.

Par « Hal » ou « halogène » on entend un atome d'halogène choisi parmi le groupe formé par le chlore, le brome, le fluor et l'iode.

• *Monomère M1*

Le premier monomère M1 du copolymère statistique polydiol (A1) de l'invention a pour formule générale (I) :

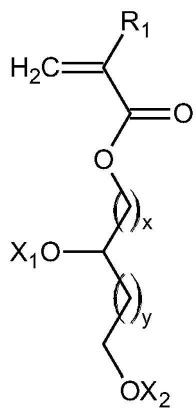

(I)

dans laquelle :

- $R_1$ est choisi parmi le groupe formé par -H, $-CH_3$ et $-CH_2-CH_3$, de préférence -H et $-CH_3$ ;
- x est un nombre entier allant de 1 à 18, de préférence allant de 2 à 18; de manière plus préférée de 3 à 8 ; de manière encore plus préférée x est égal à 4;
- y est un nombre entier égal à 0 ou 1 ; de préférence y est égal à 0 ;
- $X_1$ et $X_2$, identiques ou différents, sont choisis parmi le groupe formé par l'hydrogène, le tétrahydropyranyle, le méthyloxyméthyle, le ter-butyle, le benzyle, le triméthylsilyle et le t-butyle diméthylsilyle ;
  ou bien
- $X_1$ et $X_2$ forment avec les atomes d'oxygène un pont de formule suivante :

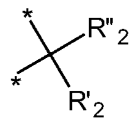

  dans laquelle :

  - les étoiles (*) symbolisent les liaisons aux atomes d'oxygène,
  - $R'_2$ et $R''_2$, identiques ou différents, sont choisis parmi le groupe formé par l'hydrogène et un groupe alkyle en $C_1$-$C_{11}$ ;
    ou bien

- $X_1$ et $X_2$ forment avec les atomes d'oxygène un ester boronique de formule suivante :

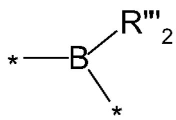

dans laquelle :

- les étoiles (*) symbolisent les liaisons aux atomes d'oxygène,
- R‴$_2$ est choisi parmi le groupe formé par un aryle en $C_6$-$C_{30}$, un aralkyle en $C_7$-$C_{30}$ et un alkyle en $C_2$-$C_{30}$, de préférence un aryle en $C_6$-$C_{18}$, de manière plus préférée le phényle.

De préférence, lorsque R'$_2$ et R"$_2$ est un groupe alkyle en $C_1$-$C_{11}$; la chaîne hydrocarbonée est une chaîne linéaire. De préférence, le groupe alkyle en $C_1$-$C_{11}$ est choisi parmi le groupe formé par le méthyle, l'éthyle, le n-propyle, le n-butyle, le n-pentyle, le n-hexyle, le n-heptyle, le n-octyle, le n-nonyle, le n-décycle et le n-undécyle. De manière plus préférée, le groupe alkyle $C_1$-$C_{11}$ est le méthyle.

De préférence, lorsque R‴$_2$ est un groupe alkyle en $C_2$-$C_{30}$ ; la chaîne hydrocarbonée est une chaîne linéaire.

Parmi les monomères de formule (I), les monomères répondant à la formule (I-A) font partie des préférés :

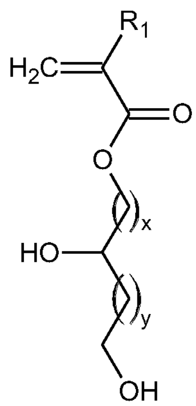

(I-A)

dans laquelle :

- $R_1$ est choisi parmi le groupe formé par -H, $-CH_3$ et $-CH_2$-$CH_3$, de préférence -H et $-CH_3$ ;
- x est un nombre entier allant de 1 à 18, de préférence allant de 2 à 18; de manière plus préférée de 3 à 8 ; de manière encore plus préférée x est égal à 4;
- y est un nombre entier égal à 0 ou 1; de préférence y est égal à 0.

Parmi les monomères de formule (I), les monomères répondant à la formule (I-B) font partie des préférés :

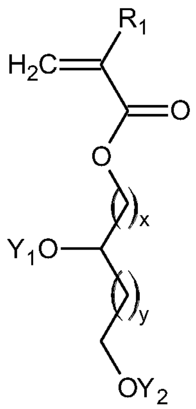

(I-B)

dans laquelle :

- $R_1$ est choisi parmi le groupe formé par -H, $-CH_3$ et $-CH_2$-$CH_3$, de préférence -H et $-CH_3$ ;
- x est un nombre entier allant de 1 à 18, de préférence allant de 2 à 18; de manière plus préférée de 3 à 8 ; de manière encore plus préférée x est égal à 4;
- y est un nombre entier égal à 0 ou 1; de préférence y est égal à 0 ;

- $Y_1$ et $Y_2$, identiques ou différents, sont choisis parmi le groupe formé par le tétrahydropyranyle, le méthyloxyméthyle, le ter-butyle, le benzyle, le triméthylsilyle et le t-butyle diméthylsilyle ;
  ou bien
- $Y_1$ et $Y_2$ forment avec les atomes d'oxygène un pont de formule suivante :

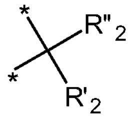

dans laquelle :

- les étoiles (*) symbolisent les liaisons aux atomes d'oxygène,
- R'$_2$ et R"$_2$, identiques ou différents, sont choisis parmi le groupe formé par l'hydrogène et un groupe alkyle en $C_1$-$C_{11}$ ;
  ou bien

- $Y_1$ et $Y_2$ forment avec les atomes d'oxygène un ester boronique de formule suivante :

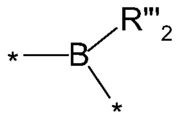

dans laquelle :

- les étoiles (*) symbolisent les liaisons aux atomes d'oxygène,
- R"$_2$ est choisi parmi le groupe formé par un aryle en $C_6$-$C_{18}$, un aralkyle en $C_7$-$C_{18}$ et un alkyle en $C_2$-$C_{30}$, de préférence un aryle en $C_6$-$C_{18}$, de manière plus préférée le phényle.

De préférence, lorsque R'$_2$ et R"$_2$ est un groupe alkyle en $C_1$-$C_{11}$; la chaîne hydrocarbonée est une chaîne linéaire. De préférence, le groupe alkyle $C_1$-$C_{11}$ est choisi parmi le groupe formé par le méthyle, l'éthyle, le n-propyle, le n-butyle, le n-pentyle, le n-hexyle, le n-heptyle, le n-octyle, le n-nonyle, le n-décycle et le n-undécyle. De manière plus préférée, le groupe alkyle $C_1$-$C_{11}$ est le méthyle.
De préférence, lorsque R"$_2$ est un groupe alkyle en $C_2$-$C_{30}$, la chaîne hydrocarbonée est une chaîne linéaire.
La synthèse du copolymère statistique polydiol (A1) peut comprendre la copolymérisation de monomères (I-B) sous forme protégée avec d'autres comonomères, suivie de la déprotection des fonctions diol des monomères (I-B).

• *Obtention du monomère M1*

Le monomère M1 de formule générale formule (I-A) est obtenu par déprotection des fonctions alcools du monomère de formule générale (I-B) selon le schéma réactionnel 1 ci-dessous :

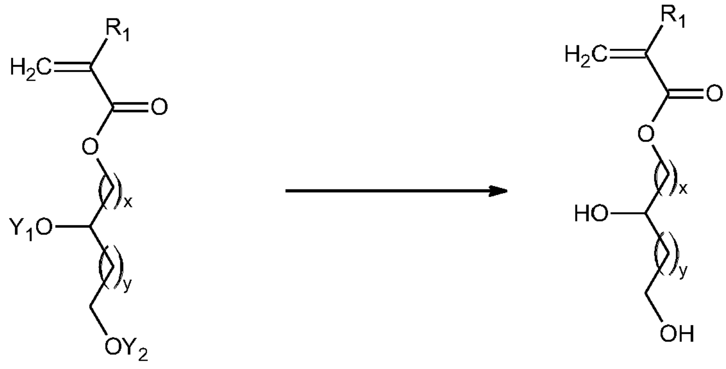

(I-B) (I-A)

Schéma 1

avec $R_1$, $Y_1$, $Y_2$, x et y tels que définis dans la formule générale (I-B) décrite ci-dessus.

La réaction de déprotection des fonctions diols du monomère de formule générale (I-B) est bien connue de l'homme du métier. Il sait adapter les conditions réactionnelles de déprotection en fonction de la nature des groupes protecteurs $Y_1$ et $Y_2$.

Le monomère M1 de formule générale (I-B) peut être obtenu par une réaction d'un composé de formule générale (I-c) avec un composé alcool de formule générale (I-b) selon le schéma réactionnel 2 ci-dessous :

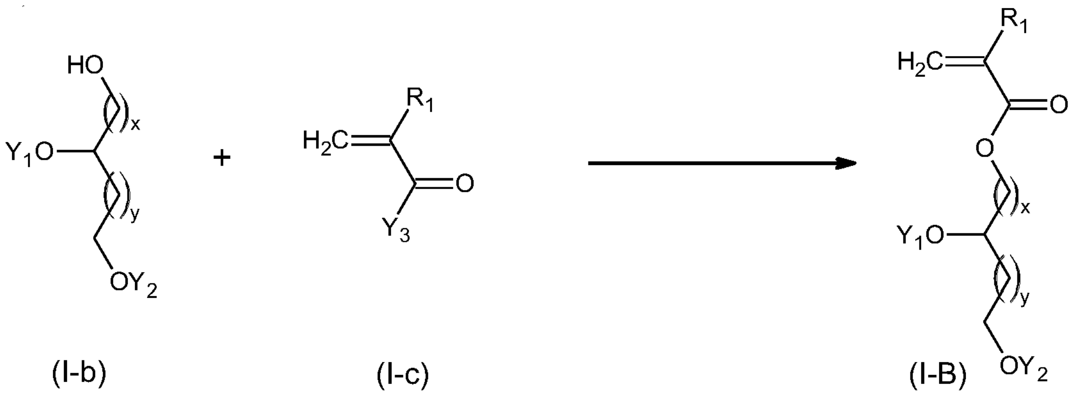

(I-b) (I-c) (I-B)

Schéma 2

dans lequel :

- $Y_3$ est choisi parmi le groupe formé par un atome d'halogène, de préférence le chlore, -OH et O-C(O)-R'$_1$ avec R'$_1$ choisi parmi le groupe formé par -H, $-CH_3$ et $-CH_2-CH_3$, de préférence -H et $-CH_3$ ;
- $R_1$, $Y_1$, $Y_2$, x et y ont la même signification que celle donnée dans formule générale (I-B).

Ces réactions de couplages sont bien connues de l'homme du métier.

Le composé de formule générale (I-c) est disponible commercialement auprès des fournisseurs : Sigma-Aldrich® et Alfa Aesar®.

Le composé alcool de formule générale (I-b) est obtenu à partir du polyol correspondant de formule (I-a) par protection des fonctions diols selon le schéma réactionnel 3 suivant :

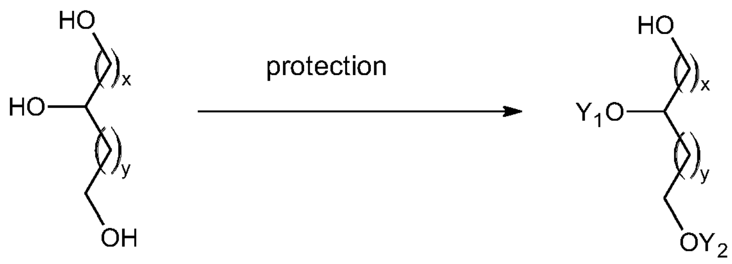

(I-a) (I-b)

Schéma 3

avec x, y, $Y_1$ et $Y_2$ tels que définis dans la formule générale (I-B).

La réaction de protection des fonctions diols du composé de formule générale (I-a) est bien connue de l'homme du métier. Il sait adapter les conditions réactionnelles de protection en fonction de la nature des groupes protecteurs $Y_1$ et $Y_2$ utilisés.

Le polyol de formule générale (I-a) est disponible commercialement auprès des fournisseurs : Sigma-Aldrich® et Alfa Aesar®.

Des exemples de synthèse des monomères M1 sont illustrés dans la partie expérimentale des demandes WO2015/110642, WO2015/110643 et WO2016113229.

• *Monomère M2*

Le second monomère du copolymère statistique de l'invention a pour formule générale (II) :

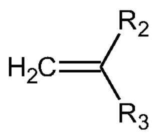

(II)

dans laquelle :

- $R_2$ est choisi parmi le groupe formé par -H, $-CH_3$ et $-CH_2-CH_3$, de préférence -H et-$CH_3$ ;
- $R_3$ est choisi parmi le groupe formé par un groupement aryle en $C_6-C_{18}$, un aryle en $C_6-C_{18}$ substitué par un groupement $R'_3$, -C(O)-O-$R'_3$ ; -O-$R'_3$, -S-$R'_3$ et -C(O)-N(H)-$R'_3$, avec $R'_3$ un groupe alkyle en $C_1-C_{30}$.

De préférence, $R'_3$ est un groupe alkyle en $C_1-C_{30}$ dont la chaîne hydrocarbonée est linéaire.

Parmi les monomères de formule (II), les monomères répondant à la formule (II-A) font partie des préférés :

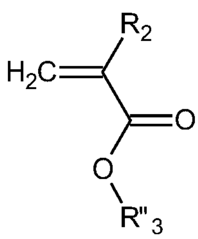

(II-A)

dans laquelle :

- $R_2$ est choisi parmi le groupe formé par -H, $-CH_3$ et $-CH_2-CH_3$, de préférence -H et $-CH_3$ ;
- $R''_3$ est un groupe alkyle en $C_1-C_8$.

Par « groupe alkyle en $C_1$-$C_8$ », on entend une chaîne hydrocarbonée saturée, linéaire ou ramifiée comprenant de 1 à 8 atomes de carbone. De préférence, la chaîne hydrocarbonée est linéaire.

Parmi les monomères de formule (II), les monomères répondant à la formule (II-B) font aussi partie des préférés :

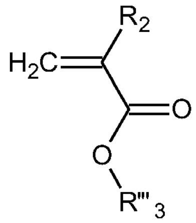

(II-B)

dans laquelle :

- $R_2$ est choisi parmi le groupe formé par -H, $-CH_3$ et $-CH_2-CH_3$, de préférence -H et $-CH_3$ ;
- $R'''_3$ est un groupe alkyle en $C_9$-$C_{30}$.

Par « groupe alkyle en $C_9$-$C_{30}$ », on entend une chaîne hydrocarbonée saturée, linéaire ou ramifiée comprenant de 9 à 30 atomes de carbone. De préférence, la chaîne hydrocarbonée est linéaire.

• *Obtention du monomère M2*

Les monomères de formule (II), (II-A) et, (II-B) sont bien connus de l'homme du métier. Ils sont commercialisés par Sigma-Aldrich® et TCI®.

• *Monomère M3*

Le troisième monomère, optionnel, du copolymère statistique de l'invention a pour formule générale (X) :

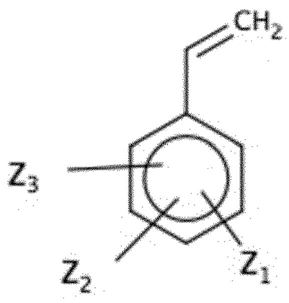

(X)

dans laquelle :

- $Z_1$, $Z_2$, $Z_3$, identiques ou différents, représentent des groupements choisis parmi un atome d'hydrogène, un alkyle en $C_1$-$C_{12}$, un groupement
- OZ', -C(O)-O-Z' avec Z' un alkyle en $C_1$-$C_{12}$.

Par « groupe alkyle en $C_1$-$C_{12}$ », on entend une chaîne hydrocarbonée saturée, linéaire ou ramifiée comprenant de 1 à 12 atomes de carbone. De préférence, la chaîne hydrocarbonée est linéaire. De manière préférée, la chaîne hydrocarbonée comprend de 1 à 6 atomes de carbone.

Avantageusement, $Z_1$, $Z_2$, $Z_3$, identiques ou différents, représentent des groupements choisis parmi un atome d'hydrogène, un alkyle en $C_1$-$C_6$, un groupement -OZ', -C(O)-O-Z' avec Z' un alkyle en $C_1$-$C_6$.

De manière encore préférée, $Z_1$, $Z_2$, $Z_3$, identiques ou différents, représentent des groupements choisis parmi

un atome d'hydrogène, un alkyle en C1-C4, un groupement -OZ', -C(O)-O-Z' avec Z' un alkyle en $C_1$-$C_4$.

Parmi les monomères M3 préférés, on peut citer : le styrène, le para tert-butyl styrène, le para méthoxy styrène, le para acétoxy styrène, le 2,4,6 triméthylstyrène, Selon un mode de réalisation préféré M3 est le styrène.

• *Obtention du monomère M3*

Certains monomères de formule (X), tels que le styrène, le para tert-butyl styrène, le para méthoxy styrène, le para acétoxy styrène, le 2,4,6 triméthylstyrène sont bien connus de l'homme du métier. Ils sont commercialisés notamment par Sigma-Aldrich®. D'autres monomères peuvent être préparés à partir de ces monomères commerciaux par des méthodes de synthèse bien connues de l'homme du métier.

• *Copolymères polydiols préférés*

Dans un mode de réalisation, un copolymère statistique préféré résulte de la copolymérisation d'au moins :

- un premier monomère M1 de formule générale (I) tel que décrit précédemment ; notamment de formule générale (I-A) telle que décrit précédemment ;
- un second monomère M2 de formule (II) tel que décrit précédemment, dans laquelle $R_2$ est $-CH_3$ et $R_3$ est un un groupement -C(O)-O-R'$_3$ avec -R'$_3$ un alkyle en $C_1$-$C_{30}$ ;
- éventuellement, un troisième monomère M3 de formule générale (X) tel que décrit précédemment ; notamment le styrène.

Dans un autre mode de réalisation, un copolymère statistique préféré résulte de la copolymérisation d'au moins :

- un premier monomère M1 de formule générale (I) tel que décrit précédemment ; notamment de formule générale (I-A) telle que décrit précédemment ;
- un second monomère M2 de formule (II-B) tel que décrit précédemment ;
- un troisième monomère M2 de formule (II-B) tel que décrit précédemment, distinct du premier monomère de formule (II-B) ; et
- un quatrième monomère M3 de formule générale (X) tel que décrit précédemment, notamment le styrène.

Selon cet autre mode de réalisation, un copolymère statistique préféré résulte de la copolymérisation d'au moins :

- un premier monomère M1 de formule générale (I) tel que décrit précédemment ; notamment de formule générale (I-A) telle que décrit précédemment ;
- un second monomère M2 de formule (II-B) dans laquelle $R_2$ est $-CH_3$ et $R'''_3$ est un groupe alkyle en $C_9$-$C_{30}$, de préférence un alkyle linéaire en $C_9$-$C_{30}$, encore mieux un alkyle linéaire en $C_9$-$C_{15}$;
- un troisième monomère M2 de formule (II-B), distinct du second monomère de formule (II-B), dans laquelle $R_2$ est $-CH_3$ et $R'''_3$ est un groupe alkyle en $C_9$-$C_{30}$, de préférence un alkyle linéaire en $C_9$-$C_{30}$, encore mieux un alkyle linéaire en $C_{16}$-$C_{24}$ ; et
- un quatrième monomère M3 de formule générale (X) tel que décrit précédemment, notamment le styrène.

Selon ce mode de réalisation, un copolymère statistique préféré résulte de la copolymérisation d'au moins :

- un premier monomère M1 de formule générale (I) tel que décrit précédemment ; notamment de formule générale (I-A) telle que décrit précédemment ;
- un second monomère M2 choisi dans le groupe formé par le méthacrylate de n-décyle et le méthacrylate de n-dodécyle ;
- un troisième monomère M2 choisi dans le groupe formé par le méthacrylate de palmityle, le méthacrylate de stéaryle, le méthacrylate d'arachidyle et le méthacrylate de béhényle,
- éventuellement, un quatrième monomère M3 de formule générale (X) tel que décrit précédemment, notamment le styrène.

Dans un autre mode de réalisation, un copolymère statistique préféré résulte de la copolymérisation d'au moins :

- un premier monomère M1 de formule générale (I) tel que décrit précédemment ; notamment de formule générale (I-A) telle que décrit précédemment ;

- un second monomère M2 de formule (II-A) tel que décrit précédemment ; et
- un troisième monomère M2 de formule (II-B) tel que décrit précédemment,
- éventuellement, un quatrième monomère M3 de formule générale (X) tel que décrit précédemment, notamment le styrène.

Selon cet autre mode de réalisation, un copolymère statistique préféré résulte de la copolymérisation d'au moins :

- un premier monomère M1 de formule générale (I) tel que décrit précédemment ; notamment de formule générale (I-A) telle que décrit précédemment ;
- un second monomère M2 de formule (II-A) dans laquelle $R_2$ est $-CH_3$ et $R''_3$ est un groupe alkyle en $C_1$-$C_8$, de préférence un alkyle linéaire en $C_1$-$C_8$ ;
- un troisième monomère M2 de formule (II-B) dans laquelle $R_2$ est - $CH_3$ et $R'''_3$ est un groupe alkyle en $C_9$-$C_{30}$, de préférence un alkyle linéaire en $C_9$-$C_{30}$,
- éventuellement, un quatrième monomère M3 de formule générale (X) tel que décrit précédemment, notamment le styrène.

Selon ce mode de réalisation, un copolymère statistique préféré résulte de la copolymérisation d'au moins :

- un premier monomère M1 de formule générale (I) tel que décrit précédemment ; notamment de formule générale (I-A) telle que décrit précédemment ;
- un second monomère M2 qui est le méthacrylate de n-octyle ;
- un troisième monomère M2 choisi dans le groupe formé par le méthacrylate de palmityle, le méthacrylate de stéaryle, le méthacrylate d'arachidyle et le méthacrylate de béhényle,
- éventuellement, un quatrième monomère M3 de formule générale (X) tel que décrit précédemment, notamment le styrène.

• *Procédé d'obtention des copolymères polydiol*

L'homme du métier est à même de synthétiser les copolymères statistiques polydiol A1 en faisant appel à ses connaissances générales.

La copolymérisation peut être amorcée en masse ou en solution dans un solvant organique par des composés générateurs de radicaux libres. Par exemple, les copolymères de l'invention sont obtenus par les procédés connus de copolymérisation radicalaire, notamment contrôlée telle que la méthode dénommée polymérisation radicalaire contrôlée par transfert de chaîne réversible par addition-fragmentation (en anglais : Réversible Addition-Fragmentation Chain Transfer (RAFT)) et la méthode dénommée polymérisation radicalaire contrôlée par transfert d'atome (en anglais Atom Transfer Radical Polymerization (ARTP)). La polymérisation radicalaire conventionnelle et la télomérisation peuvent également être employées pour préparer les copolymères de l'invention (Moad, G.; Solomon, D. H., The Chemistry of Radical Polymerization. 2nd ed.; Elsevier Ltd: 2006; p 639 ; Matyaszewski, K.; Davis, T. P. Handbook of Radical Polymerization; Wiley-Interscience: Hoboken, 2002; p 936).

Selon un mode de réalisation préféré, la copolymérisation est réalisée par polymérisation radicalaire conventionnelle, sans agent de transfert de chaîne RAFT.

Le copolymère statistique polydiol A1 est préparé selon un procédé de préparation qui comprend au moins une étape de polymérisation (a) dans laquelle on met en contact au moins :

i) un premier monomère M1 de formule générale (I) telle que décrite précédemment ;
ii) au moins un second monomère M2 de formule générale (II) telle que décrite précédemment ;
iii) éventuellement un troisième monomère M3 de formule générale (X) telle que décrite précédemment ;
iv) au moins une source de radicaux libres.

Dans un mode de réalisation, le procédé peut comprendre en outre v) au moins un agent de transfert de chaîne.

Par « une source de radicaux libres » on entend un composé chimique permettant de générer une espèce chimique possédant un ou plusieurs électrons non appariés sur sa couche externe. L'homme du métier peut utiliser toute source de radicaux libres connue en soi et adaptée aux procédés de polymérisation, notamment de polymérisation radicalaire contrôlée. Parmi les sources de radicaux libres, on préfère, à titre illustratif, le peroxyde de benzoyle, le peroxyde de tertio-butyle, les composés diazoïques tels que l'azobisisobutyronitrile, les composés péroxygénés tels que les persulfates ou l'eau oxygénée, les systèmes redox tels que l'oxydation de $Fe^{2+}$, les mélanges persulfates/sodium-métabisulfite, ou l'acide ascorbique/eau oxygénée ou encore les composés clivables photochimiquement ou par radiations ionisantes, par exemple les rayons ultra-violet ou par rayonnement beta ou gamma.

Par « agent de transfert de chaîne », on entend un composé dont le but est d'assurer une croissance homogène des chaînes macromoléculaires par réactions de transfert réversible entre espèces en croissance, i.e. chaînes polymères terminées par un radical carboné, et espèces dormantes, i.e. chaînes polymères terminées par un agent de transfert. Ce processus de transfert réversible permet de contrôler les masses moléculaires de copolymères ainsi préparés. De préférence dans le procédé de l'invention, l'agent de transfert de chaîne comprend un groupe thiocarbonylthio -S-C(=S)- . A titre illustratif d'agent de transfert de chaîne, on peut citer les dithioesters, les trithiocarbonates, les xanthates et les dithiocarbamates. Un agent de transfert préféré est le dithiobenzoate de cumyle ou le 2-cyano-2-propyl benzodithioate.

Par « agent de transfert de chaîne », on entend également un composé dont le but est de limiter la croissance des chaînes macromoléculaires en cours de formation par addition de molécules monomères et d'amorcer de nouvelles chaînes, ce qui permet de limiter les masses moléculaires finales, voire de les contrôler. Un tel type d'agent de transfert est utilisé en télomérisation. Un agent de transfert préféré est la cystéamine.

Dans un mode de réalisation, le procédé de préparation d'un copolymère statistique polydiol comprend :

- au moins une étape de polymérisation (a) telle que définie ci-dessus, dans laquelle les monomères M1 et M2 sont choisis avec $X_1$ et $X_2$ représentent l'hydrogène.

Dans un mode de réalisation, l'étape de polymérisation (a) comprend la mise en contact d'au moins un monomère M1 avec au moins deux monomères M2 ayant des groupes $R_3$ différents et éventuellement au moins un monomère M3, de préférence le styrène.

Selon un mode de réalisation (lorsque l'on a procédé à une polymérisation radicalaire avec agent de transfert de chaîne RAFT), après la synthèse directe du polymère contenant les fonctions diol, le procédé comprend une étape d'élimination du bout de chaîne RAFT par aminolyse puis addition de Michael.

Les préférences et définitions décrites pour les formules générales (I), (I-A), (I-B), (II-A), (II-B) et (X) s'appliquent également aux procédés décrits ci-dessus.

• *Propriétés des copolymères polydiols A1*

Les copolymères statistiques polydiols A1 sont des copolymères linéaires. De façon alternative, certains monomères pourraient donner accès à des copolymères peignes.

Par « copolymères peignes », on entend un copolymère disposant d'une chaîne principale (aussi appelée squelette) et de chaînes latérales. Les chaînes latérales sont pendantes de part et d'autre de la chaîne principale. La longueur de chaque chaîne latérale est inférieure à la longueur de la chaîne principale. La figure 2 représente de manière schématique un polymère peigne.

Les copolymères A1 présentent un squelette de fonctions polymérisables, notamment un squelette de fonctions méthacrylate, et éventuellement de fonctions styrène, et un mélange de chaînes latérales hydrocarbonées substituées ou non par des fonctions diol.

Comme les monomères de formule (I), (II) et éventuellement (X) présentent des fonctions polymérisables de réactivité identique ou sensiblement identique, on obtient un copolymère dont les monomères ayant des fonctions diols sont distribués statistiquement le long du squelette du copolymère par rapport aux monomères styréniques et par rapport à ceux dont les chaînes alkyles sont non substituées par des fonctions diols.

Les copolymères statistiques polydiol A1 présentent l'avantage d'être sensibles à des stimuli extérieurs, tels que la température, la pression, la vitesse de cisaillement ; cette sensibilité se traduisant par un changement de propriétés. En réponse à un stimulus, la conformation dans l'espace des chaînes de copolymère est modifiée et les fonctions diols sont rendues plus ou moins accessibles aux réactions d'association, pouvant générer une réticulation, ainsi qu'aux réactions d'échanges. Ces processus d'association et d'échange sont réversibles. Le copolymère statistique A1 est un copolymère thermosensible, c'est-à-dire qu'il est sensible aux changements de température.

Avantageusement, les chaînes latérales du copolymère statistique polydiol A1 ont une longueur moyenne allant de 8 à 20 atomes de carbone, de préférence de 9 à 18 atomes de carbone. Par « longueur moyenne de chaîne latérale » on entend la longueur moyenne des chaînes latérales des monomères M1 de formule (I) et M2 de formule (II) entrant dans la constitution du copolymère. Les chaînes latérales issues du ou des éventuels monomères styréniques ne sont pas prises en compte dans le calcul des longueurs moyennes des chaînes latérales. L'homme du métier sait obtenir cette longueur moyenne en sélectionnant de manière appropriée les types et le ratio de monomères constituant le copolymère statistique polydiol. Le choix de cette longueur moyenne de chaîne permet d'obtenir un polymère soluble dans un milieu hydrophobe, quelle que soit la température à laquelle le copolymère est dissous. Le copolymère statistique polydiol A1 est donc miscible dans un milieu hydrophobe. Par « milieu hydrophobe » on entend un milieu qui n'a pas d'affinité ou qui a une très faible affinité pour l'eau, c'est à dire qu'il n'est pas miscible dans l'eau ou dans un milieu aqueux.

Avantageusement, le copolymère statistique polydiol A1 a un pourcentage molaire de monomère M1 de formule

(I) dans ledit copolymère allant de 1 à 30 %, de préférence de 5 à 25%.

Selon un mode de réalisation avantageux, le copolymère statistique polydiol A1 a un pourcentage molaire de monomère M3 de formule (X), avantageusement du styrène, dans ledit copolymère allant de 3 à 40%, de manière plus préférée allant de 5 à 35%.

Dans un mode de réalisation préféré, le copolymère statistique polydiol A1 a un pourcentage molaire de monomère M1 de formule (I) dans ledit copolymère allant de 1 à 30 %, de préférence 5 à 25%, un pourcentage molaire de monomère(s) M2 de formule (II-B) dans ledit copolymère allant de 0,1 à 95%, de préférence de 5 à 80% et un pourcentage molaire de monomère M3 de formule (X), avantageusement du styrène, dans ledit copolymère allant de 3 à 40%, de manière plus préférée allant de 5 à 35%.

Dans un autre mode de réalisation préféré, le copolymère statistique polydiol A1 a un pourcentage molaire de monomère M1 de formule (I) dans ledit copolymère allant de 1 à 30 %, de préférence 5 à 25%, un pourcentage molaire de monomère M2 de formule (II-A) dans ledit copolymère allant de 8 à 92% un pourcentage molaire de monomère M2 de formule (II-B) dans ledit copolymère allant de 0,1 à 62%, et éventuellement un pourcentage molaire de monomère M3 de formule (X), avantageusement du styrène, dans ledit copolymère allant de 3 à 40%, de manière plus préférée allant de 5 à 35%. Le pourcentage molaire de monomères dans le copolymère résulte directement de l'ajustement des quantités de monomères mises en oeuvre pour la synthèse du copolymère.

Avantageusement, le copolymère statistique polydiol A1 a un degré de polymérisation moyen en nombre allant de 40 à 2000, de préférence de 40 à 1000. De façon connue, le degré de polymérisation est contrôlé en utilisant une technique de polymérisation radicalaire contrôlée, une technique de télomérisation ou en ajustant la quantité de source de radicaux libres lorsque les copolymères de l'invention sont préparés par polymérisation radicalaire conventionnelle.

Avantageusement, le copolymère statistique polydiol A1 a un indice de polydispersité (Ip) allant de 1,05 à 4,0 ; de préférence allant de 1,10 à 3,8. L'indice de polydispersité est obtenu par mesure de chromatographie d'exclusion stérique en utilisant une calibration poly(méthacrylate de méthyle).

Le copolymère statistique polydiol A1 a une masse molaire moyenne en nombre allant de 5 000 à 400 000 g/mol, de préférence de 10 000 à 200 000 g/mol, la masse molaire moyenne en nombre étant obtenue par mesure de chromatographie d'exclusion stérique en utilisant une calibration poly(méthacrylate de méthyle)

La méthode de mesure de chromatographie d'exclusion stérique en utilisant une calibration poly(méthacrylate de méthyle)est décrite dans l'ouvrage (Fontanille, M.; Gnanou, Y., Chimie et physico-chimie des polymères. 2nd ed.; Dunod: 2010; p 546).

◦ *Composé A2*

• *Composé A2 diester boronique*

Dans un mode de réalisation, le composé A2 comprenant deux fonctions esters boroniques a pour formule générale (III) :

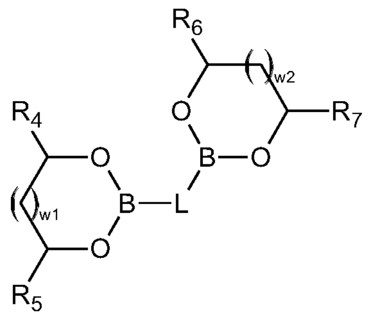

(III)

dans laquelle :

- $w_1$ et $w_2$, identiques ou différents sont des nombres entiers égaux à 0 ou 1,
- $R_4$, $R_5$, $R_6$ et $R_7$, identiques ou différents, sont choisis parmi le groupe formé par l'hydrogène et un groupement hydrocarboné comprenant de 1 à 30 atomes de carbone, de préférence entre 4 et 18 atomes de carbone, encore plus préférentiellement entre 6 et 14 atomes de carbone, ledit groupement hydrocarboné étant éventuellement substitué par un ou plusieurs groupements choisis parmi : un hydroxyle, un groupement -OJ, -C(O)-O-J avec J un

groupement hydrocarboné comprenant de 1 à 24 atomes de carbone ;

- L est un groupement de liaison divalent et choisi parmi le groupe formé par un aryle en $C_6$-$C_{18}$, un aralkyle en $C_7$-$C_{24}$ et une chaîne hydrocarbonée en $C_2$-$C_{24}$, de préférence un aryle en $C_6$-$C_{18}$.

Par « groupement hydrocarboné comprenant de 1 à 30 atomes de carbone », on entend un groupement alkyle linéaire, ramifié ou cyclique comprenant de 1 à 30 atomes de carbone, alcényle linéaire, ramifié ou cyclique comprenant de 2 à 30 atomes de carbone, un groupement aryle comprenant de 6 à 30 atomes de carbone ou un groupement aralkyle comprenant de 7 à 30 atomes de carbone.

Par « groupement hydrocarboné comprenant de 1 à 24 atomes de carbone » on entend un groupe alkyle linéaire ou ramifié, comprenant de 1 à 24 atomes de carbone ou alcényle linéaire ou ramifié, comprenant de 2 à 24 atomes de carbone, un groupe aryle comprenant de 6 à 24 atomes de carbone, ou un groupe aralkyle comprenant de 7 à 24 atomes de carbone. De préférence, J comprend de 4 à 18 atomes de carbone, de préférence entre 6 et 12 atomes de carbone.

Par « chaîne hydrocarbonée en $C_2$-$C_{24}$ », on entend un groupe alkyle ou alcényle, linéaire ou ramifié, comprenant de 2 à 24 atomes de carbone. De préférence, la chaîne hydrocarbonée est un groupe alkyle linéaire. De préférence la chaîne hydrocarbonée comprend de 6 à 16 atomes de carbone.

Dans un mode de réalisation de l'invention, le composé A2 est un composé de formule générale (III) ci-dessus dans laquelle :

- $w_1$ et $w_2$, identiques ou différents sont des nombres entiers égaux à 0 ou 1;
- $R_4$ et $R_6$ sont identiques et sont des atomes d'hydrogène ;
- $R_5$ et $R_7$ sont identiques et sont un groupe hydrocarboné, de préférence un alkyle linéaire, ayant de 1 à 24 atomes de carbone, de préférence de 4 à 18 atomes de carbone, de préférence de 6 à 16 atomes de carbone ;
- L est un groupement de liaison divalent et est un aryle en $C_6$-$C_{18}$, de préférence le phényle.

Le composé A2 diester boronique de formule (III) telle que décrite ci-dessus est obtenu par une réaction de condensation entre un acide boronique de formule générale (III-a) et des fonctions diols des composés de formule générale (III-b) et (III-c) selon le schéma réactionnel 4 ci-dessous :

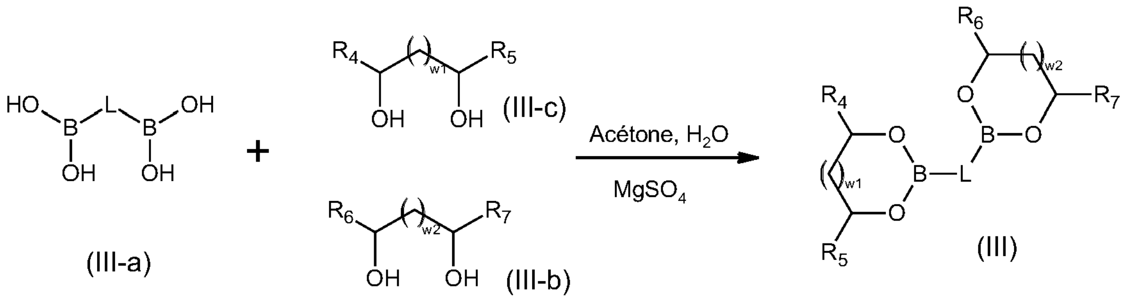

(III-a) (III-c) (III-b) (III)

Schéma 4

avec $w_1$, $w_2$, L, $R_4$, $R_5$, $R_6$ et $R_7$, tels que définis ci-dessus.

En effet, par condensation des fonctions acides boroniques du composé (III-a) avec des fonctions diols des composés de formule (III-b) et de formule (III-c), on obtient des composés ayant deux fonctions esters boronique (composé de formule (III)). Cette étape s'effectue selon des moyens bien connus de l'homme du métier.

Dans le cadre de la présente invention, le composé de formule générale (III-a) est dissous, en présence d'eau, dans un solvant polaire tel que l'acétone. La présence d'eau permet de déplacer les équilibres chimiques entre les molécules d'acide boronique de formule (III-a) et les molécules de boroxine obtenues à partir des acides boroniques de formule (III-a). En effet, il est bien connu que les acides boroniques peuvent former spontanément à température ambiante des molécules de boroxine. Or, la présence de molécules de boroxine n'est pas souhaitable dans le cadre de la présente invention.

La réaction de condensation s'effectue en présence d'un agent de déshydratation tel que le sulfate de magnésium. Cet agent permet de piéger les molécules d'eau initialement introduites ainsi que celles qui sont libérées par la condensation entre le composé de formule (III-a) et le composé de formule (III-b) et entre le composé de formule (III-a) et le composé de formule (III-c).

Dans un mode de réalisation, le composé (III-b) et le composé (III-c) sont identiques.

L'homme du métier sait adapter les quantités de réactifs de formule (III-b) et/ou (III-c) et de formule (III-a) pour

obtenir le produit de formule (III).

• *Composé A2 copolymère statistique poly(ester boronique)*

Dans un autre mode de réalisation, le composé A2 comprenant au moins deux fonctions esters boroniques est un copolymère statistique poly(ester boronique) résultant de la copolymérisation d'au moins un monomère M4 de formule (IV) telle que décrite ci-dessous avec au moins un monomère M5 de formule (V) telle que décrite ci-dessous.
Dans la suite de la demande, les expressions « copolymère statistique ester boronique » ou « copolymère statistique poly(ester boronique) » sont équivalentes et désigne le même copolymère.

✓Monomère M4 de formule (IV)

Le monomère M4 du composé A2 copolymère statistique ester boronique a pour formule générale (IV): dans laquelle :

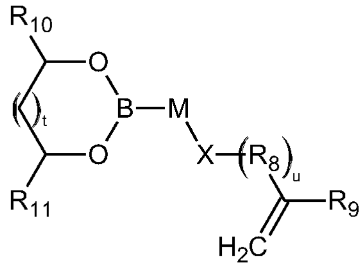

(IV)

- t est un nombre entier égal à 0 ou 1 ;
- u est un nombre entier égal à 0 ou 1 ;
- M et $R_8$ sont des groupements de liaison divalent, identiques ou différents, et sont choisis parmi le groupe formé par un aryle en $C_6$-$C_{18}$, un aralkyle en $C_7$-$C_{24}$ et alkyle en $C_2$-$C_{24}$, de préférence un aryle en $C_6$-$C_{18}$,
- X est une fonction choisie parmi le groupe formé par -O-C(O)-, - C(O)-O-, -C(O)-N(H)-, -N(H)-C(O)-, -S- , -N(H)- , -N(R'$_4$)-et -O- avec R'$_4$ une chaîne hydrocarbonée comprenant de 1 à 15 atomes de carbone;
- $R_9$ est choisi parmi le groupe formé par -H, $-CH_3$ et $-CH_2$-$CH_3$ ; de préférence -H et-$CH_3$;
- $R_{10}$ et $R_{11}$, identiques ou différents, sont choisis parmi le groupe formé par l'hydrogène et un groupement hydrocarboné ayant de 1 à 30 atomes de carbone, éventuellement substitué par un ou plusieurs groupements choisis parmi : un hydroxyle, un groupement -OJ, - C(O)-O-J avec J un groupement hydrocarboné comprenant de 1 à 24 atomes de carbone ;

Par « alkyle en $C_1$-$C_{24}$ », on entend une chaîne hydrocarbonée saturée, linéaire ou ramifiée, comprenant de 1 à 24 atomes de carbone. De préférence, la chaîne hydrocarbonée est linéaire. De préférence la chaîne hydrocarbonée comprend de 6 à 16 atomes de carbone.
Par « chaîne hydrocarbonée comprenant de 1 à 15 atomes de carbone », on entend, un groupe alkyle ou alcényle linéaire ou ramifié, comprenant de 1 à 15 atomes de carbone. De préférence, la chaîne hydrocarbonée est un groupe alkyle linéaire. De préférence, elle comprend de 1 à 8 atomes de carbone.
Par « groupement hydrocarboné comprenant de 1 à 30 atomes de carbone », on entend un groupement alkyle linéaire, ramifié ou cyclique comprenant de 1 à 30 atomes de carbone, alcényle linéaire, ramifié ou cyclique comprenant de 2 à 30 atomes de carbone, un groupement aryle comprenant de 6 à 30 atomes de carbone ou un groupement aralkyle comprenant de 7 à 30 atomes de carbone.
Par « groupement hydrocarboné comprenant de 1 à 24 atomes de carbone » on entend un groupe alkyle linéaire ou ramifié, comprenant de 1 à 24 atomes de carbone ou alcényle linéaire ou ramifié, comprenant de 2 à 24 atomes de carbone, un groupe aryle comprenant de 6 à 24 atomes de carbone, ou un groupe aralkyle comprenant de 7 à 24 atomes de carbone. De préférence, J comprend de 4 à 18 atomes de carbone, de préférence entre 6 et 12 atomes de carbone.
Dans un mode de réalisation, le monomère M4 a pour formule générale (IV) dans laquelle :

- t est un nombre entier égal à 0 ou 1 ;
- u est un nombre entier égal à 0 ou 1 ;
- M et $R_8$ sont des groupements de liaison divalents et sont différents, M est un aryle en $C_6$-$C_{18}$, de préférence le

phényle, $R_8$ est un aralkyle en $C_7$-$C_{24}$, de préférence le benzyle ;

- X est une fonction choisie parmi le groupe formé par -O-C(O)-, -C(O)-O-, -C(O)-N(H)- et -O-, de préférence -C(O)-O- ou - O-C(O)- ;
- $R_9$ est choisi parmi le groupe formé par -H, $-CH_3$, de préférence -H ;
- $R_{10}$ et $R_{11}$ sont différents, l'un des groupes $R_{10}$ ou $R_{11}$ est H et l'autre groupe $R_{10}$ ou $R_{11}$ est une chaîne hydrocarbonée, de préférence un groupe alkyle linéaire, ayant de 1 à 24 atomes de carbone, de préférence entre 4 et 18 atomes de carbone, de préférence entre 6 et 12 atomes de carbone.

Dans un mode de réalisation, le monomère M4 est un monomère styrénique. C'est le cas lorsque, dans la formule (IV) : u = 1, $R_9$ est H et $R_8$ représente un aryle en $C_6$-$C_{18}$ ou un aralkyle en $C_7$-$C_{24}$ et la double liaison du monomère M4 de formule (IV) est directement connectée au groupement aryle.

✓ Synthèse du monomère M4 de formule (IV)

Dans tous les schémas exposés ci-dessous, sauf indication contraire, les variables $R_{10}$, $R_{11}$, M, u, t, X, $R_8$, $R'_4$ et $R_9$ ont la même définition que dans la formule (IV) ci-dessus.

Les monomère M4 de formule (IV) sont notamment obtenus à partir d'un procédé de préparation comprenant au moins une étape de condensation d'un acide boronique de formule générale (IV-f) avec un composé diol de formule générale (IV-g) selon le schéma réactionnel 5 ci-dessous :

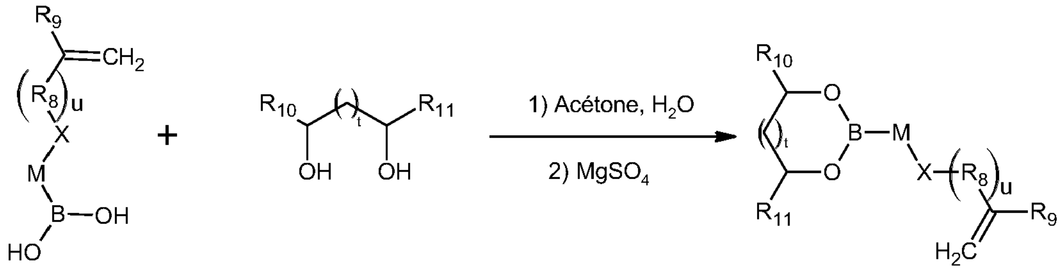


(IV-f) (IV-g) (IV)

Schéma 5

En effet, par condensation des fonctions acide boronique du composé de formule (IV-f) avec des fonctions diols des composés de formule (IV-g), on obtient un composé ester boronique de formule (IV). Cette étape s'effectue selon des méthodes bien connues de l'homme du métier.

Dans le cadre de la présente invention, le composé de formule générale (IV-f) est dissous, en présence d'eau, dans un solvant polaire tel que l'acétone. La réaction de condensation s'effectue en présence d'un agent de déshydratation, tel que le sulfate de magnésium.

Les composés de formule (IV-g) sont disponibles commercialement auprès des fournisseurs suivants : Sigma-Aldrich®, Alfa Aesar® et TCI®.

Le composé de formule (IV-f) est obtenu directement à partir du composé de formule (IV-e) par hydrolyse selon le schéma réactionnel 6 suivant :

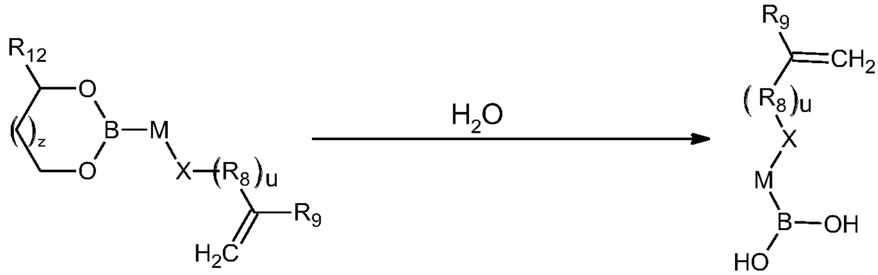


(IV-e) (IV-f)

Schéma 6

avec

- z un nombre entier égal à 0 ou 1 ;
- $R_{12}$ est choisi parmi le groupe formé par -H, $-CH_3$ et $-CH_2-CH_3$ ;
- u, X, M, $R_8$ et $R_9$ tels que définis ci-dessus.

Le composé de formule (IV-e) est obtenu par réaction d'un composé de formule (IV-c) avec un composé de formule (IV-d) selon le schéma réactionnel 7 suivant :

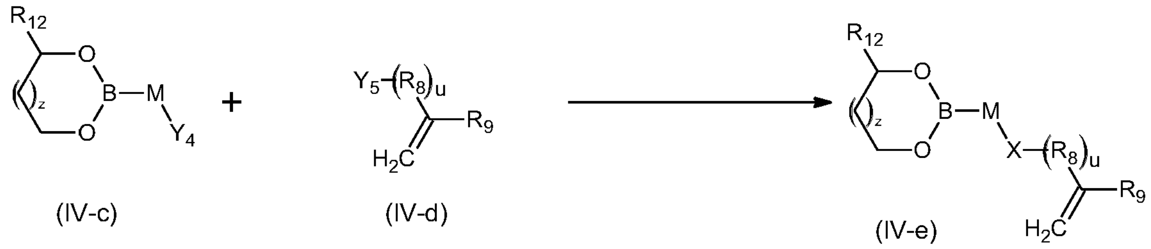

(IV-c) (IV-d) (IV-e)

Schéma 7

avec

- z, u, $R_{12}$, M, $R'_4$, $R_9$ et $R_8$ tels que définis ci-dessus ;

et dans ce schéma lorsque :

- X représente -O-C(O)-, alors $Y_4$ représente une fonction alcool -OH ou un atome d'halogène, de préférence le chlore ou le brome et $Y_5$ est une fonction acide carboxylique -C(O)-OH ;
- X représente -C(O)-O-, alors $Y_4$ représente une fonction acide carboxylique -C(O)-OH et $Y_5$ est une fonction alcool -OH ou un atome d'halogène, et de préférence le chlore ou le brome ;
- X représente -C(O)-N(H)-, alors $Y_4$ représente une fonction acide carboxylique -C(O)-OH ou une fonction -C(O)-Hal, et $Y_5$ est une fonction amine $NH_2$ ;
- X représente -N(H)-C(O)-, alors $Y_4$ représente une fonction amine $NH_2$ et $Y_5$ est une fonction acide carboxylique -C(O)-OH ou une fonction -C(O)-Hal ;
- X représente -S-, alors $Y_4$ est un atome d'halogène et $Y_5$ est une fonction thiol -SH ou bien $Y_4$ est une fonction thiol -SH et $Y_5$ est un atome d'halogène ;
- X représente -N(H)-, alors $Y_4$ est un atome d'halogène et $Y_5$ est une fonction amine $-NH_2$ ou bien $Y_4$ est une fonction amine $-NH_2$ et $Y_5$ est un atome d'halogène ;
- X représente $-N(R'_4)-$ , alors $Y_4$ est un atome d'halogène et $Y_5$ est une fonction amine $-N(H)(R'_4)$ ou bien $Y_4$ est une fonction amine $-N(H)(R'_4)$ et $Y_5$ est un atome d'halogène ;
- X représente -O-, alors $Y_4$ est un atome d'halogène et $Y_5$ est une fonction alcool -OH ou bien $Y_4$ est une fonction alcool -OH et $Y_5$ est un atome d'halogène.

Ces réactions d'estérification, d'éthérification, de thioéthérification, d'alkylation ou de condensation entre une fonction amine et une fonction acide carboxylique sont bien connues de l'homme du métier. L'homme du métier sait donc choisir en fonction de la nature chimique des groupes $Y_1$ et $Y_2$ les conditions réactionnelles pour obtenir le composé de formule (IV-e).

Les composés de formule (IV-d) sont disponibles commercialement auprès des fournisseurs : Sigma-Aldrich®, TCI® et Acros Organics®.

Le composé de formule (IV-c) est obtenu par une réaction de condensation entre un acide boronique de formule (IV-a) avec au moins un composé diol de formule (IV-b) selon le schéma réactionnel 8 suivant :

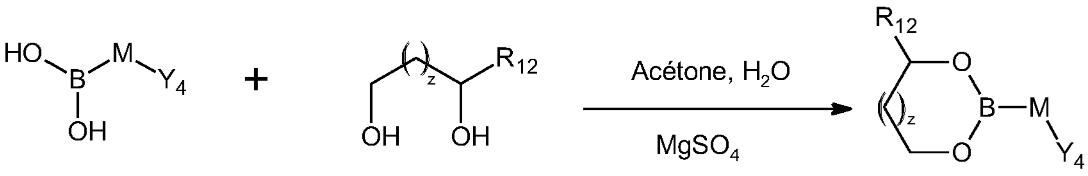


(IV-a) (IV-b) (IV-c)

Schéma 8

avec M, $Y_4$, z et $R_{12}$ tels que définis ci-dessus,

Parmi les composés de formule (IV-b), on préfère celui dans lequel $R_{12}$ est le méthyle et z=0.

Les composés de formule (IV-a) et (IV-b) sont disponibles commercialement auprès des fournisseurs suivant Sigma-Aldrich®, Alfa Aesar® et TCI®.

✓<u>Monomère M5 de formule générale (V) :</u>

Le monomère M5 du composé A2 copolymère statistique ester boronique a pour formule générale (V)

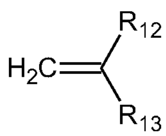
(V)

dans laquelle :

- $R_{12}$ est choisi parmi le groupe formé par -H, $-CH_3$ et $-CH_2-CH_3$, de préférence -H et $-CH_3$ ;
- $R_{13}$ est choisi parmi le groupe formé par un aryle en $C_6$-$C_{18}$, un aryle en $C_6$-$C_{18}$ substitué par un groupement $R'_{13}$, -C(O)-O-$R'_{13}$ ; -O-$R'_{13}$, -S-$R'_{13}$ et -C(O)-N(H)-$R'_{13}$ avec $R'_{13}$ un groupe alkyle en $C_1$-$C_{25}$.

Par « groupe alkyle en $C_1$-$C_{25}$ », on entend une chaîne hydrocarbonée saturée, linéaire ou ramifiée, comprenant de 1 à 25 atomes de carbone. De préférence, la chaîne hydrocarbonée est linéaire.

Par groupe « aryle en $C_6$-$C_{18}$ substitué par un groupement $R_{13}$ », on entend un composé hydrocarboné aromatique comprenant de 6 à 18 atomes de carbone dont au moins un atome de carbone du cycle aromatique est substitué par un groupe alkyle en $C_1$-$C_{25}$ tel que défini ci-dessus.

Parmi les monomères de formule (V), les monomères répondant à la formule (V-A) font partie des préférés :

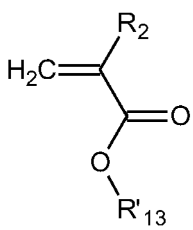
(V-A)

dans laquelle :

- $R_2$ est choisi parmi le groupe formé par -H, $-CH_3$ et $-CH_2-CH_3$, de préférence -H et $-CH_3$ ;
- $R'_{13}$ un groupe alkyle en $C_1$-$C_{25}$, de préférence un alkyle linéaire en $C_1$-$C_{25}$, de manière encore plus préféré un alkyle linéaire en $C_5$-$C_{15}$.

Selon un mode de réalisation, le monomère M5 est un monomère styrénique. C'est le cas lorsque, dans la formule (V) : $R_{12}$ représente H et $R_{13}$ est choisi parmi le groupe formé par un aryle en $C_6$-$C_{18}$ et un aryle en $C_6$-$C_{18}$

substitué par un groupement $R'_{13}$ avec $R'_{13}$ un groupe alkyle en $C_1$-$C_{25}$ et la double liaison du monomère M5 de formule (V) est directement connectée au groupement aryle.

Avantageusement, selon ce mode de réalisation, le monomère M5 est le styrène.

✓ Obtention du monomère M5 :

Les monomères de formules (V) et (V-A) sont bien connus de l'homme du métier. Ils sont commercialisés par Sigma-Aldrich® et TCI®.

✓ Monomère styrénique :

Avantageusement, le copolymère A2 comprend au moins un monomère à caractère styrénique, c'est à dire soit le styrène, soit un dérivé du styrène, tel qu'un styrène substitué par un autre groupement sur le cycle aromatique.

Le monomère M4 peut être un monomère styrénique lorsque, dans la formule (IV) : u = 1, $R_9$ est H et $R_8$ représente un aryle en $C_6$-$C_{18}$ ou un aralkyle en $C_7$-$C_{24}$ et la double liaison du monomère M4 de formule (IV) est directement connectée au groupement aryle.

Le monomère M5 peut aussi être un monomère styrénique lorsque, dans la formule (V) : $R_{12}$ représente H et $R_{13}$ est choisi parmi le groupe formé par un aryle en $C_6$-$C_{18}$ et un aryle en $C_6$-$C_{18}$ substitué par un groupement $R'_{13}$ avec $R'_{13}$ un groupe alkyle en $C_1$-$C_{25}$ et la double liaison du monomère M5 de formule (V) est directement connectée au groupement aryle.

Lorsque ni M4 ni M5 ne présentent un caractère styrénique, avantageusement, le copolymère A2 comprend au moins un troisième monomère M3 de formule (X) :

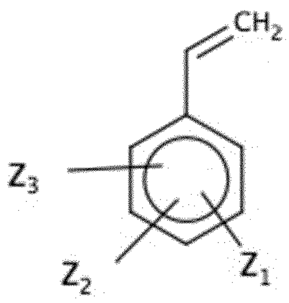

dans laquelle :

- $Z_1$, $Z_2$, $Z_3$, identiques ou différents, représentent, des groupements choisis parmi un atome d'hydrogène, un alkyle en $C_1$-$C_{12}$, un groupement -OZ', -C(O)-O-Z' avec Z' un alkyle en $C_1$-$C_{12}$.

M3 a été décrit de façon détaillée ci-dessus pour la préparation du copolymère A1. Les monomères M3 préférés et leurs quantités préférées sont les mêmes dans A2 que dans A1.

Avantageusement, lorsque A2 comprend un troisième monomère M3 de formule (X), ce monomère M3 est le styrène.

✓ Synthèse du composé A2 copolymère statistique poly(ester boronique)

L'homme du métier est à même de synthétiser les copolymères statistiques ester boronique en faisant appel à ses connaissances générales. La copolymérisation peut être amorcée en masse ou en solution dans un solvant organique par des composés générateurs de radicaux libres. Par exemple, les copolymères statistiques ester boronique sont obtenus par les procédés connus de copolymérisation radicalaire, notamment contrôlée telle que la méthode dénommée polymérisation radicalaire contrôlée par transfert de chaîne réversible par addition-fragmentation (en anglais : Réversible Addition-Fragmentation Chain Transfer (RAFT)) et la méthode dénommée polymérisation radicalaire contrôlée par transfert d'atome (en anglais Atom Transfer Radical Polymerization (ARTP)). La polymérisation radicalaire conventionnelle et la télomérisation peuvent également être employées pour préparer les copolymères de l'invention (Moad, G.; Solomon, D. H., The Chemistry of Radical Polymerization. 2nd ed.; Elsevier Ltd: 2006; p 639 ; Matyaszewski, K.; Davis, T. P. Handbook of Radical Polymerization; Wiley-Interscience: Hoboken, 2002; p 936)).

Le copolymère statistique ester boronique est préparé selon un procédé qui comprend au moins une étape de polymérisation (a) dans laquelle on met en contact au moins :

i) un premier monomère M4 de formule générale (IV) telle que définie précédemment ;
ii) au moins un second monomère M5 de formule générale (V) telle que définie précédemment :
iii) au moins une source de radicaux libres.

Dans un mode de réalisation, le procédé peut comprendre en outre iv) au moins un agent de transfert de chaîne.
Les préférences et définitions décrites pour les formules générales (IV) et (V) s'appliquent également au procédé.
Les sources de radicaux et les agents de transferts sont ceux qui ont été décrits pour la synthèse de copolymères statistiques polydiols. Les préférences décrites pour les sources de radicaux et les agents de transferts s'appliquent également à ce procédé.

✓Propriétés des composés A2 copolymères statistiques poly(ester boronique)

Avantageusement, la chaîne formée par l'enchaînement des groupes $R_{10}$, M, $(R_8)_u$ avec u, un nombre entier égal à 0 ou 1, et X du monomère M4 de formule générale (IV) présente un nombre total d'atomes de carbone allant de 8 à 38, de préférence allant de 10 à 26.
Avantageusement, les chaînes latérales du copolymère statistique ester boronique ont une longueur moyenne supérieure à 8 atomes de carbone, de préférence allant de 11 à 16. Cette longueur de chaînes permet de solubiliser le copolymère statistique ester boronique dans un milieu hydrophobe. Par « longueur moyenne de chaîne latérale » on entend la longueur moyenne des chaînes latérales de chaque monomère constituant le copolymère. Les chaînes latérales issues du ou des éventuels monomères styréniques ne sont pas prises en compte dans le calcul des longueurs moyennes des chaînes latérales. L'homme du métier sait obtenir cette longueur moyenne en sélectionnant de manière appropriée les types et le ratio de monomères constituants le copolymère statistique ester boronique.
Avantageusement, le copolymère statistique ester boronique A2 a un pourcentage molaire de monomère de formule (IV) dans ledit copolymère allant de 0,25 à 30%, de préférence de 1 à 25%, encore mieux de 5 à 20%.
Avantageusement, le copolymère statistique ester boronique A2 a un pourcentage molaire de monomère de formule (IV) dans ledit copolymère allant de 0,25 à 30%, de préférence de 1 à 25% et un pourcentage molaire de monomère de formule (V) dans ledit copolymère allant de 70 à 99,75%, de préférence de 75 à 99%.
Avantageusement, le copolymère statistique ester boronique A2 a un pourcentage molaire de monomère(s) styrénique(s), de formule (IV), (V) et/ou (X), dans ledit copolymère allant de 2 à 50% molaire, préférentiellement de 3 à 40%, de manière plus préférée de 5 à 35% molaire.
Par « pourcentage molaire de monomère(s) styrénique(s) », on entend la somme des teneurs en chacun des monomères styréniques dans le copolymère statistique ester boronique A2, et les monomères styréniques peuvent être :

- de formule (IV) lorsque, dans la formule (IV) : u = 1, $R_9$ est H et $R_8$ représente un aryle en $C_6$-$C_{18}$ ou un aralkyle en $C_7$-$C_{24}$ et la double liaison du monomère M4 de formule (IV) est directement connectée au groupement aryle.
- de formule (V) lorsque, dans la formule (V) : $R_{12}$ représente H et $R_{13}$ est choisi parmi le groupe formé par un aryle en $C_6$-$C_{18}$ et un aryle en $C_6$-$C_{18}$ substitué par un groupement $R'_{13}$ avec $R'_{13}$ un groupe alkyle en $C_1$-$C_{25}$ et la double liaison du monomère M5 de formule (V) est directement connectée au groupement aryle.
  et/ou
- de formule (X), comme explicité ci-dessus.

Avantageusement, le copolymère statistique ester boronique a un degré de polymérisation moyen en nombre allant de 50 à 1500, de préférence de 50 à 800.
Avantageusement, le copolymère statistique ester boronique a un indice de polydispersité (Ip) allant de 1,04 à 3,54 ; de préférence allant de 1,10 à 3,10. Ces valeurs sont obtenues par chromatographie d'exclusion stérique en utilisant le tétrahydrofurane comme éluant et une calibration poly(méthacrylate de méthyle).
Avantageusement, le copolymère statistique ester boronique a une masse molaire moyenne en nombre allant de 10 000 à 200 000 g/mol de préférence de 25 000 à 100 000 g/mol. Ces valeurs sont obtenues par chromatographie d'exclusion stérique en utilisant le tétrahydrofurane comme éluant et une calibration poly(méthacrylate de méthyle)
Le composé A2, notamment le copolymère statistique ester boronique, présente la propriété de pouvoir réagir dans un milieu hydrophobe, notamment apolaire, avec un composé porteur de fonction(s) diol par une réaction de transestérification. Cette réaction de transestérification peut être représentée selon le schéma 9 suivant :

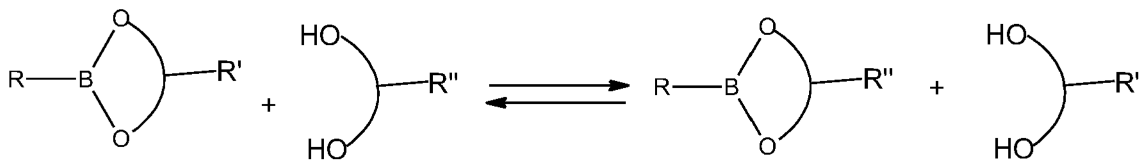

Schéma 9

Ainsi, lors d'une réaction de transestérification, il se forme un ester boronique de structure chimique différente de l'ester boronique de départ par échange des groupes hydrocarbonés symbolisé par

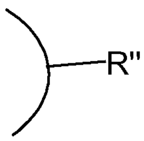 et 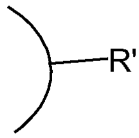

o *Composé exogène A5*

Selon l'invention, la composition d'additifs résulte du mélange d'au moins :

- un copolymère statistique polydiol A1,
- un copolymère statistique A2 comprenant au moins deux fonctions esters boroniques et pouvant s'associer avec ledit copolymère statistique polydiol A1 par au moins une réaction de transestérification,
- un composé exogène A5 choisi parmi ceux répondant à la formule (XI) :

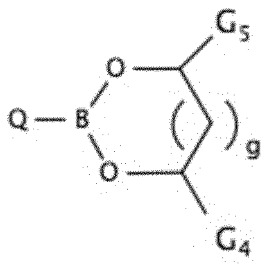

(XI)

dans laquelle :

- Q représente un groupement choisi parmi un groupement hydrocarboné comprenant de 1 à 30 atomes de carbone, éventuellement substituée par un ou plusieurs groupements choisis parmi : un hydroxyle, un groupement -OJ, -C(O)-O-J avec J un groupement hydrocarboné comprenant de 1 à 24 atomes de carbone,
- $G_4$, $G_5$, identiques ou différents, représentent des groupements choisis parmi un atome d'hydrogène, une chaîne hydrocarbonée comprenant de 1 à 24 atomes de carbone, un hydroxyle, un groupement -OJ, -C(O)-O-J avec J un groupement hydrocarboné comprenant de 1 à 24 atomes de carbone,
- g représente 0 ou 1,

le copolymère statistique polydiol A1 ayant une masse molaire moyenne en nombre allant de 5 000 à 400 000 g/mol, le pourcentage molaire de composé exogène A5 par rapport aux fonctions diol du copolymère statistique A1 allant de 0,025 à 5000%, et le ratio massique entre le copolymère A1 et le composé A2 (ratio A1/A2) allant de 0,1 à 10.

Par « groupement hydrocarboné comprenant de 1 à 30 atomes de carbone », on entend un groupement alkyle linéaire, ramifié ou cyclique comprenant de 1 à 30 atomes de carbone, alcényle linéaire, ramifié ou cyclique comprenant de 2 à 30 atomes de carbone, un groupement aryle comprenant de 6 à 30 atomes de carbone ou un groupement aralkyle comprenant de 7 à 30 atomes de carbone.

Par « groupement hydrocarboné comprenant de 1 à 24 atomes de carbone » on entend un groupe alkyle linéaire ou ramifié, comprenant de 1 à 24 atomes de carbone ou alcényle linéaire ou ramifié, comprenant de 2 à 24

atomes de carbone, un groupe aryle comprenant de 6 à 24 atomes de carbone, ou un groupe aralkyle comprenant de 7 à 24 atomes de carbone. De préférence, J comprend de 4 à 18 atomes de carbone, de préférence entre 6 et 12 atomes de carbone.

De façon avantageuse, le composé exogène A5 répond à la formule (XIA) ci-dessous :

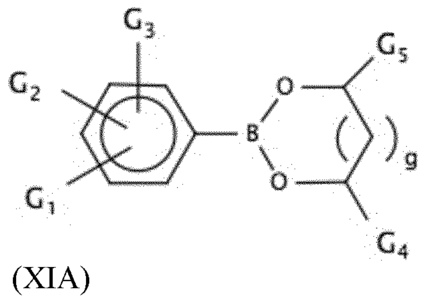

(XIA)

dans laquelle :

- $G_1$, $G_2$, $G_3$, $G_4$, $G_5$, identiques ou différents, représentent des groupements choisis parmi un atome d'hydrogène, un alkyle en C1-C24, un hydroxyle, un groupement -OJ, -C(O)-O-J avec J un alkyle en C1-C24,
- g représente 0 ou 1.

Selon ce mode de réalisation de l'invention, le pourcentage molaire de composé exogène A5, dans la composition d'additifs, par rapport aux fonctions diol du copolymère statistique A1 va de 0,025 à 5000%, de préférence de 0,1% à 1000%, de manière encore plus préférée de 0,5% à 500%, de manière encore plus préférée de 1% à 150%.

Par « composé exogène » on entend au sens de la présente invention un composé qui est rajouté à la composition d'additifs résultant du mélange d'au moins un copolymère statistique A1 polydiol et d'au moins un composé A2, notamment le copolymère statistique poly(ester boronique).

Dans un mode de réalisation, le composé exogène A5 est choisi parmi ceux répondant à la formule (XI B) :

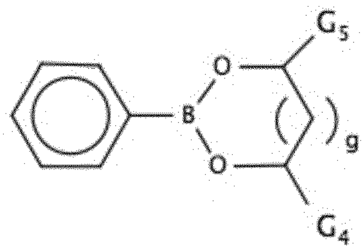

(XI B)

dans laquelle :

- $G_4$, $G_5$, identiques ou différents, représentent des groupements choisis parmi un atome d'hydrogène, un alkyle en C1-C24, un hydroxyle, un groupement -OJ, -C(O)-O-J avec J un alkyle en C1-C24,
- g représente 0 ou 1.

Dans un mode de réalisation, le composé exogène A5 a pour formule générale (XI B) dans laquelle :

- g est un nombre entier égal à 0 ou 1 ;
- $G_4$ et $G_5$ sont différents, l'un des groupes $G_4$ ou $G_5$ est H et l'autre groupe $G_4$ ou $G_5$ est une chaîne hydrocarbonée, de préférence un groupe alkyle linéaire, ayant de 1 à 24 atomes de carbone, de préférence entre 4 et 18 atomes de carbone, de préférence entre 6 et 12 atomes de carbone.

Dans un mode de réalisation, le fragment diol A6 de formule (XII), libéré in situ par le composé exogène A5 par réaction de transestérification, a une structure chimique différente de celle du composé diol A3 libéré in situ par le composé A2 par réaction de transestérification. Dans ce mode de réalisation, au moins un des substituants $G_4$, $G_5$ ou la valeur de l'indice (g) du composé exogène A5 de formule (XI) est différent respectivement des substituants $R_4$ et $R_5$

ou de la valeur de l'indice ($w_1$) ou des substituants $R_5$ et $R_7$ ou de la valeur de l'indice ($w_2$) du composé A2 diester boronique de formule (III) ou est différent respectivement des substituants $R_{10}$, Ru ou de la valeur de l'indice (t) du monomère (IV) du copolymère statistique poly(ester boronique) A2.

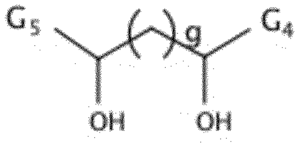

(XII)

Dans un autre mode de réalisation, le fragment diol A6 de formule (XII), libéré in situ par le composé exogène A5 par réaction de transestérification, a une structure chimique identique à celle du composé diol A3 libéré in situ par le composé A2 par réaction de transestérification. Dans ce mode de réalisation, les substituants $G_4$, $G_5$ et la valeur de l'indice (g) du composé exogène A5 de formule (XI) est identique respectivement aux substituants $R_4$ et $R_5$ et à la valeur de l'indice ($w_1$) ou aux $R_5$ et $R_7$ et à la valeur de l'indice ($w_2$) du composé A2 diester boronique de formule (III) ou est identique respectivement aux substituants $R_{10}$, Ru et à la valeur de l'indice (t) du monomère (IV) du copolymère statistique poly(ester boronique) A2.

Selon sa température d'utilisation, la composition d'additifs résultant du mélange d'au moins un copolymère statistique polydiol A1, d'au moins un composé A2, notamment un copolymère statistique A2, comprenant au moins deux fonctions esters boroniques et pouvant s'associer avec ledit copolymère statistique polydiol A1 par une réaction de transestérification, et d'un ajout d'au moins un composé exogène A5 tel que défini ci-dessus, peut comprendre en outre un composé A3 diol libéré in situ. En outre, selon sa température d'utilisation, cette même composition peut comprendre un composé A6 diol libéré in situ.

Par « diol libéré in situ », on entend au sens de la présente invention le composé porteur d'une fonction diol, ce composé étant produit dans la composition d'additifs lors de l'échange des groupes hydrocarbonés du composé ester boronique A2, notamment du copolymère statistique poly(ester boronique), et/ou du composé exogène A5 pendant la réaction de transestérification. Le polymère statistique A1 polydiol n'est pas un diol libéré in situ au sens de la présente invention.

Les composés de formule (VI) sont disponibles commercialement auprès des fournisseurs suivants : Sigma-Aldrich®, Alfa Aesar® et TCI®.

✓<u>Caractéristiques des nouvelles compositions d'additifs de l'invention</u>

Les compositions d'additifs de l'invention résultant du mélange d'au moins un copolymère statistique polydiol A1 tel que défini ci-dessus, d'au moins un composé A2 tel que défini précédemment, notamment d'au moins un copolymère statistique poly(ester boronique) tel que défini ci-dessus, et d'au moins un composé exogène A5 tel que défini ci-dessus présentent des propriétés rhéologiques très variées en fonction de la température et selon la proportion des composés A1, A2 et A5 utilisés.

Les copolymères statistiques polydiols A1 et les composés A2 tels que définis ci-dessus, présentent l'avantage d'être associatifs et d'échanger des liens chimiques de manière thermoréversible, notamment dans un milieu hydrophobe, notamment un milieu hydrophobe apolaire.

Dans certaines conditions, les copolymères statistiques polydiols A1 et les composés A2 tels que définis ci-dessus peuvent être réticulés.

Les copolymères statistiques polydiols A1 et les composés A2 présentent aussi l'avantage d'être échangeables.

Par « associatif », on entend qu'il s'établit des liens chimiques covalents de type ester boronique entre les copolymères statistiques polydiols A1 et les composés A2 comprenant au moins deux fonctions esters boroniques, notamment avec le copolymère statistique poly(ester boronique). Suivant la fonctionnalité des polydiols A1 et des composés A2 et suivant la composition des mélanges, la formation des liens covalents entre les polydiols A1 et les composés A2 pourra amener ou non à la formation d'un réseau polymérique tridimensionnel.

Par « lien chimique », on entend un lien chimique covalent de type ester boronique.

Par « échangeable », on entend que les composés sont capables d'échanger des liens chimiques entre eux sans que le nombre total et la nature des fonctions chimiques soient modifiés. La réaction chimique d'échange (transestérification) est illustrée dans le schéma réactionnel 10 suivant :

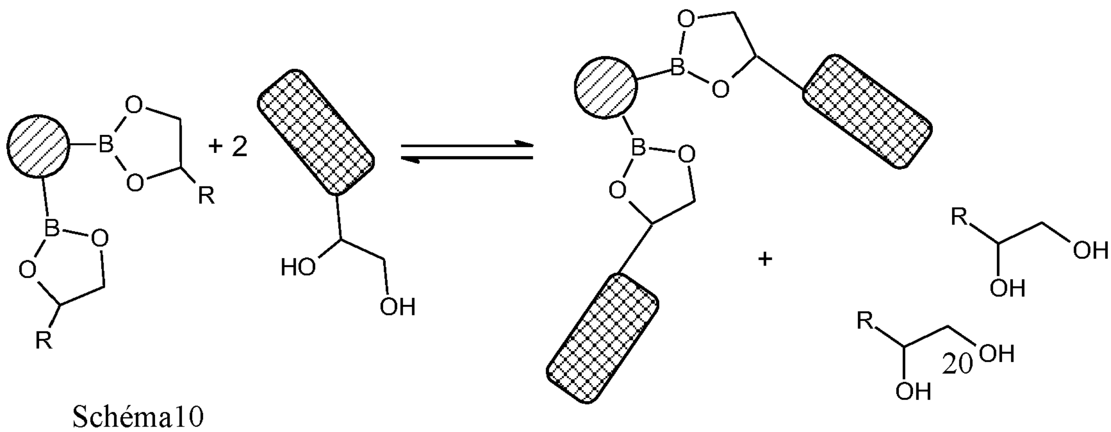

Schéma10

avec :

- R un groupe chimique du composé A2,
- le rond hachuré symbolise le reste de la structure chimique du composé A2,
- le rectangle quadrillé symbolise le reste de la structure chimique du polymère statistique polydiol A1.

Les liens esters boroniques des composés A2, les liens esters boroniques formés par réaction de transestérification entre les diols des composés A1 et les composés exogènes A5, ainsi que les liens esters boroniques formés par association des copolymères statistiques polydiols A1 et des composés A2 peuvent s'échanger avec les fonctions diols portées par les composés A3 libérés in situ et avec des fonctions diols A6 libérées par les composés exogènes A5 pour former de nouveaux esters boroniques et de nouvelles fonctions diols sans que le nombre total de fonctions esters boroniques et de fonctions diols ne soit affecté. Cet autre processus d'échange de liens chimiques s'effectue par réaction de métathèse, via des échanges successifs des fonctions ester boronique en présence de diols. Un autre processus d'échange de liens chimiques est illustré en figure 3, dans laquelle on peut observer que le copolymère statistique polydiol A1-1, qui était associé au polymère A2-1, a échangé deux liens esters boroniques avec le copolymère statistique ester boronique A2-2. Le copolymère statistique polydiol A1-2, qui était en associé au polymère A2-2, a échangé deux liens esters boroniques avec le copolymère statistique ester boronique A2-1 ; le nombre total de lien ester boronique dans la composition étant inchangé et est égal à 4. Le copolymère A1-1 est alors associé avec le polymère A2-2. Le copolymère A1-2 est alors avec le polymère A2-1. Le copolymère A2-1 a été échangé avec le polymère A2-2.

Par « réticulé », on entend un copolymère sous forme d'un réseau obtenu par l'établissement de ponts entre les chaînes macromoléculaires du copolymère. Ces chaînes reliées entre elles sont pour la plupart distribuées dans les trois dimensions de l'espace. Un copolymère réticulé forme un réseau tridimensionnel. Dans la pratique, la formation d'un réseau de copolymère est assurée par un test de solubilité. On peut s'assurer qu'un réseau de copolymères a été formé en plaçant le réseau de copolymère dans un solvant connu pour dissoudre les copolymères non réticulés de même nature chimique. Si le copolymère gonfle au lieu de se dissoudre, l'homme du métier sait qu'un réseau a été formé. La figure 4 illustre ce test de solubilité.

Par « réticulable » on entend un copolymère susceptible d'être réticulé.

Par « réticulé de manière réversible » on entend un copolymère réticulé dont les ponts sont formés par une réaction chimique réversible. La réaction chimique réversible peut se déplacer dans un sens ou dans un autre, entraînant un changement de structure du réseau de polymère. Le copolymère peut passer d'un état initial non réticulé à un état réticulé (réseau tridimensionnel de copolymères) et d'un état réticulé à un état initial non réticulé. Dans le cadre de la présente invention, les ponts qui se forment entre les chaînes de copolymères sont labiles. Ces ponts peuvent se former ou s'échanger grâce à une réaction chimique qui est réversible. Dans le cadre de la présente invention, la réaction chimique de réticulation réversible est une réaction de transestérification entre des fonctions diols d'un copolymère statistique (copolymère A1) et des fonctions ester boronique d'un agent de réticulation (composé A2). Les ponts formés sont des liaisons de type ester boronique. Ces liaisons ester boronique sont covalentes et labiles du fait de la réversibilité de la réaction de transestérification.

Par « réticulé de manière thermoréversible », on entend un copolymère réticulé grâce à une réaction réversible dont le déplacement dans un sens ou dans l'autre sens est contrôlé par la température. Le mécanisme de réticulation thermoréversible de la composition de l'invention est présenté schématiquement en figure 5. A faible température, le copolymère polydiol A1 (symbolisé par le copolymère portant des fonctions A sur la figure 5) n'est pas ou peu réticulé par les composés esters boroniques A2 (symbolisé par le composé portant des fonctions B sur la figure 5). Quand la

température augmente, les fonctions diol du copolymère A1 réagissent avec les fonctions ester boronique du composé A2 par une réaction de transestérification. Les copolymères statistiques polydiols A1 et les composés A2 comprenant au moins deux fonctions esters boroniques se lient alors ensemble et peuvent s'échanger. Suivant la fonctionnalité des polydiols A1 et des composés A2 et suivant la composition des mélanges, il peut se former un gel dans le milieu, notamment lorsque le milieu est apolaire. Lorsque la température diminue à nouveau, les liaisons esters boroniques entre les copolymères statistiques polydiols A1 et les composés A2 se rompent, et le cas échéant, la composition perd son caractère gélifié.

La quantité de liaisons ester boronique (ou lien ester boronique) pouvant s'établir entre les copolymères statistiques polydiols A1 et les composés A2 est ajustée par l'homme du métier au moyen d'une sélection appropriée du copolymère statistique polydiol A1, du composé A2 et de la composition du mélange.

En outre, l'homme du métier sait sélectionner la structure du composé A2 en fonction de la structure du copolymère statistique A1. De préférence, lorsque dans le copolymère statistique A1 comprenant au moins un monomère M1 dans lequel y=1, alors le composé A2 de formule générale (III) ou le copolymère A2 comprenant au moins un monomère M4 de formule (IV) sera choisi de de préférence avec $w_1$= 1, $w_2$=1 et t=1, respectivement.

En contrôlant le taux d'association du copolymère statistique polydiol A1 et du composé A2, notamment du copolymère statique poly(ester boronique), on module la viscosité et le comportement rhéologique de cette composition. Lorsqu'il est présent, le composé exogène A5 permet de moduler la viscosité de cette composition en fonction de la température et selon l'utilisation désirée.

Pour que le système fonctionne de façon associative, le composé A1 doit être présent dans la composition sous forme de diol libre. Si A1 était porteur de groupes protecteurs sur les diols, ces groupes protecteurs doivent donc être retirés.

Selon un mode de réalisation préféré, le composé A1 est introduit dans la composition sous forme de diol libre et le composé A2 est introduit dans la composition sous forme d'acide boronique.

Dans un mode de réalisation préférée de l'invention, le radical diol du composé exogène A5 est de même nature chimique que le composé diol A3 libéré in situ par réaction de transestérification entre le copolymère statistique polydiol A1 et le composé A2, notamment le copolymère statistique poly(ester boronique). Selon ce mode de réalisation, la quantité totale de diols libres est sensiblement égale à la quantité de composés diols libérés in situ, le composé A5 étant susceptible de comprendre une part minoritaire de diol libre. Par « diols libres », on entend les fonctions diol qui sont susceptibles de pouvoir former un lien chimique de type ester boronique par réaction de transestérification. Par « quantité totale de diols libres », on entend au sens de la présente demande, le nombre total de fonctions diol susceptibles de pouvoir former un lien chimique de type ester boronique par transestérification.

La quantité de diols libérés in situ dans le cadre des réactions de transestérification entre A1 et A2 est égale à la somme du nombre de fonctions ester boronique reliant les copolymères A1 et A2 et du nombre de fonctions ester boronique reliant le copolymère A1 et la partie ester boronique de A5.

L'homme du métier sait sélectionner la structure chimique et la quantité de composés exogènes A5 qu'il ajoute à la composition d'additifs en fonction du pourcentage molaire de fonction ester boronique du composé A2, notamment en fonction du copolymère statistique poly(ester boronique), et du nombre de fonctions diols du copolymère polydiol A1, pour moduler les comportements rhéologique de la composition.

Avantageusement, la teneur en copolymère statistique A1 dans la composition va de 0,1% à 50,0% en poids par rapport au poids total de la composition, de préférence va de 0,25% à 40% en poids par rapport au poids total de la composition finale, de manière plus préférée de 1% à 30% en poids par rapport au poids total de la composition finale.

Avantageusement, la teneur en composé A2 dans la composition va de 0,1% à 50,0% en poids par rapport au poids total de la composition, de préférence va de 0,25% à 40% en poids par rapport au poids total de la composition finale, de manière plus préférée de préférence de 0,5% à 30% en poids par rapport au poids total de la composition finale.

Dans un mode de réalisation, la teneur en copolymère statistique A1 dans la composition va de 0,5 à 50,0% en poids par rapport au poids total de la composition et la teneur en composé A2, notamment en copolymère statistique ester boronique dans la composition va de 0,5% à 50,0% en poids par rapport au poids total de la composition.

Le ratio massique entre le copolymère statistique polydiol A1 et le composé A2 (ratio A1/A2) dans la composition va de 0,005 à 200, de préférence de 0,05 à 20, de manière encore plus préférée de 0,1 à 10.

Le pourcentage molaire de composé exogène A5 dans la composition d'additifs va de 0,025% à 5000%, de préférence va de 0,1 % à 1000%, de manière plus préférée de 0,5 à 500%, de manière encore plus préférée de 1% à 150% par rapport aux fonctions diol du copolymère statistique polydiol A1.

Dans un mode de réalisation, la composition de l'invention se présente sous forme d'une composition-mère. Par « composition-mère » on entend, une composition dont l'homme du métier pourra faire des solutions-filles par prélèvement d'une certaine quantité de solution mère complétée par l'apport d'une quantité nécessaire de diluant (solvant ou autre) pour obtenir une concentration souhaitée. Une composition-fille est donc obtenue par dilution d'une composition-mère.

Un milieu hydrophobe peut être un solvant, une huile minérale, une huile naturelle, une huile synthétique.

Dans un mode de réalisation, la composition de l'invention peut comprendre en outre au moins un additif choisi parmi le groupe formé par les thermoplastiques, les élastomères, les élastomères thermoplastiques, les polymères thermodurcissables, les pigments, les colorants, les charges, les plastifiants, les fibres, les antioxydants, les additifs pour lubrifiants, les agents de compatibilité, les agents anti-mousses, les additifs dispersants, les promoteurs d'adhérences et les stabilisants.

Autre additif - composé exogène A4

Selon un mode de réalisation, la composition d'additifs comprend en outre, en plus des composés A1, A2 et A5, au moins un composé exogène A4 choisi parmi les 1,2-diols et les 1,3-diols. De tels composés, ainsi que leurs procédés d'utilisation, sont décrits de façon détaillée dans la demande WO2016/113229.
Le composé exogène A4 peut avoir pour formule générale (VI) :

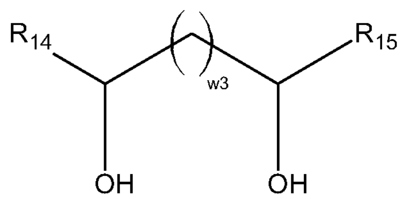

(VI)

dans laquelle :

w3 est un nombre entier égal à 0 ou 1,
$R_{14}$ et Ris, identiques ou différents, sont choisis parmi le groupe formé par l'hydrogène et une chaîne hydrocarbonée ayant de 1 à 24 atomes de carbone, de préférence entre 4 et 18 atomes de carbone, de préférence entre 6 et 12 atomes de carbone.

Dans les compositions de l'invention, le composé exogène A4 peut être mis en oeuvre dans les mêmes conditions que décrit dans WO2016/113229.
Les composés de formule (VI) sont disponibles commercialement auprès des fournisseurs suivants : Sigma-Aldrich®, Alfa Aesar® et TCI®.

✓ Procédé de préparation des nouvelles compositions d'additifs de l'invention

Les nouvelles compositions d'additifs de l'invention sont préparées par des moyens bien connus de l'homme du métier. Par exemple, il suffit à l'homme du métier notamment de :

- prélever une quantité voulue d'une solution comprenant le copolymère statistique polydiol A1 tel que défini ci-dessus ;
- prélever une quantité voulue d'une solution comprenant le composé A2 tel que défini ci-dessus ; notamment une quantité voulue d'une solution comprenant le copolymère statistique poly(ester boronique) tel que défini précédemment ; et
- prélever une quantité voulue d'une solution comprenant le composé exogène A5 tel que défini ci-dessus
- mélanger les solutions prélevées, soit simultanément, soit séquentiellement, pour obtenir la composition de l'invention.

L'ordre d'ajout des composés n'a pas d'influence dans la mise en oeuvre du procédé de préparation de la composition d'additifs.
L'homme du métier sait aussi ajuster les différents paramètres de la composition de l'invention pour obtenir soit une composition dans laquelle le copolymère statistique polydiol A1 et le composé A2, notamment le copolymère statistique ester boronique, sont associés soit une composition dans laquelle le copolymère statistique polydiol A1 et le composé A2, notamment le copolymère statistique ester boronique, sont réticulés et pour en moduler le taux d'association ou le taux de réticulation pour une température d'utilisation donnée. Par exemple, l'homme du métier sait ajuster notamment:

- le pourcentage molaire de monomère M1 portant des fonctions diols dans le copolymère statistique polydiol A1 ;

- la teneur en monomère styrénique M3 dans le copolymère statistique polydiol A1 ;
- le pourcentage molaire de monomère M4 portant des fonctions ester boronique dans le copolymère statistique ester boronique A2 ;
- la longueur moyenne des chaînes latérales du copolymère statistique polydiol A1 ;
- la longueur moyenne des chaînes latérales du copolymère statistique ester boronique A2 ;
- la longueur du monomère M4 du copolymère statistique ester boronique A2 ;
- la teneur en monomère styrénique M4 de formule (IV), ou M5 de formule (V) ou M3 de formule (X) dans le copolymère statistique ester boronique A2 ;
- la longueur du composé diester boronique A2 ;
- le degré de polymérisation moyen en nombre des copolymères statistiques polydiol A1 et des copolymères statistiques ester boronique A2 ;
- le pourcentage massique du copolymère statistique polydiol A1 ;
- le pourcentage massique du composé diester boronique A2 ;
- le pourcentage massique du copolymère statistique ester boronique A2 ; et le cas échéant :
- la quantité molaire du composé exogène A5 par rapport aux fonctions diol du du copolymère statistique polydiol A1,
- la nature chimique du composé exogène A5 ;
- le pourcentage molaire de composé exogène A5 ;

✓ Utilisation des nouvelles compositions de l'invention

Les compositions de l'invention peuvent être utilisées dans tous les milieux dont la viscosité varie en fonction de la température. Les compositions de l'invention permettent d'épaissir un fluide et de moduler la viscosité en fonction de la température d'utilisation. La composition d'additifs selon l'invention peut être utilisée dans les domaines aussi variés que la récupération améliorée du pétrole, l'industrie papetière, les peintures, les additifs alimentaires, la formulation cosmétique ou pharmaceutique.

➢ Composition lubrifiante selon l'invention

Un autre objet de la présente invention concerne une composition lubrifiante résultant du mélange d'au moins :

- une huile lubrifiante,
- un copolymère statistique polydiol A1 tel que défini précédemment,
- un copolymère statistique A2, tel que défini précédemment, comprenant au moins deux fonctions esters boroniques et pouvant s'associer avec ledit copolymère statistique polydiol A1 par au moins une réaction de transestérification,
- un composé exogène A5, choisi parmi les esters boroniques de formule (XI), tel que défini précédemment.

Les préférences et définitions décrites pour les formules générales (I), (I-A), (I-B), (II-A), (II-B), (X) s'appliquent également au copolymère statistique A1 polydiol utilisée dans les compositions lubrifiantes de l'invention.

Les préférences et définitions décrites pour les formules générales (IV) et (V) s'appliquent également au copolymère statistique A2 ester boronique utilisé dans les compositions lubrifiantes de l'invention.

Les préférences et définitions décrites pour les formules générales (XI) et (XIA) s'appliquent également au composé exogène A5 utilisé dans les compositions lubrifiantes de l'invention.

Les compositions lubrifiantes selon l'invention ont un comportement inversé vis-à-vis d'une modification de la température par rapport au comportement de l'huile de base et des additifs rhéologiques de type polymère de l'art antérieur et présentent l'avantage que ce comportement rhéologique peut être modulé en fonction de la température d'utilisation. Contrairement à l'huile de base qui se fluidifie lorsque la température augmente, les compositions de la présente invention présentent l'avantage de s'épaissir lorsque la température augmente. La formation des liaisons covalentes réversibles permet d'augmenter (de façon réversible) la masse molaire des polymères et limite donc la chute de la viscosité de l'huile de base à hautes températures. L'ajout supplémentaire de composés diols permet de contrôler le taux de formation de ces liaisons réversibles. De manière avantageuse, la viscosité de la composition lubrifiante est ainsi contrôlée et dépend moins des fluctuations de température. En outre, pour une température donnée d'utilisation, il est possible de moduler la viscosité de la composition lubrifiante et son comportement rhéologique en jouant sur la quantité de composés ester boronique A5 ajoutés dans la composition lubrifiante.

◦ ***Huile lubrifiante***

Par « huile » on entend un corps gras liquide à température ambiante (25°C) et pression atmosphérique (760 mm de Hg soit $10^5$ Pa).

Par « huile lubrifiante » on entend une huile qui atténue le frottement entre deux pièces en mouvements en vue de faciliter le fonctionnement de ces pièces. Les huiles lubrifiantes peuvent être d'origine naturelle, minérale ou synthétique.

Les huiles lubrifiantes d'origine naturelle peuvent être des huiles d'origine végétale ou animale, de préférence des huiles d'origine végétale telles que l'huile de colza, l'huile de tournesol, l'huile de palme, l'huile de coprah...

Les huiles lubrifiantes d'origine minérale sont d'origine pétrolière et sont extraites de coupes pétrolières provenant de la distillation atmosphérique et sous vide du pétrole brut. La distillation peut être suivie d'opérations de raffinage telles que l'extraction au solvant, le désalphatage, le déparaffinage au solvant, l'hydrotraitement, l'hydrocraquage, l'hydroisomérisation, l'hydrofinition... A titre illustratif, on peut citer les huiles de base minérales paraffiniques telle que l'huile Bright Stock Solvent (BSS), les huiles de base minérales napthéniques, les huiles minérales aromatiques, les bases minérales hydroraffinées dont l'indice de viscosité est d'environ 100, les bases minérales hydrocraquées dont l'indice de viscosité est compris entre 120 et 130, les bases minérales hydroisomérisées dont l'indice de viscosité est compris entre 140 et 150.

Les huiles lubrifiantes d'origine synthétique (ou base synthétiques) proviennent comme leur nom l'indique de la synthèse chimique telles que l'addition d'un produit sur lui-même ou polymérisation, ou l'addition d'un produit sur un autre comme l'estérification, l'alkylation, la fluoration, etc., de composants provenant de la pétrochimie, la carbochimie, et de la chimie minérale tels que : oléfines, aromatiques, alcools, acides, composés halogénés, phosphorés, siliciés, etc. A titre illustratif, on peut citer :

- les huiles synthétiques à base d'hydrocarbures de synthèse telles que les polyalphaoléfines (PAO), les polyoléfines internes (PIO), les polybutènes et polyisobutènes (PIB), les dialkylbenènes, les polyphényles alkylés ;
- les huiles synthétiques à base d'esters telles que les esters de diacides, les esters de néopolyols ;
- les huiles synthétiques à base de polyglycols telles que les monoalkylèneglycols, les polyalkylèneglycols et les monoéthers de polyalkylèneglycols ;
- les huiles synthétiques à base d'ester-phosphates ;
- les huiles synthétiques à base de dérivés siliciés telles que les huiles silicones ou les polysiloxanes.

Les huiles lubrifiantes qui peuvent être utilisées dans la composition de l'invention peuvent être choisies parmi n'importe quelles huiles des groupes I à V spécifiées dans les directives de l'API (Base Oil Interchangeability Guidelines de l'American Petroleum Institute (API)) (ou leurs équivalents selon la classification ATIEL (Association Technique de l'Industrie Européenne des Lubrifiants) telles que résumées ci-dessous :

| | Teneur en composés saturés* | Teneur en soufre** | Indice de viscosité (VI)*** |
|---|---|---|---|
| Groupe I Huiles minérales | < 90 % | > 0.03 % | 80 ≤ VI < 120 |
| Groupe II Huiles hydrocraquées | ≥ 90 % | ≤ 0.03 % | 80 ≤ VI < 120 |
| Groupe III Huiles hydrocraquées ou hydro-isomérisées | ≥ 90 % | ≤ 0.03 % | ≥ 120 |
| Groupe IV | (PAO) Polyalphaoléfines | | |
| Groupe V | Esters et autres bases non incluses dans bases groupes I à IV | | |
| * mesuré selon la norme ASTM D2007<br>** mesuré selon les normes ASTM D2622, ASTM D4294, ASTM D4927 et ASTM D3120<br>*** mesuré selon la norme ASTM D2270 | | | |

Les compositions de l'invention peuvent comprendre une ou plusieurs huiles lubrifiantes. L'huile lubrifiante ou le mélange d'huile lubrifiante est l'ingrédient majoritaire dans la composition lubrifiante. On parle alors d'huile de base lubrifiante. Par ingrédient majoritaire, on entend que l'huile lubrifiante ou le mélange d'huiles lubrifiantes représente au moins 51 % en poids par rapport au poids total de la composition.

De préférence, l'huile lubrifiante ou le mélange d'huiles lubrifiantes représente au moins 70 % en poids par rapport au poids total de la composition.

Dans un mode de réalisation de l'invention, l'huile lubrifiante est choisi dans le groupe formés par les huiles du groupe I, du groupe II, du groupe III, du groupe IV, du groupe V de la classification API et l'un de leur mélange. De préférence, l'huile lubrifiante est choisie parmi le groupe formé par les huiles du groupe III, du groupe IV, du groupe V de la classification API et leur mélange. De préférence, l'huile lubrifiante est une huile du groupe III de la classification API.

L'huile lubrifiante a une viscosité cinématique à 100°C mesurée selon la norme ASTM D445 allant de 2 à 150 cSt, de manière préférée allant de 2 à 15 cSt.

◦ **Additifs fonctionnels**

Dans un mode de réalisation, la composition de l'invention peut comprendre en outre un ou plusieurs additifs fonctionnels choisis parmi le groupe formé par les détergents, les additifs anti-usure, les additifs extrême pression, les antioxydants, les polymères améliorant l'indice de viscosité, les améliorants de point d'écoulement, les anti-mousse, les épaississants, les additifs anticorrosion, les dispersants, les modificateurs de frottements et leurs mélanges.

Le ou les additifs fonctionnels qui sont ajoutés à la composition de l'invention sont choisis en fonction de l'utilisation finale de la composition lubrifiante. Ces additifs peuvent être introduits de deux façons différentes :

- soit chaque additif est ajouté isolément et séquentiellement dans la composition,
- soit l'ensemble des additifs est ajouté simultanément dans la composition, les additifs sont dans ce cas généralement disponibles sous forme d'un paquet, appelé paquet d'additifs.

L'additif fonctionnel ou les mélanges d'additifs fonctionnels, lorsqu'ils sont présents, représentent de 0,1 à 10% en poids par rapport au poids total de la composition.

✓ **Les détergents** :

Ces additifs réduisent la formation de dépôts à la surface des pièces métalliques par dissolution des produits secondaires d'oxydation et de combustion. Les détergents utilisables dans les compositions lubrifiantes selon la présente invention sont bien connus de l'homme de métier. Les détergents communément utilisés dans la formulation de compositions lubrifiantes sont typiquement des composés anioniques comportant une longue chaîne hydrocarbonée lipophile et une tête hydrophile. Le cation associé est typiquement un cation métallique d'un métal alcalin ou alcalino-terreux. Les détergents sont préférentiellement choisis parmi les sels de métaux alcalins ou alcalino-terreux d'acides carboxyliques, sulfonates, salicylates, naphténates, ainsi que les sels de phénates. Les métaux alcalins et alcalino-terreux sont préférentiellement le calcium, le magnésium, le sodium ou le baryum. Ces sels métalliques peuvent contenir le métal en quantité approximativement stoechiométrique ou bien en excès (en quantité supérieure à la quantité stoechiométrique). Dans ce dernier cas, on a affaire à des détergents dits surbasés. Le métal en excès apportant le caractère surbasé au détergent se présente sous la forme de sels métalliques insolubles dans l'huile, par exemple carbonate, hydroxyde, oxalate, acétate, glutamate, préférentiellement carbonate.

✓ **Les additifs anti-usure et les additifs extrême pression :**

Ces additifs protègent les surfaces en frottement par formation d'un film protecteur adsorbé sur ces surfaces. Il existe une grande variété d'additifs anti-usure et extrême pression. A titre illustratifs on peut citer les additifs phosphosoufrés comme les alkylthiophosphates métalliques, en particulier les alkylthiophosphates de zinc, et plus spécifiquement les dialkyldithiophosphates de zinc ou ZnDTP, les phosphates d'amines, les polysulfures, notamment les oléfines soufrées et les dithiocarbamates métalliques.

✓ **Les antioxydants** :

Ces additifs retardent la dégradation de la composition. La dégradation de la composition peut se traduire par la formation de dépôts, la présence de boues, ou une augmentation de la viscosité de la composition. Les antioxydants agissent comme inhibiteurs radicalaires ou destructeurs d'hydropéroxydes. Parmi les antioxydants couramment employés on trouve les antioxydants de type phénolique ou aminé.

✓ **Les anticorrosions** :

Ces additifs couvrent la surface d'un film qui empêche l'accès de l'oxygène à la surface du métal. Ils peuvent parfois neutraliser les acides ou certains produits chimiques pour éviter la corrosion du métal. A titre illustratif, on peut citer par exemple le dimercaptothiadiazole (DMTD), les benzotriazoles, les phosphites (capture du soufre libre).

✓ **Les polymères améliorant l'indice de viscosité :**

Ces additifs permettent de garantir une bonne tenue à froid et une viscosité minimale à haute température de

la composition. A titre illustratif, on peut citer par exemple les esters polymères, les oléfines copolymères (OCP), ou et les polyméthacrylates d'alkyle (PMA).

✓ **Les améliorants de point d'écoulement :**

Ces additifs améliorent le comportement à froid des compositions, en ralentissant la formation de cristaux de paraffine. Ce sont par exemple des polyméthacrylates d'alkyle, des polyacrylates, des polyarylamides, des polyalkylphénols, des polyalkylnaphtalènes et des polystyrènes alkylés.

✓ **Les anti-mousse :**

Ces additifs ont pour effet de contrer l'effet des détergents. A titre illustratif, on peut citer les polyméthylsiloxanes et les polyacrylates.

✓ **Les épaississants :**

Les épaississants sont des additifs utilisés surtout pour la lubrification industrielle et permettent de formuler des lubrifiants de plus forte viscosité que les compositions lubrifiantes pour moteur. A titre illustratif, on peut citer les polysiobutènes ayant une masse molaire en poids de 10 000 à 100 000 g/mol.

✓ **Les dispersants :**

Ces additifs assurent le maintien en suspension et l'évacuation des contaminants solides insolubles constitués par les produits secondaires d'oxydation qui se forment au cours de l'utilisation de la composition. A titre illustratif, on peut citer par exemple les succinimides, les PIB (polyisobutène) succinimides et les bases de Mannich.

✓ **Les modificateurs de frottements ;**

Ces additifs améliorent le coefficient de frottement de la composition. A titre illustratif, on peut citer le dithiocarbamate de molybdène, les amines ayant au moins une chaîne hydrocarbonée d'au moins 16 atomes de carbone, les esters d'acides gras et de polyols tels que les esters d'acides gras et de glycérol, en particulier le glycérol monooléate.

✓ **Procédé de préparation des compositions lubrifiantes de l'invention**

Les compositions lubrifiantes de l'invention sont préparées par des moyens bien connus de l'homme du métier. Par exemple, il suffit à l'homme du métier notamment de:

- prélever une quantité voulue d'une solution comprenant le copolymère statistique polydiol A1 tel que défini ci-dessus, notamment celui résultant de la copolymérisation d'au moins un monomère de formule (I) avec au moins un monomère de formule (II-A) et au moins un monomère de formule (II-B) ;
- prélever une quantité voulue d'une solution comprenant le copolymère statistique A2 poly(ester boronique) tel que défini précédemment ;
- prélever une quantité voulue d'une solution comprenant le composé exogène A5 tel que défini ci-dessus
- mélanger soit simultanément, soit séquentiellement les solutions prélevées dans une huile de base lubrifiante, pour obtenir la composition lubrifiante de l'invention.

L'ordre d'ajout des composés n'a pas d'influence dans la mise en oeuvre du procédé de préparation de la composition lubrifiante.

✓ **Propriétés des compositions lubrifiantes selon l'invention**

Les compositions lubrifiantes de l'invention résultent du mélange de polymères associatifs qui présentent la propriété d'augmenter la viscosité de l'huile lubrifiante par des associations. Les compositions lubrifiantes selon l'invention présentent l'avantage que ces associations ou réticulation sont thermoréversibles et éventuellement que le taux d'association ou de réticulation peut être contrôlé grâce à l'ajout d'un composé ester boronique A5 supplémentaire.
L'homme du métier sait ajuster les différents paramètres des différents constituants de la composition pour obtenir une composition lubrifiante dont la viscosité augmente lorsque la température augmente et pour en moduler sa viscosité et son comportement rhéologique.

La quantité de liaisons ester boronique (ou lien ester boronique) pouvant s'établir entre les copolymères statistiques polydiols A1 et les composés A2, notamment le copolymère statistique ester boronique A2, est ajustée par l'homme du métier au moyen d'une sélection appropriée du copolymère statistique polydiol A1, du composé A2, notamment le copolymère statistique ester boronique A2, du composé exogène A5, et notamment du pourcentage molaire de composé exogène A5.
En outre, l'homme du métier sait sélectionner la structure du composé A2, notamment du copolymère statistique ester boronique, en fonction de la structure du copolymère statistique A1. De préférence, lorsque dans le copolymère statistique A1, comprenant au moins un monomère M1 dans lequel y=1, alors le composé A2 de formule générale (III) ou le copolymère A2 comprenant au moins un monomère M4 de formule (IV) sera choisi de préférence avec wi= 1, $w_2$=1 et t=1, respectivement.
Par ailleurs, l'homme du métier sait ajuster notamment :

- le pourcentage molaire de monomère M1 portant des fonctions diols dans le copolymère statistique polydiol A1 ;
- le pourcentage molaire de monomère M3 de formule (X) dans le copolymère statistique polydiol A1, notamment le pourcentage molaire de styrène ;
- le pourcentage molaire de monomère M4 portant des fonctions ester boronique dans le copolymère statistique ester boronique A2,
- la longueur moyenne des chaînes latérales du copolymère statistique polydiol A1 ;
- la longueur moyenne des chaînes latérales du copolymère statistique ester boronique A2,
- la longueur du monomère M4 du copolymère statistique ester boronique A2,
- le degré de polymérisation moyen des copolymères statistique polydiol A1, et des copolymères statistiques esters boroniques A2,
- le pourcentage massique du copolymère statistique polydiols A1,
- le pourcentage massique du copolymère statistique ester boronique A2,
- le pourcentage molaire de composé exogène A5 par rapport aux fonctions diol du copolymère statistique polydiols A1,

Avantageusement, la teneur en copolymère statistique A1 dans la composition lubrifiante va de 0,25% à 20% en poids par rapport au poids total de la composition lubrifiante, de préférence de 1% à 10% en poids par rapport au poids total de la composition lubrifiante.
Avantageusement, la teneur en composé A2, notamment la teneur en copolymère statistique ester boronique, va de 0,25% à 20% en poids par rapport au poids total de la composition lubrifiante, de préférence de préférence de 0,5 à 10% en poids par rapport au poids total de la composition lubrifiante.
Le ratio massique (ratio A1/A2) entre le composé statistique polydiol A1 et le composé A2, notamment le copolymère statistique ester boronique, va de 0, 1 à 10, de manière plus préférée de 0,2 à 5.
Dans un mode de réalisation, la somme des masses du copolymère statistique A1 et du composé A2, notamment du copolymère statistique ester boronique, va de 0,5 à 20% par rapport à la masse totale de la composition lubrifiante, de préférence de 4% à 15% par rapport à la masse totale de la composition lubrifiante et la masse d'huile lubrifiante va de 60% à 99 % par rapport à la masse totale de la composition lubrifiante.
Pour les applications moteur, avantageusement, la somme des masses du copolymère statistique A1 et du composé A2 représente de 0,1 à 15%, par rapport à la masse totale de la composition lubrifiante.
Pour les applications transmission, avantageusement, la somme des masses du copolymère statistique A1 et du composé A2 représente de 0,5 à 50%, par rapport à la masse totale de la composition lubrifiante.
Dans un mode de réalisation, le pourcentage molaire de composé exogène A5 dans la composition lubrifiante va de 0,05% à 5000%, de préférence va de 0,1% à 1000%, de manière plus préférée de 0,5% à 500%, de manière encore plus préférée de 1% à 150% par rapport aux fonctions diol du copolymère statistique A1.
Dans un mode de réalisation, la composition lubrifiante de l'invention résulte du mélange de :

- 0,5% à 20 % en poids d'au moins un copolymère statistique polydiol A1 tel que défini précédemment, par rapport au poids total de la composition lubrifiante ;
- 0,5% à 20 % en poids d'au moins d'au moins un composé A2 tel que défini précédemment, notamment en copolymère statistique ester boronique ; par rapport au poids total de la composition lubrifiante ; et
- 0,001% à 0,5 % en poids d'au moins un composé exogène A5 tel que défini précédemment, par rapport au poids total de la composition lubrifiante, et
- 60% à 99 % en poids d'au moins une huile lubrifiante tel que défini précédemment, par rapport au poids total de la composition lubrifiante.

Dans un autre mode de réalisation, la composition lubrifiante de l'invention résulte du mélange de :

- 0,5% à 20 % en poids d'au moins un copolymère statistique polydiol A1 tel que défini précédemment, par rapport au poids total de la composition lubrifiante ;
- 0,5% à 20 % en poids d'au moins d'au moins un composé A2 tel que défini précédemment, notamment en copolymère statistique ester boronique ; par rapport au poids total de la composition lubrifiante ; et
- éventuellement 0,001% à 0,5 % en poids d'au moins un composé exogène A5 tel que défini précédemment, par rapport au poids total de la composition lubrifiante, et
- 0,5% à 15 % en poids d'au moins un additif fonctionnel tel que défini précédemment, par rapport au poids total de la composition lubrifiante, et
- 60% à 99 % en poids d'au moins une huile lubrifiante tel que défini précédemment, par rapport au poids total de la composition lubrifiante.

➢ Procédé pour moduler la viscosité d'une composition lubrifiante

La demande divulgue également un procédé pour moduler la viscosité d'une composition lubrifiante, le procédé comprenant au moins :

- la fourniture d'une composition lubrifiante résultant du mélange d'au moins une huile lubrifiante, d'au moins un copolymère statistique polydiol A1 et d'au moins un copolymère statistique A2 comprenant au moins deux fonctions esters boroniques et pouvant s'associer avec ledit copolymère statistique polydiol A1 par au moins une réaction de transestérification,

l'ajout dans ladite composition lubrifiante d'au moins un composé exogène A5 choisi parmi les diesters boroniques de formule (XI).

Par « moduler la viscosité d'une composition lubrifiante », on entend au sens de la présente invention, une adaptation de la viscosité à une température donnée en fonction de l'utilisation de la composition lubrifiante. Ceci est obtenu en ajoutant un composé exogène A5 tel que défini précédemment. Ce composé permet de contrôler le taux d'association et de réticulation des deux copolymères polydiol A1 et poly(ester boronique) A2.

Autres objets selon l'invention

Un autre objet de la présente invention est l'utilisation de la composition lubrifiante telle que définie ci-dessus pour lubrifier une pièce mécanique.

Dans la suite de la description, les pourcentages sont exprimés en poids par rapport au poids total de la composition lubrifiante.

Les compositions de l'invention sont utilisables pour lubrifier les surfaces des pièces que l'on trouve classiquement dans un moteur telles que le système pistons, segments, chemises.

Ainsi un autre objet de la présente invention est une composition pour lubrifier au moins un moteur, ladite composition comprenant, notamment consiste essentiellement en, une composition résultant du mélange de :

- 85% à 99,98% en poids, avantageusement de 92 à 99% en poids d'une huile lubrifiante, et
- 0,1% à 15% en poids, avantageusement de 1 à 8% en poids d'un mélange d'au moins un copolymère statistique A1 tel que défini précédemment, et d'au moins un copolymère statistique ester boronique A2 tel que défini précédemment ; et
- 0,001% à 0,1% en poids au moins un composé exogène A5 tel que défini précédemment ;

la composition ayant une viscosité cinématique à 100°C mesurée selon la norme ASTM D445 allant de 3,8 à 26,1 cSt ; les pourcentages en poids étant exprimé par rapport au poids total de ladite composition.

Dans une composition pour lubrifier au moins un moteur telle que définie ci-dessus, les copolymères statistique A1, et les copolymères statistiques ester boronique A2 tels que définis précédemment peuvent s'associer et s'échanger de manière thermoréversible, en présence du composé exogène A5; mais ils ne forment pas de réseaux tridimensionnels. Ils ne sont pas réticulés.

Dans un mode de réalisation, la composition pour lubrifier au moins un moteur comprend en outre au moins un additif fonctionnel choisi parmi le groupe formé par les détergents, les additifs anti-usure, les additifs extrême pression, les antioxydants supplémentaires, les additifs anticorrosion, les polymères améliorant l'indice de viscosité, les améliorants de point d'écoulement, les anti-mousse, les épaississants, les dispersants, les modificateurs de frottements et leurs mélanges.

Dans un mode de réalisation de l'invention, la composition pour lubrifier au moins un moteur, ladite composition comprenant, notamment consiste essentiellement en une composition résultant du mélange de :

- 80% à 99% en poids d'une huile lubrifiante, et
- 0,1% à 15% en poids d'un mélange d'au moins copolymère statistique A1 tel que défini précédemment, d'au moins un copolymère statistique ester boronique A2 tel que défini précédemment ; et
- 0,001% à 0,1% en poids au moins un composé exogène A5 tel que défini précédemment ;
- 0,5 à 15% en poids d'au moins un additif fonctionnel choisi parmi le groupe formé par les détergents, les additifs anti-usure, les additifs extrême pression, les antioxydants supplémentaires, les additifs anticorrosion, les polymères améliorant l'indice de viscosité, les améliorants de point d'écoulement, les anti-mousse, les épaississants, les dispersants, les modificateurs de frottements et leurs mélanges ;

la composition ayant une viscosité cinématique à 100°C mesurée selon la norme ASTM D445 allant de 3,8 à 26,1 cSt ; les pourcentages en poids étant exprimé par rapport au poids total de ladite composition.

Les définitions et préférences relatives aux huiles lubrifiantes, aux copolymères statistiques A1, aux copolymère statistique ester boronique A2 et aux composé exogène A5 s'appliquent également aux compositions pour lubrifier au moins un moteur.

Un autre objet de la présente invention est une composition pour lubrifier au moins une transmission, telle que les boîtes de vitesses manuelles ou automatiques.

Ainsi un autre objet de la présente invention est une composition pour lubrifier au moins une transmission, ladite composition comprenant, notamment consiste essentiellement en une composition résultant du mélange de :

- 50% à 99,4% en poids d'une huile lubrifiante, et
- 0,5% à 15% en poids d'un mélange d'au moins copolymère statistique A1 tel que défini précédemment, et d'au moins un copolymère statistique ester boronique A2 tel que défini précédemment ; et
- 0,001% à 0,5% en poids au moins un composé exogène A5 tel que défini précédemment ;

la composition ayant une viscosité cinématique à 100°C mesurée selon la norme ASTM D445 allant de 4,1 à 41 cSt, les pourcentages en poids étant exprimé par rapport au poids total de ladite composition.

Dans une composition pour lubrifier au moins une transmission telle que définie ci-dessus, les copolymères statistique A1, et les copolymères statistiques ester boronique A2 tels que définis précédemment peuvent s'associer et s'échanger de manière thermoréversible, en présence du composé exogène A5 ; mais ils ne forment pas de réseaux tridimensionnels. Ils ne sont pas réticulés.

Dans un mode de réalisation la composition pour lubrifier au moins une transmission comprend en outre au moins un additif fonctionnel choisi parmi le groupe formé par les détergents, les additifs anti-usure, les additifs extrême pression, les antioxydants supplémentaires, les additifs anticorrosion, les polymères améliorant l'indice de viscosité, les améliorants de point d'écoulement, les anti-mousses, les épaississants, les dispersants, les modificateurs de frottements et leurs mélanges.

Dans un mode de réalisation de l'invention, la composition pour lubrifier au moins une transmission comprend, notamment consiste essentiellement en, une composition résultant du mélange de :

- 45% à 99,39% en poids d'une huile lubrifiante, et
- 0,5% à 50% en poids d'un mélange d'au moins un copolymère statistique A1 tel que défini précédemment, et d'au moins un copolymère statistique ester boronique A2 tel que défini précédemment ; et
- 0,001% à 0,5% en poids au moins un composé exogène A5 tel que défini précédemment ;
- 0,1% à 15% en poids d'au moins un additif fonctionnel choisi parmi le groupe formé par les détergents, les additifs anti-usure, les additifs extrême pression, les antioxydants supplémentaires, les additifs anticorrosion, les polymères améliorant l'indice de viscosité, les améliorants de point d'écoulement, les anti-mousses, les épaississants, les dispersants, les modificateurs de frottements et leurs mélanges ;

la composition ayant une viscosité cinématique à 100°C mesurée selon la norme ASTM D445 allant de 4,1 à 41 cSt les pourcentages en poids étant exprimé par rapport au poids total de ladite composition.

Les définitions et préférences relatives aux huiles lubrifiantes, aux copolymères statistiques A1 aux copolymère statistique ester boronique A2 et aux composé exogène A5 s'appliquent également aux compositions pour lubrifier au moins une transmission.

Les compositions de l'invention peuvent être utilisées pour les moteurs ou transmissions des véhicules légers, des poids-lourds mais aussi des navires.

Un autre objet de la présente invention est un procédé de lubrification d'au moins une pièce mécanique, notamment au moins un moteur ou au moins une transmission, ledit procédé comprenant une étape dans laquelle ladite pièce mécanique est mise en contact avec au moins une composition lubrifiante telle que définie ci-dessus.

Les définitions et préférences relatives aux huiles lubrifiantes, aux copolymères statistiques A1, notamment

celui résultant de la copolymérisation d'au moins un monomère de formule (I) avec au moins un monomère de formule (II-A), au moins un monomère de formule (II-B) et éventuellement un monomère M3 de formule (X), aux copolymère statistique ester boronique A2 et le cas échéant aux composé exogène A5 s'appliquent également au procédé de lubrification d'au moins une pièce mécanique.

**Figures**

La figure 1 représente de manière schématique un copolymère statistique (P1), un copolymère à gradient (P2) et un copolymère à blocs (P3), chaque rond représente un motif monomère. La différence de structure chimique entre les monomères est symbolisée par une couleur différente (gris clair/noir).

La figure 2 représente de manière schématique un copolymère peigne.

La figure 3 illustre de manière schématique les réactions d'échanges de liens esters boroniques entre deux polymères statistiques polydiols (A1-1 et A1-2) et deux polymères statistiques esters boroniques (A2-1 et A2-2) en présence de diols.

La figure 4 illustre et représente de manière schématique la réticulation de la composition selon l'invention dans du tétrahydrofurane (THF).

La figure 5 représente de manière schématique le comportement de la composition de l'invention en fonction de la température. Un copolymère statistique (2) possédant des fonctions diols (fonction A) peut s'associer de manière thermoréversible avec un copolymère statistique (1) possédant des fonctions esters boroniques (fonction B) via une réaction de transestérification. Le groupement organique des fonctions ester boronique (fonction B) qui s'échange lors de la réaction de transestérification est un diol symbolisé par un croissant noir. Il se forme un lien chimique (3) de type ester boronique avec libération d'un composé diol.

La figure 6 représente l'évolution de la viscosité relative (sans unité, l'axe des ordonnées) en fonction de la température (°C, l'axe des abscisses) des compositions B et C.

La figure 7 représente l'évolution de la viscosité relative (sans unité, l'axe des ordonnées) en fonction de la température (°C, l'axe des abscisses) des compositions A, E, F et G.

**Partie expérimentale :**

Les exemples suivants illustrent l'invention sans la limiter.

**1. Synthèse de copolymères statistiques A1 porteurs de fonction diol**

◦ *1.1 : A partir d'un monomère porteur d'une fonction diol*

Dans un mode de réalisation, le copolymère statistique A1 de l'invention est obtenu selon le schéma réactionnel 11 suivant :

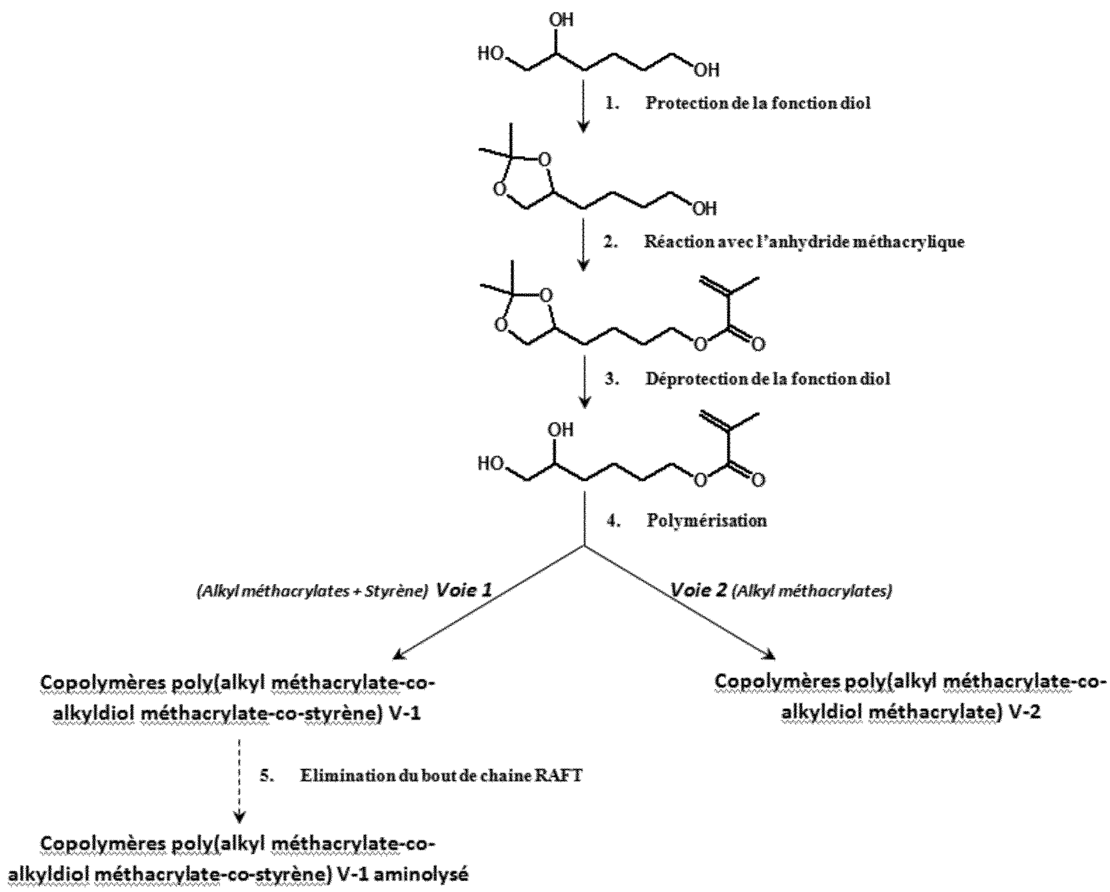


Le copolymère obtenu après élimination du bout de chaîne RAFT contient, entre autres, du styrène comme comonomère et le résidu thiocarbonylthio a été éliminé, par exemple en le convertissant en un thioéther.

*1.1.1 Synthèse du monomère M1 porteur d'une fonction diol*

La synthèse d'un monomère méthacrylate porteur d'une fonction diol s'effectue en trois étapes (étapes 1, 2 et 3 du schéma réactionnel 11) selon le protocole ci-dessous :

1$^{ière}$ étape :

42,1 g (314 mmol) d' 1,2,6-hexane triol (1,2,6-HexTri) sont introduits dans un ballon d'1L. 5,88 g de tamis moléculaire (4°A) sont ajoutés suivis de 570 mL d'acétone. 5,01 g (26,3 mmol) d'acide para-toluène-sulfonique (pTSA) sont ensuite lentement additionnés. Le milieu réactionnel est laissé sous agitation pendant 24 heures à température ambiante. 4,48 g (53,3 mmol) de $NaHCO_3$ sont alors ajoutés. Le milieu réactionnel est laissé sous agitation pendant 3 heures à température ambiante avant d'être filtré. Le filtrat est alors concentré sous vide au moyen d'un évaporateur rotatif jusqu'à obtention d'une suspension de cristaux blancs. 500 mL d'eau sont alors ajoutés à cette suspension. La solution ainsi obtenue est extraite avec 4 × 300 mL de dichlorométhane. Les phases organiques sont regroupées et séchées sur $MgSO_4$. Le solvant est ensuite entièrement évaporé sous vide à 25°C au moyen d'un évaporateur rotatif.

2$^{ème}$ étape :

5,01 g (28,8 mmol) du produit ainsi obtenu est ensuite introduit dans un ballon d'1L. 4,13 g (31,9 mmol) de DIPEA et 37,9 mg (0,31 mmol) de DMAP sont ensuite introduits dans le ballon suivis de 5,34 g (34,6 mmol) d'anhydride méthacrylique. Le ballon est alors placé sous agitation à température ambiante pendant 24 heures. 0,95 g de méthanol (29,7 mmol) sont ensuite ajoutés à la solution et le ballon fut laissé sous agitation 1 heure supplémentaire. Le produit est alors dissout dans 40 mL d'hexane. La phase organique est ensuite successivement lavée avec 25 mL d'eau, 3 × 25 mL d'une solution aqueuse d'acide chlorhydrique à 0,5 M, 3 × 25 mL d'une solution aqueuse de NaOH à 0,5 M et de nouveau avec 25 mL d'eau. La phase organique est séchée sur $MgSO_4$, filtrée puis concentrée sous vide à l'aide

d'un évaporateur rotatif pour donner un liquide jaune clair dont les caractéristiques sont les suivantes :

3ème étape :

17,23 g (71,2 mmol) du produit ainsi obtenu est ensuite introduit dans un ballon d'1L. 90 mL d'eau et 90 mL d'acétonitrile sont ensuite introduits dans le ballon suivis de 59,1 mL (159 mmol) d'acide acétique. Le ballon est alors placé sous agitation pendant 24 heures à 30°C en laissant buller un léger flux d'azote pour pousser l'élimination de l'acétone. La solution ainsi obtenue est extraite avec 6 × 30 mL d'acétate d'éthyle. La phase organique est ensuite successivement lavée avec 5 × 30 mL d'une solution aqueuse de NaOH à 0,5 M puis 3 × 30 mL d'eau. La phase organique est ensuite séchée sur $MgSO_4$, filtrée puis concentrée sous vide à l'aide d'un évaporateur rotatif pour donner un liquide jaune clair dont les caractéristiques sont les suivantes :
$^1$H RMN (400 MHz, CDCl3) $\delta$ : 6,02 (singulet, 1H), 5,49 (singulet, 1H), 4,08 (triplet, J = 6,4 Hz, 1H), 3,65-3,58 (multiplet, 1H), 3,57-3,50 (multiplet, 3H), 3,35 (doublet de doublets, J = 7,6 Hz et J = 11,2 Hz, 1H), 1,86 (doublet de doublets, J = 1,2 Hz et J = 1,6 Hz, 3H), 1,69-1,31 (multiplet, 6H).

*1.1.2 Synthèse de copolymères méthacrylates portant des fonctions diol*

La synthèse des copolymères méthacrylates portant des fonctions diol s'effectue en deux étapes (étapes 4 et 5 du schéma réactionnel 11) :

- Copolymérisation de deux monomères méthacrylate d'alkyle avec un monomère méthacrylate porteur d'une fonction diol et un monomère styrène ;
- Elimination du bout de chaîne RAFT (aminolyse suivie d'une addition de Michael du thiol sur un acrylate d'alkyle).

Plus précisément, la synthèse du copolymère s'effectue selon le protocole suivant:

1ère étape :

12,56 g (37,1 mmol) de méthacrylate de stéaryle (StMA), 12,59 g (49,5 mmol) de méthacrylate de lauryle (LMA), 2,57 g (24,7 mmol) de styrène (Sty), 2,54 g (12,4 mmol) de méthacrylate porteur d'une fonction diol obtenu selon le protocole décrit au paragraphe 1.1.1, 82,5 mg (0,30 mmol) de dithiobenzoate de cumyle, 15 mg (0,09 mmol) d'azobisisobutyronitrile (AIBN) et 30 mL d'anisole sont introduits dans un tube Schlenk de 250 mL. Le milieu réactionnel est placé sous agitation et dégazé pendant 30 minutes en faisant buller de l'azote avant d'être porté à 65°C pour une durée de 24 heures.

2ème étape

Après 24h de polymérisation, le tube de Schlenk est placé dans un bain de glace pour arrêter la polymérisation, 30 mL de diméthyl formamide (DMF) et 0,4 mL de n-butylamine (4 mmol) sont ajoutés à la solution sans dégazer le milieu. Quinze heures plus tard, le polymère était totalement décoloré et 3 mL (21 mmol) d'acrylate de butyle sont ajoutés. 16 heures plus tard, le polymère est isolé par 3 précipitations successives dans le méthanol, filtration et séchage sous vide à 50°C pendant une nuit. On obtient ainsi un copolymère présentant une masse molaire moyenne en nombre ($M_n$) de 53 000 g/mol, un indice de polydispersité (Ip) de 1,19 et un degré de polymérisation moyen en nombre ($DP_n$) de 253. Ces valeurs sont respectivement obtenues par chromatographie d'exclusion stérique en utilisant le tétrahydrofurane comme éluant et une calibration poly(méthacrylate de méthyle)et par suivi de la conversion en monomères au cours de la copolymérisation.
On obtient un copolymère poly(alkyl méthacrylate-co-alkyldiol méthacrylate-co-styrène) contenant environ 10% molaire d'unités monomères diol M1.

## 2. Synthèse du copolymère poly(alkyl méthacrylate-co-monomère ester boronique)

Cette synthèse est réalisée selon le protocole décrit dans la demande WO2016/113229 (partie expérimentale §2.).

## 3. Synthèse du composé A5

La synthèse du composé A5 s'effectue en une étape en suivant le protocole ci-dessous :
5,000 g d'acide phénylboronique (PBA) (41,0 mmol) sont introduits dans un ballon de 500 mL suivis de 200 mL de THF.

Le milieu réactionnel est ensuite placé sous agitation. 0,5 mL (28 mmol) d'eau sont ajoutés goutte à goutte jusqu'à dissolution complète de l'acide phénylboronique. Le milieu réactionnel devient alors transparent et homogène. 8,286 g de 1,2-dodécanediol (1,2-DDD) (41,0 mmol) sont ensuite lentement ajoutés, suivis d'un excès de sulfate de magnésium anhydre afin de piéger l'eau initialement introduite ainsi que l'eau libérée par la condensation entre le PBA et le 1,2-DDD. Le milieu réactionnel est laissé sous agitation pendant 2 heures à 25°C avant d'être filtré. Le solvant est ensuite éliminé du filtrat au moyen d'un évaporateur rotatif. Le composé obtenu est un mélange contenant environ 93% molaire de l'ester boronique et 7% molaire de 1,2-dodécandiol résiduel.

$^1$H RMN (400 MHz, CDCl3) $\delta$ : 7,90 (multiplet, 2H), 7,60-7,40 (multiplet, 3H), 4,60 (multiplet, 1H), 4,46 (triplet, J = 9,2 Hz, 1H), 3,99 (doublet de doublets, J = 7,2 Hz et J = 8,8 Hz, 1H), 1,85-1,20 (multiplet, 18H), 0,96 (triplet, J = 6,8 Hz, 3,2H).

## 4. Etudes rhéologiques

### ◦ *4.1 Ingrédients pour la formulation de compositions A à G*

**Huile de base lubrifiante**

L'huile de base lubrifiante utilisée dans les compositions à tester est une huile du groupe III de la classification API, commercialisée par SK sous le nom Yubase 4. Elle présente les caractéristiques suivantes :

- Sa viscosité cinématique à 40°C mesurée selon la norme ASTM D445 est de 19,57 cSt;
- Sa viscosité cinématique mesurée à 100°C selon la norme ASTM D445 est de 4,23 cSt ;
- Son indice de viscosité mesuré selon la norme ASTM D2270 est de 122 ;
- Sa volatilité Noack en pourcentage poids, mesurée selon la norme DIN 51581 est de 14,5 ;
- Sont point flash (flash point en anglais) en degré Celsius mesuré selon la norme ASTM D92 est de 230°C ;
- Son point d'écoulement (pour point en anglais) en degré Celsius mesuré selon la norme ASTM D97 est de -15°C.

\- **Copolymère statistique polydiol A-1**

Ce copolymère comprend 10% molaire de monomères ayant des fonctions diols et 25% molaire de monomères styrène. La longueur moyenne de chaîne latérale est de 13,5 atomes de carbone. Sa masse molaire moyenne en nombre est de 50 500 g/mol. Son indice de polydispersité est de 1,26. Son degré de polymérisation moyen en nombre (DPn) est de 240. La masse molaire moyenne en nombre et l'indice de polydispersité sont mesurés par mesure de chromatographie d'exclusion stérique en utilisant une calibration poly(méthacrylate de méthyle). Ce copolymère est obtenu par la mise en oeuvre du protocole décrit au paragraphe 1 ci-dessus

\- **Copolymère statistique A-2 ester boronique :**

Ce copolymère comprend 5 % molaire de monomères ayant des fonctions esters boroniques. La longueur moyenne de chaînes latérale est égale à 12 atomes de carbone. Sa masse molaire moyenne en nombre est de 39 000 g/mol. Son indice de polydispersité est de 1,41. Son degré de polymérisation moyen en nombre (DPn) est de 192. Sa masse molaire moyenne en nombre et l'indice de polydispersité sont mesurés par mesure de chromatographie d'exclusion stérique en utilisant une calibration poly(méthacrylate de méthyle). Ce copolymère est obtenu par la mise en oeuvre du protocole décrit au paragraphe 2 ci-dessus.

\- **Composé A5** :

Ce composé est constitué de 93% molaire de l'ester boronique formé de l'estérification de l'acide phénylboronique et du 1,2-dodecanediol et à 7% molaire d'un excès de 1,2-dodécandiol. Ce composé est obtenu par la mise en oeuvre du protocole décrit au paragraphe 3 ci-dessus.

### *4.2 Formulation de compositions pour l'étude de la viscosité*

**La composition A (comparative) est obtenue de la manière suivante :**

Elle contient une solution à 4,2 % massique d'un polymère polyméthacrylate dans une huile de base lubrifiante du groupe III de la classification API. Le polymère a une masse molaire moyenne en nombre (Mn) égale à 106 000 g/mol, un indice de polydispersité (Ip) égale à 3,06, un degré de polymérisation moyen en nombre de 466 et la longueur

moyenne des chaînes pendantes est de 14 atomes de carbone.

Ce polyméthacrylate est utilisé comme additif améliorant l'indice de viscosité. 4,95 g d'une formulation ayant une concentration massique de 42 % de ce polyméthacrylate dans une huile de base groupe III et 44,6 g d'huile de base groupe III sont introduits dans un flacon. La solution ainsi obtenue est maintenue sous agitation à 90°C jusqu'à dissolution complète du polyméthacrylate.

On obtient une solution à 4,2 % massique de ce polyméthacrylate. Cette composition est utilisée comme référence pour l'étude de la viscosité. Elle représente le comportement rhéologique des compositions lubrifiantes commercialisées.

**La composition B (comparative) est obtenue de la manière suivante :**

6,52 g de copolymère polydiol A-1 et 58,68 g d'une huile de base groupe III sont introduits dans un flacon. La solution ainsi obtenue est maintenue sous agitation à température ambiante jusqu'à dissolution complète du polydiol A-1. On obtient une solution à 10% massique de copolymère polydiol A-1.

4,2 g de cette solution de polydiol A-1 à 10% massique dans l'huile de base de groupe III sont mélangés avec 2,8 g de cette même huile de base. La solution ainsi obtenue est maintenue sous agitation à température ambiante pendant 5 minutes. On obtient une solution à 6 % massique de copolymère polydiol A-1.

**La composition C (comparative) est obtenue de la manière suivante :**

7,33 g de copolymère poly ester boronique A-2 et 65,97 g d'une huile de base groupe III sont introduits dans un flacon. La solution ainsi obtenue est maintenue sous agitation à température ambiante jusqu'à dissolution complète du poly ester boronique A-2. On obtient une solution à 10% massique de copolymère poly ester boronique A-2.

4,2 g de cette solution de poly ester boronique A-2 à 10% massique dans l'huile de base de groupe III sont mélangés avec 2,8 g de cette même huile de base. La solution ainsi obtenue est maintenue sous agitation à température ambiante pendant 5 minutes. On obtient une solution à 6 % massique de copolymère poly ester boronique A-2.

**La composition D est obtenue de la manière suivante :**

1,65 g de composé A-5 et 14,85 g d'une huile de base groupe III sont introduits dans un flacon. La solution ainsi obtenue est maintenue sous agitation à température ambiante jusqu'à dissolution complète du composé A-5. On obtient une solution à 10% massique de composé A-5.

**La composition E (comparative) est obtenue de la manière suivante :**

2,80 g de la solution à 10% massique de polydiol A-1 préparée précédemment et 1,40 g d'huile de base de groupe III sont introduits dans un flacon. 2,80 g de la solution à 10% massique de poly ester boronique A-2 préparée précédemment sont ajoutés à cette solution. La solution ainsi obtenue est maintenue sous agitation à température ambiante pendant 5 minutes. On obtient une solution à 4 % massique de copolymère polydiol A-1 et 4 % massique de copolymère poly(ester boronique) A-2.

**La composition F (selon l'invention) est obtenue de la manière suivante :**

2,80 g de la solution à 10% massique de polydiol A-1 préparée précédemment et 1,26 g d'huile de base de groupe III sont introduits dans un flacon. 2,80 g de la solution à 10% massique de poly ester boronique A-2 préparée précédemment et 0,14 g de la composition D préparée précédemment sont ajoutés à cette solution. La solution ainsi obtenue est maintenue sous agitation à température ambiante pendant 5 minutes. On obtient une solution à 4 % massique de copolymère polydiol A-1, 4 % massique de copolymère poly(ester boronique) A-2 et 48,6 μmol du composé A-5. La solution comprend donc 43 % molaire de composé A-5 par rapport aux fonctions diol du polydiol A-1 et 93 % molaire de composé A-5 par rapport aux fonctions EB du poly(ester boronique) A-2.

**La composition G (selon l'invention) est obtenue de la manière suivante :**

2,80 g de la solution à 10% massique de polydiol A-1 préparée précédemment et 1,12 g d'huile de base de groupe III sont introduits dans un flacon. 2,80 g de la solution à 10% massique de poly ester boronique A-2 préparée précédemment et 0,28 g de la composition D préparée précédemment sont ajoutés à cette solution. La solution ainsi obtenue est maintenue sous agitation à température ambiante pendant 5 minutes. On obtient une solution à 4 % massique de copolymère polydiol A-1, 4 % massique de copolymère poly(ester boronique) A-2 et 97,2 μmol du composé A-5. La

solution comprend donc 86 % molaire de composé A-5 par rapport aux fonctions diol du polydiol A-1 et 186 % molaire de composé A-5 par rapport aux fonctions EB du poly(ester boronique) A-2.

◦ ***4.4 Appareillages et protocoles de mesure de la viscosité***

Les études rhéologiques ont été effectuées à l'aide d'un rhéomètre de Couette MCR 501 à contrainte contrôlée de la société Anton Paar.

Dans le cas des formulations de polymères qui ne forment pas de gels dans une huile de base du groupe III sur la plage de température de l'étude (compositions A à G), les mesures de rhéologie ont été réalisée en utilisant une géométrie cylindrique de référence DG 26.7 La viscosité a été mesurée en fonction de la vitesse de cisaillement pour une gamme de température variant de 10°C à 110°C. Pour chaque température, la viscosité du système a été mesurée en fonction de la vitesse de cisaillement de 1 à 100 $s^{-1}$. Les mesures de viscosité en fonction de la vitesse de cisaillement à T = 10°C, 50°C, 70°C et 110°C ont été réalisées (en allant de 10°C à 110°C). Une viscosité moyenne a alors été calculée pour chaque température en utilisant les points de mesure situés sur le même plateau.

La viscosité relative calculée selon la formule suivante

$$(\eta_{relative} = \frac{\eta_{solution}}{\eta_{huile\ de\ base}})$$

a été choisie pour représenter l'évolution de la viscosité du système en fonction de la température, car cette grandeur reflète directement la compensation à la perte de viscosité naturelle d'une huile de base de groupe III des systèmes polymères étudiés.

◦ ***4.5 Résultats obtenus en rhéologie***

La viscosité des compositions A à F a été étudiée sur une plage de températures allant de 10°C à 110°C. La viscosité relative de ces compositions est illustrée en figures 6 et 7. Le copolymère statistique polydiol A-1, seul dans la composition B, ne permet pas une compensation significative de la perte de viscosité naturelle de l'huile de base de groupe III (Figure 6). Il en est de même pour le copolymère poly(ester boronique) A-2 lorsque ce copolymère est utilisé seul dans la composition C (Figure 6).

Lorsque le copolymère statistique polydiol A-1 et le copolymère poly(ester boronique) A-2 sont présents ensemble dans la même composition lubrifiante (composition E), on observe une compensation de la perte de viscosité naturelle de l'huile de base de groupe III plus importante que celle qui résulte de l'ajout du polymère polyméthacylate dans l'huile de base de groupe III (composition A) (Figure 7).

Lorsque la composition (composition F) comprend en outre 43 % molaire de composé A-5 libre par rapport aux fonctions diol du copolymère polydiol A-1 ; on observe une légère diminution de la viscosité relative à basses températures ainsi qu'une légère diminution de la compensation de la perte de viscosité à chaud par rapport à celle de la composition E qui comprend le copolymère statistique polydiol A-1 et le copolymère poly(ester boronique) A-2 ) (Figure 7). La composition F permet en outre d'obtenir une meilleure compensation de la perte de viscosité de l'huile à chaud que la composition A tout en donnant une plus faible viscosité que cette même composition A de 10°C à 70°C.

Lorsque la composition (composition G) comprend en outre 86 % molaire de composé A-5 libre par rapport aux fonctions diol du copolymère polydiol A-1 ; on observe une plus forte diminution de la viscosité relative à basses températures ainsi qu'une diminution de la compensation de la perte de viscosité à chaud par rapport à celle de la composition E qui comprend le copolymère statistique polydiol A-1 et le copolymère poly(ester boronique) A-2 ) (Figure 7). Les compositions F et G conservent toujours la propriété de compenser la perte de viscosité de l'huile de base du groupe III pour des températures élevées. Le composé A-5 permet donc de modifier en fonction de la température, la viscosité d'une composition lubrifiante résultant du mélange d'au moins un copolymère statistique polydiol A-1 et d'au moins un copolymère statistique A-2 poly(ester boronique) en contrôlant le taux d'association des chaînes de ces deux copolymères.

**Revendications**

**1.** Composition résultant du mélange d'au moins :

- un copolymère statistique polydiol A1,
- un composé A2 comprenant au moins deux fonctions esters boroniques,

- un composé exogène A5 choisi parmi ceux répondant à la formule (XI) :

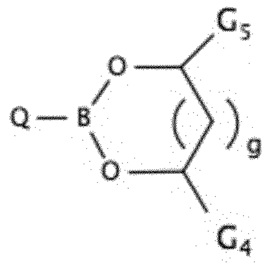

(XI)

dans laquelle :

- Q représente un groupement choisi parmi un groupement hydrocarboné comprenant de 1 à 30 atomes de carbone, éventuellement substituée par un ou plusieurs groupements choisis parmi : un hydroxyle, un groupement -OJ, -C(O)-O-J avec J un groupement hydrocarboné comprenant de 1 à 24 atomes de carbone,
- $G_4$, $G_5$, identiques ou différents, représentent des groupements choisis parmi un atome d'hydrogène, une chaîne hydrocarbonée comprenant de 1 à 24 atomes de carbone, un hydroxyle, un groupement -OJ, -C(O)-O-J avec J un groupement hydrocarboné comprenant de 1 à 24 atomes de carbone,
- g représente 0 ou 1.

le copolymère statistique polydiol A1 ayant une masse molaire moyenne en nombre allant de 5 000 à 400 000 g/mol, le pourcentage molaire de composé exogène A5 par rapport aux fonctions diol du copolymère statistique A1 allant de 0,025 à 5000%, et le ratio massique entre le copolymère A1 et le composé A2 (ratio A1/A2) allant de 0,1 à 10.

**2.** Composition selon la revendication 1 dans laquelle le composé exogène A5 est choisi parmi ceux répondant à la formule (XIA) :

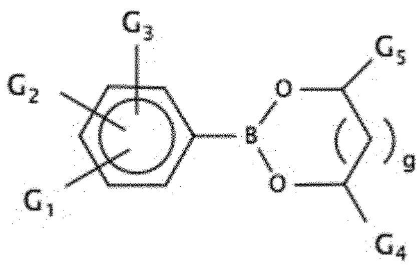

(XIA)

dans laquelle :

- $G_1$, $G_2$, $G_3$, $G_4$, $G_5$, identiques ou différents, représentent des groupements choisis parmi un atome d'hydrogène, une chaîne hydrocarbonée comprenant de 1 à 24 atomes de carbone, un hydroxyle, un groupement -OJ, -C(O)-O-J avec J un groupement hydrocarboné comprenant de 1 à 24 atomes de carbone,
- g représente 0 ou 1.

**3.** Composition selon la revendication 1 ou selon la revendication 2, dans laquelle le pourcentage molaire de composé exogène A5 par rapport aux fonctions diol du copolymère statistique A1 va de 0,1% à 1000%, de manière encore plus préférée de 0,5% à 500%, de manière encore plus préférée de 1% à 150%.

**4.** Composition selon la revendication 2 ou selon la revendication 3, dans laquelle le composé exogène A5 est choisi parmi ceux répondant à la formule (XI B) :

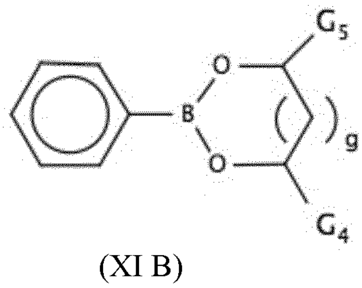

(XI B)

**5.** Composition selon la revendication 4, dans laquelle le composé exogène A5 est choisi parmi ceux répondant à la formule (XI B) avec g = 0, $G_4$ = H et $G_5$ représente un alkyle en C1-C24.

**6.** Composition selon l'une quelconque des revendications précédentes, dans laquelle, le copolymère statistique A1 résulte de la copolymérisation :

- d'au moins un premier monomère M1 de formule générale (I) :

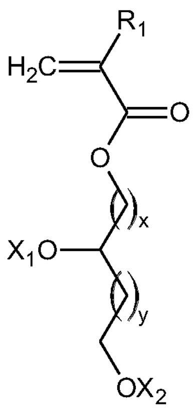

(I)

dans laquelle :

- $R_1$ est choisi parmi le groupe formé par -H, $-CH_3$, et $-CH_2-CH_3$ ;
- x est un nombre entier allant de 1 à 18, de préférence de 2 à 18 ;
- y est un nombre entier égal à 0 ou 1;
- $X_1$ et $X_2$, identiques ou différents, sont choisis parmi le groupe formé par l'hydrogène, le tétrahydropyranyle, le méthyloxyméthyle, le ter-butyle, le benzyle, le triméthylsilyle et le t-butyle diméthylsilyle ;

ou bien

- $X_1$ et $X_2$ forment avec les atomes d'oxygène un pont de formule suivante

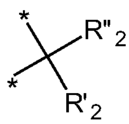

dans laquelle:

- les étoiles (*) symbolisent les liaisons aux atomes d'oxygène,
- $R'_2$ et $R''_2$, identiques ou différents, sont choisis parmi le groupe formé par l'hydrogène et un alkyle en $C_1$-$C_{11}$, de préférence le méthyle ;

ou bien

- $X_1$ et $X_2$ forment avec les atomes d'oxygène un ester boronique de formule suivante :

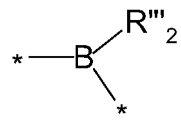

dans laquelle :

- les étoiles (*) symbolisent les liaisons aux atomes d'oxygène,
- R‴$_2$ est choisi parmi le groupe formé par un aryle en $C_6$-$C_{30}$, un aralkyle en $C_7$-$C_{30}$ et un alkyle en $C_2$-$C_{30}$, de préférence un aryle en Ce-Cis;

▪ avec au moins un second monomère M2 de formule générale (II) :

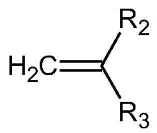

(II)

dans laquelle :

- $R_2$ est choisi parmi le groupe formé par -H, $-CH_3$ et $-CH_2-CH_3$,
- $R_3$ est choisi parmi le groupe formé par un aryle en $C_6$-$C_{18}$, un aryle en $C_6$-$C_{18}$ substitué par un groupement R'$_3$, -C(O)-O-R'$_3$,
- O-R'$_3$, -S-R'$_3$ et -C(O)-N(H)-R'$_3$ avec R'$_3$ un groupe alkyle en $C_1$-$C_{30}$.

**7.** Composition selon la revendication 6, dans laquelle le copolymère statistique A1 résulte de la copolymérisation :

▪ d'au moins un premier monomère M1 de formule générale (I),
▪ avec au moins un second monomère M2 de formule générale (II) :

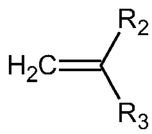

(II)

dans laquelle :

- $R_2$ est choisi parmi le groupe formé par -H, $-CH_3$ et $-CH_2-CH_3$,

▪ $R_3$ est choisi parmi le groupe formé par -C(O)-O-R'$_3$, -O-R' 3, -S-R'$_3$ et -C(O)-N(H)-R'$_3$ avec R'$_3$ un groupe alkyle en $C_1$-$C_{30}$,
et
▪ avec au moins un troisième monomère M3 de formule générale (X) :

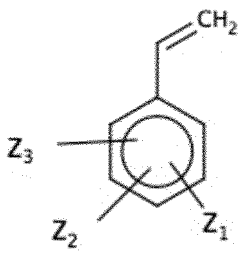

(X)

dans laquelle :

- $Z_1$, $Z_2$, $Z_3$, identiques ou différents, représentent des groupements choisis parmi un atome d'hydrogène, un alkyle en $C_1$-$C_{12}$, un groupement -OZ', -C(O)-O-Z' avec Z' un alkyle en $C_1$-$C_{12}$.

**8.** Composition selon la revendication 7, dans laquelle le troisième monomère M3 est le styrène.

**9.** Composition selon l'une quelconque des revendications 7 et 8, dans laquelle le copolymère statistique A1 résulte de la copolymérisation d'au moins un monomère M1 avec au moins deux monomères M2 ayant des groupes $R_3$ différents et au moins un monomère M3.

**10.** Composition selon l'une quelconque des revendications précédentes, dans laquelle le copolymère statistique A1 a un degré de polymérisation moyen en nombre allant de 40 à 2000, de préférence de 40 à 1000 et un indice de polydispersité (Ip) allant de 1,05 à 4,0 ; de préférence allant de 1,10 à 3,8.

**11.** Composition selon l'une quelconque des revendications précédentes dans laquelle le composé A2 est un composé de formule (III) :

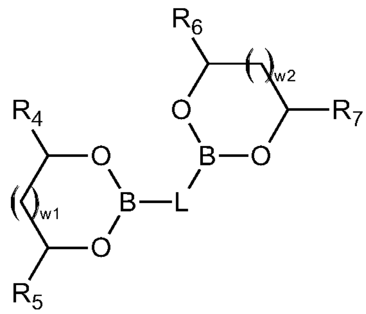

(III)

dans laquelle :

- $w_1$ et $w_2$, identiques ou différents sont des nombres entiers choisis entre 0 et 1 ;
- $R_4$, $R_5$, $R_6$ et $R_7$, identiques ou différents sont choisis parmi le groupe formé par l'hydrogène et un groupement hydrocarboné comprenant de 1 à 30 atomes de carbone, éventuellement substitué par un ou plusieurs groupements choisis parmi : un hydroxyle, un groupement -OJ, -C(O)-O-J avec J un groupement hydrocarboné comprenant de 1 à 24 atomes de carbone ;
- L est un groupement de liaison divalent et choisi parmi le groupe formé par un aryle en $C_6$-$C_{18}$, un aralkyle en $C_6$-$C_{18}$ et une chaîne hydrocarbonée en $C_2$-$C_{24}$.

**12.** Composition selon l'une quelconque des revendications 1 à 10 dans laquelle le composé A2 est un copolymère statistique résultant de la copolymérisation

- d'au moins un monomère M4 de formule (IV) :

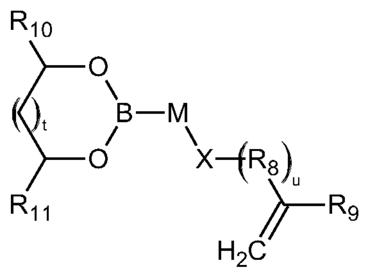

(IV)

dans laquelle :

- t est un nombre entier égal à 0 ou 1 ;

- u est un nombre entier égal à 0 ou 1 ;
- M et $R_8$ sont des groupements de liaison divalents, identiques ou différents, choisis parmi le groupe formé par un aryle en $C_6$-$C_{18}$, un aralkyle en $C_7$-$C_{24}$ et un alkyle en $C_2$-$C_{24}$, de préférence un aryle en $C_6$-$C_{18}$,
- X est une fonction choisie parmi le groupe formé par -O-C(O)-, - C(O)-O-, -C(O)-N(H)-, -N(H)-C(O)-, -S-, -N(H)- , -N(R'$_4$)- et -O- avec R'$_4$ une chaîne hydrocarbonée comprenant de 1 à 15 atomes de carbone;
- $R_9$ est choisi parmi le groupe formé par -H, -$CH_3$ et -$CH_2$-$CH_3$ ;
- $R_{10}$ et $R_{11}$ identiques ou différents sont choisis parmi le groupe formé par l'hydrogène et un groupe hydrocarboné ayant de 1 à 30 atomes de carbone, éventuellement substitué par un ou plusieurs groupements choisis parmi : un hydroxyle, un groupement -OJ, -C(O)-O-J avec J un groupement hydrocarboné comprenant de 1 à 24 atomes de carbone ;

- avec au moins un second monomère M5 de formule générale (V) :

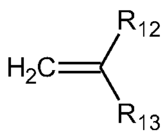

(V)

dans laquelle :

- $R_{12}$ est choisi parmi le groupe formé par -H, -$CH_3$ et-$CH_2$-$CH_3$,
- $R_{13}$ est choisi parmi le groupe formé par un aryle en $C_6$-$C_{18}$, un aryle en $C_6$-$C_{18}$ substitué par un groupement R'$_{13}$, -C(O)-O-R'$_{13}$ ; -O-R'$_{13}$, -S-R'$_{13}$ et -C(O)-N(H)-R'$_{13}$ avec R'$_{13}$ un groupe alkyle en $C_1$-$C_{30}$.

**13.** Composition selon la revendication 12, dans laquelle le copolymère A2 a un degré de polymérisation moyen en nombre allant de 50 à 1500, de préférence de 50 à 800 et indice de polydispersité (Ip) allant de 1,04 à 3,54 ; de préférence allant de 1,10 à 3,10.

**14.** Composition lubrifiante résultant du mélange d'au moins :

- d'une huile lubrifiante ; et
- d'une composition définie selon l'une quelconque des revendications 1 à 13.

**Patentansprüche**

**1.** Zusammensetzung, die aus dem Mischen von mindestens Folgendem resultiert:

- einem statistischen Polydiol-CopolymerA1,
- einer Verbindung A2 mit mindestens zwei Boronsäureesterfunktionen,
- einer exogenen Verbindung A5, die aus den Verbindungen der Formel (XI) ausgewählt ist:

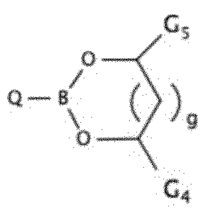

(XI)

in der:

- Q für eine Gruppe steht, die aus einer Kohlenwasserstoffgruppe mit 1 bis 30 Kohlenstoffatomen, die

gegebenenfalls durch eine oder mehrere Gruppen, die aus einem Hydroxyl oder einer -O-J- oder -C(O)O-J-Gruppe ausgewählt sind, substituiert ist, ausgewählt ist, wobei J für eine Kohlenwasserstoffgruppe mit 1 bis 24 Kohlenstoffatomen steht,
- $G_4$ und $G_5$ gleich oder verschieden sind und für Gruppen stehen, die aus einem Wasserstoffatom, einer Kohlenwasserstoffkette mit 1 bis 24 Kohlenstoffatomen, einem Hydroxyl oder einer -O-J- oder -C(O)O-J-Gruppe ausgewählt sind, wobei J für eine Kohlenwasserstoffgruppe mit 1 bis 24 Kohlenstoffatomen steht,
- g für 0 oder 1 steht,

wobei das statistische Polydiol-Copolymer A1 eine zahlenmittlere Molmasse im Bereich von 5000 bis 400.000 g/mol aufweist, der Molprozentanteil von exogener Verbindung A5, bezogen auf die Diolfunktionen des statistischen Copolymers A1, im Bereich von 0,025 bis 5000 % liegt und das Massenverhältnis zwischen dem Copolymer A1 und der Verbindung A2 (Verhältnis A1/A2) im Bereich von 0,1 bis 10 liegt.

2. Zusammensetzung nach Anspruch 1, wobei die exogene Verbindung A5 aus den Verbindungen der Formel (XIA) ausgewählt ist:

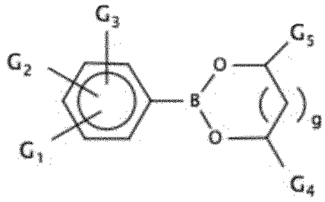

(XIA)

in der:

- $G_1$, $G_2$, $G_3$, $G_4$ und $G_5$ gleich oder verschieden sind und für Gruppen stehen, die aus einem Wasserstoffatom, einer Kohlenwasserstoffkette mit 1 bis 24 Kohlenstoffatomen, einem Hydroxyl oder einer -O-J- oder -C(O)O-J-Gruppe ausgewählt sind, wobei J für eine Kohlenwasserstoffgruppe mit 1 bis 24 Kohlenstoffatomen steht,
- g für 0 oder 1 steht.

3. Zusammensetzung nach Anspruch 1 oder nach Anspruch 2, wobei der Molprozentanteil von exogener Verbindung A5, bezogen auf die Diolfunktionen des statistischen Copolymers A1, im Bereich von 0,1 % bis 1000 %, noch weiter bevorzugt von 0,5 % bis 500 %, noch weiter bevorzugt von 1 % bis 150 %, liegt.

4. Zusammensetzung nach Anspruch 2 oder nach Anspruch 3, wobei die exogene Verbindung A5 aus den Verbindungen der Formel (XI B) ausgewählt ist:

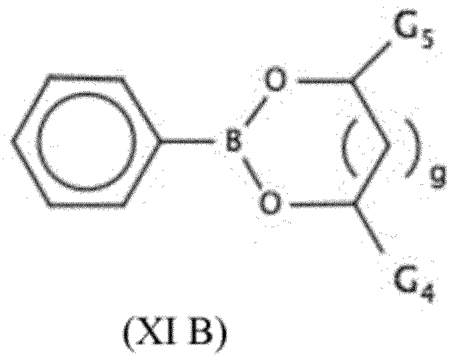

(XI B)

5. Zusammensetzung nach Anspruch 4, wobei die exogene Verbindung A5 aus den Verbindungen der Formel (XI B), in denen g = 0, G4 = H und $G_5$ für eine C1-C24-Alkylgruppe steht, ausgewählt ist.

6. Zusammensetzung nach einem der vorhergehenden Ansprüche, wobei das statistische Copolymer A1 aus der Copolymerisation von Folgendem resultiert:

- mindestens einem ersten Monomer der allgemeinen Formel (I):

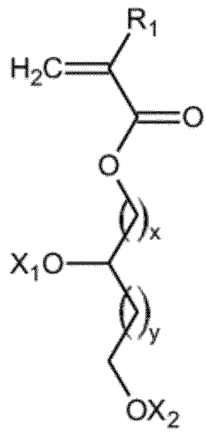

(I)

in der:

- $R_1$ aus der aus -H, $-CH_3$ und $-CH_2-CH_3$ gebildeten Gruppe ausgewählt ist;
- x eine ganze Zahl im Bereich von 1 bis 18, vorzugsweise von 2 bis 18, ist;
- y eine ganze Zahl mit einem Wert von 0 oder 1 ist;
- $X_1$ und $X_2$ gleich oder verschieden sind und aus der aus Wasserstoff, Tetrahydropyranyl, Methyloxymethyl, tert-Butyl, Benzyl, Trimethylsilyl und t-Butyldimethylsilyl gebildeten Gruppe ausgewählt sind;
oder auch
- $X_1$ und $X_2$ mit den Sauerstoffatomen eine Brücke der folgenden Formel bilden:

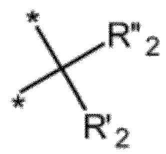

in der:

- die Sterne (*) die Bindungen an die Sauerstoffatome symbolisieren,
- $R'_2$ und $R''_2$ gleich oder verschieden sind und aus der aus Wasserstoff und einem $C_1$-$C_{11}$-Alkyl, vorzugsweise Methyl, gebildeten Gruppe ausgewählt sind;
oder auch
- $X_1$ und $X_2$ mit den Sauerstoffatomen einen Boronsäureester der folgenden Formel bilden:

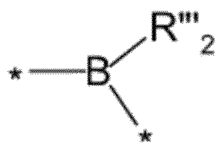

in der:

- die Sterne (*) die Bindungen an die Sauerstoffatome symbolisieren,
- $R'''_2$ aus der aus einem $C_6$-$C_{30}$-Aryl, einem $C_7$-$C_{30}$-Aralkyl und einem $C_2$-$C_{30}$-Alkyl, vorzugsweise einem $C_6$-$C_{18}$-Aryl, gebildeten Gruppe ausgewählt sind;

- mit mindestens einem zweiten Monomer M2 der allgemeinen Formel (II):

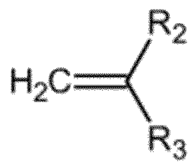

(II)

in der:

- $R_2$ aus der aus -H, $-CH_3$ und $-CH_2-CH_3$ gebildeten Gruppe ausgewählt ist;
- $R_3$ aus der aus einem $C_6-C_{18}$-Aryl, einem $C_6-C_{18}$-Aryl, das durch eine Gruppe $R'_3$ substituiert ist, -C(O)-O-$R'_3$, -O-$R'_3$, -S-$R'_3$ und -C(O)-N(H)-$R'_3$ gebildeten Gruppe ausgewählt ist, wobei $R'_3$ eine $C_1-C_{30}$-Alkylgruppe ist.

**7.** Zusammensetzung nach Anspruch 6, wobei das statistische Copolymer A1 aus der Copolymerisation von Folgendem resultiert:

- mindestens einem ersten Monomer M1 der allgemeinen Formel (I)
- mit mindestens einem zweiten Monomer M2 der allgemeinen Formel (II):

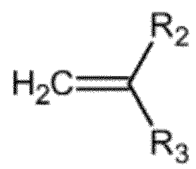

(II)

in der:

- $R_2$ aus der aus -H, $-CH_3$ und $-CH_2-CH_3$ gebildeten Gruppe ausgewählt ist;
- $R_3$ aus der aus -C(O)-O-$R'_3$, -O-$R'_3$, -S-$R'_3$ und -C(O)-N(H)-$R'_3$ bestehenden Gruppe ausgewählt ist, wobei $R'_3$ eine $C_1-C_{30}$-Alkylgruppe ist,
und

- mit mindestens einem dritten Monomer M3 der allgemeinen Formel (X):

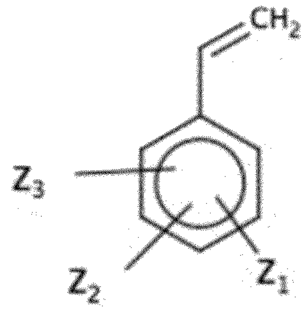

(X)

in der:

- $Z_1$, $Z_2$ und $Z_3$ gleich oder verschieden sind und für Gruppen stehen, die aus einem Wasserstoffatom, einem $C_1-C_{12}$-Alkyl oder einer -OZ'- oder -C(O)-O-Z'-Gruppe ausgewählt sind, wobei Z' ein $C_1-C_{12}$-Alkyl ist.

**8.** Zusammensetzung nach Anspruch 7, wobei sich bei dem dritten Monomer M3 um Styrol handelt.

**9.** Zusammensetzung nach einem der Ansprüche 7 und 8, wobei das statistische Copolymer A1 aus der Copolymerisation von mindestens einem Monomer M1 mit mindestens zwei Monomeren M2 mit verschiedenen $R_3$-Gruppen und mindestens einem Monomer M3 resultiert.

**10.** Zusammensetzung nach einem der vorhergehenden Ansprüche, wobei das statistische Copolymer A1 einen zahlenmittleren Polymerisationsgrad im Bereich von 40 bis 2000, vorzugsweise von 40 bis 1000, und einen Polydispersitätsindex (Ip) im Bereich von 1,05 bis 4,0, vorzugsweise im Bereich von 1,10 bis 3,8, aufweist.

**11.** Zusammensetzung nach einem der vorhergehenden Ansprüche, wobei es sich bei der Verbindung A2 um eine Verbindung der Formel (III) handelt:

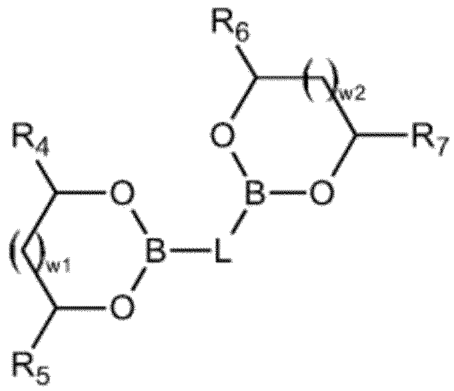

(III)

in der:

- $w_1$ und $w_2$ gleich oder verschieden sind und ganze Zahlen sind, die zwischen 0 und 1 ausgewählt sind;
- $R_4$, $R_5$, $R_6$ und $R_7$ gleich oder verschieden sind und aus der aus Wasserstoff und einer Kohlenwasserstoffgruppe mit 1 bis 30 Kohlenstoffatomen, die gegebenenfalls durch eine oder mehrere Gruppen, die aus einem Hydroxyl oder einer -O-J- oder - C(O)O-J-Gruppe ausgewählt sind, substituiert ist, bestehenden Gruppe ausgewählt sind, wobei J für eine Kohlenwasserstoffgruppe mit 1 bis 24 Kohlenstoffatomen steht;
- L eine zweiwertige Verknüpfungsgruppe ist, die aus der aus einem $C_6$-$C_{18}$-Aryl, einem $C_6$-$C_{18}$-Aralkyl und einer $C_2$-$C_{24}$-Kohlenwasserstoffkette gebildeten Gruppe ausgewählt ist.

**12.** Zusammensetzung nach einem der Ansprüche 1 bis 10, wobei es sich bei der Verbindung A2 um ein statistisches Copolymer handelt, das aus der Copolymerisation von Folgendem resultiert:

- mindestens einem Monomer M4 der Formel (IV):

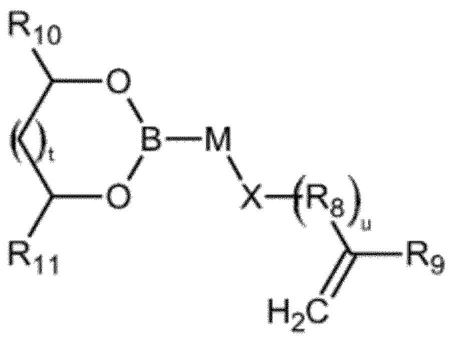

(IV)

in der:

- t eine ganze Zahl mit einem Wert von 0 oder 1 ist;
- u eine ganze Zahl mit einem Wert von 0 oder 1 ist;
- M und $R_8$ gleiche oder verschiedene zweiwertige Verknüpfungsgruppen sind, die aus der aus einem

$C_6$-$C_{18}$-Aryl, einem $C_7$-$C_{24}$-Aralkyl und einem $C_2$-$C_{24}$-Alkyl gebildeten Gruppe, vorzugsweise einem $C_6$-$C_{18}$-Aryl, ausgewählt sind,

- X eine aus der aus -O-C(O)-, -C(O)-O-, -C(O)-N(H)-, -N(H)-C(O)-, -S-, -N(H)-, -N(R'$_4$)- und -O- gebildeten Gruppe ausgewählte Funktion ist, wobei R'$_4$ eine Kohlenwasserstoffkette mit 1 bis 15 Kohlenstoffatomen ist;
- $R_9$ aus der aus -H, -$CH_3$ und -$CH_2$-$CH_3$ gebildeten Gruppe ausgewählt ist;
- $R_{10}$ und $R_{11}$ gleich oder verschieden sind und aus der aus Wasserstoff und einer Kohlenwasserstoffgruppe mit 1 bis 30 Kohlenstoffatomen, die gegebenenfalls durch eine oder mehrere Gruppen, die aus einem Hydroxyl oder einer -O-J- oder -C(O)O-J-Gruppe ausgewählt sind, substituiert ist, bestehenden Gruppe ausgewählt sind, wobei J für eine Kohlenwasserstoffgruppe mit 1 bis 24 Kohlenstoffatomen steht;

- mit mindestens einem zweiten Monomer M5 der allgemeinen Formel (V):

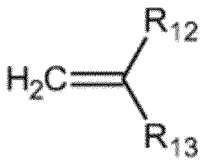

(V)

in der:

- $R_{12}$ aus der aus -H, -$CH_3$ und -$CH_2$-$CH_3$ gebildeten Gruppe ausgewählt ist;
- $R_{13}$ aus der aus einem $C_6$-$C_{18}$-Aryl und einem $C_6$-$C_{18}$-Aryl, das durch eine Gruppe R'$_{13}$ substituiert ist, -C(O)-O-R'$_{13}$, -O-R'$_{13}$, -S-R'$_{13}$ und -C(O)-N(H)-R'$_{13}$ gebildeten Gruppe ausgewählt ist, wobei R'$_{13}$ eine $C_1$-$C_{30}$-Alkylgruppe ist.

**13.** Zusammensetzung nach Anspruch 12, wobei das Copolymer A2 einen zahlenmittleren Polymerisationsgrad im Bereich von 50 bis 1500, vorzugsweise von 50 bis 800, und einen Polydispersitätsindex (Ip) im Bereich von 1,04 bis 3,54, vorzugsweise im Bereich von 1,10 bis 3,10, aufweist.

**14.** Schmiermittelzusammensetzung, die aus dem Mischen von mindestens Folgendem resultiert:

- einem Schmieröl und
- einer Zusammensetzung gemäß einem der Ansprüche 1 bis 13.

## Claims

**1.** A composition resulting from the mixing of at least

- one polydiol random copolymer A1,
- one compound A2 comprising at least two boronic ester functions,
- one exogenous compound A5 chosen from those corresponding to formula (XI):

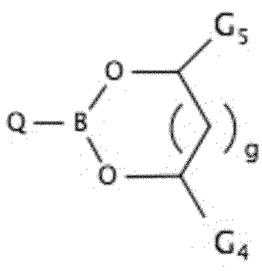

(XI)

in which:

- Q represents a group chosen from a hydrocarbon-based group comprising from 1 to 30 carbon atoms, optionally substituted with one or more groups chosen from: a hydroxyl, a group -OJ or -C(O)-O-J with J being a hydrocarbon-based group comprising from 1 to 24 carbon atoms,
- $G_4$ and $G_5$, which may be identical or different, represent groups chosen from a hydrogen atom, a hydrocarbon-based chain comprising from 1 to 24 carbon atoms, a hydroxyl and a group -OJ or -C(O)-O-J with J being a hydrocarbon-based group comprising from 1 to 24 carbon atoms,
- g represents 0 or 1,

wherein the polydiol random copolymer A1 has a number-average molecular weight from 5000 to 400,000 g/mol, the molar percentage of exogenous compound A5 relative to the diol functions of the random copolymer A1 ranges from 0.025% to 5000%, and the mass ratio between copolymer A1 and compound A2 (ratio A1/A2) ranges from 0.1 to 10.

2. The composition as claimed in claim 1, in which the exogenous compound A5 is chosen from those corresponding to formula (X!A):

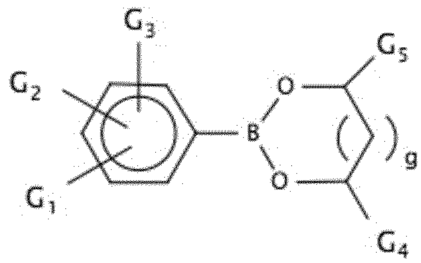

(XIA)

in which:

- $G_1$, $G_2$, $G_3$, $G_4$ and $G_5$, which may be identical or different, represent groups chosen from a hydrogen atom, a hydrocarbon-based chain comprising from 1 to 24 carbon atoms, a hydroxyl and a group -OJ or -C(O)-O-J with J being a hydrocarbon-based group comprising from 1 to 24 carbon atoms,
- g represents 0 or 1.

3. The composition as claimed in claim 1 or in claim 2, in which the molar percentage of exogenous compound A5 relative to the diol functions of the random copolymer A1 ranges from 0.1% to 1000%, more preferably from 0.5% to 500% and even more preferably from 1% to 150%.

4. The composition as claimed in claim 2 or in claim 3, in which the exogenous compound A5 is chosen from those corresponding to formula (XI B):

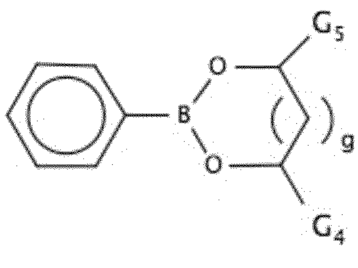

(XI B)

5. The composition as claimed in claim 4, in which the exogenous compound A5 is chosen from those corresponding to formula (X! B) with g = 0, $G_4$ = H and $G_5$ represents a C1-C24 alkyl.

6. The composition as claimed in any one of the preceding claims, in which the random copolymer A1 results from the

copolymerization:

- of at least one first monomer M1 of general formula (I):

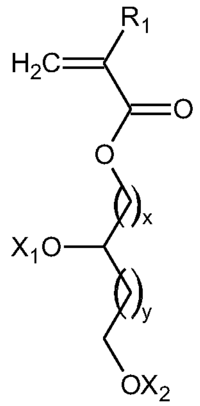

(I)

in which:

- $R_1$ is chosen from the group formed by -H, $-CH_3$ and $-CH_2-CH_3$;
- x is an integer ranging from 1 to 18 and preferably from 2 to 18;
- y is an integer equal to 0 or 1;
- $X_1$ and $X_2$, which may be identical or different, are chosen from the group formed by hydrogen, tetrahydropyranyl, methyloxymethyl, tert-butyl, benzyl, trimethylsilyl and t-butyldimethylsilyl;

or

- $X_1$ and $X_2$ form, with the oxygen atoms, a bridge having the following formula

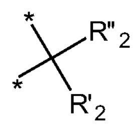

in which:

- the asterisks (*) symbolize the bonds to oxygen atoms,
- $R'_2$ and $R''_2$, which may be identical or different, are chosen from the group formed by hydrogen and a $C_1$-$C_{11}$ alkyl, preferably methyl;

or

- $X_1$ and $X_2$ form, with the oxygen atoms, a boronic ester having the following formula:

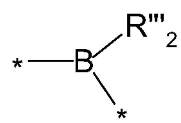

in which:

- the asterisks (*) symbolize the bonds to oxygen atoms,
- $R'''_2$ is chosen from the group formed by a $C_6$-$C_{30}$ aryl, a $C_7$-$C_{30}$ aralkyl and a $C_2$-$C_{30}$ alkyl, preferably a $C_6$-$C_{18}$ aryl;

- with at least one second monomer M2 of general formula (II):

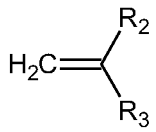

(II)

in which:

- $R_2$ is chosen from the group formed by -H, $-CH_3$ and $-CH_2-CH_3$,
- $R_3$ is chosen from the group formed by a $C_6$-$C_{18}$ aryl, a $C_6$-$C_{18}$ aryl with a group $R'_3$, $-C(O)-O-R'_3$, $-O-R'_3$, $-S-R'_3$ and $-C(O)-N(H)-R'_3$ with $R'_3$ being a $C_1$-$C_{30}$ alkyl group.

**7.** The composition as claimed in claim 6, in which the random copolymer A1 results from the copolymerization of:

- at least one first monomer M1 of general formula (I),
- with at least one second monomer M2 of general formula (II) :

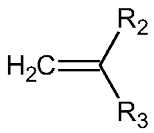

(II)

in which:
$R_2$ is chosen from the group formed by -H, $-CH_3$ and $-CH_2-CH_3$,
- $R_3$ is chosen from the group formed by $-C(O)-O-R'_3$, $-O-R'_3$, $-S-R'_3$ and $-C(O)-N(H)-R'_3$ with $R'_3$ being a $C_1$-$C_{30}$ alkyl group,

and
- with at least one third monomer M3 of general formula (X):

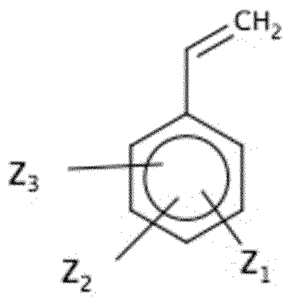

(X)

in which:
$Z_1$, $Z_2$ and $Z_3$, which may be identical or different, represent groups chosen from a hydrogen atom, a $C_1$-$C_{12}$ alkyl, and a group -OZ' or -C(O)-O-Z' with Z' being a $C_1$-$C_{12}$ alkyl.

**8.** The composition as claimed in claim 7, in which the third monomer M3 is styrene.

**9.** The composition as claimed in either one of claims 7 and 8, in which the random copolymer A1 results from the copolymerization of at least one monomer M1 with at least two monomers M2 bearing different groups $R_3$ and at least one monomer M3.

**10.** The composition as claimed in any one of the preceding claims, in which the random copolymer A1 has a number-average degree of polymerization ranging from 40 to 2000 and preferably from 40 to 1000 and a polydispersity index (Ip) ranging from 1.05 to 4.0, preferably ranging from 1.10 to 3.8.

**11.** The composition as claimed in any one of the preceding claims, in which compound A2 is a compound of formula (III):

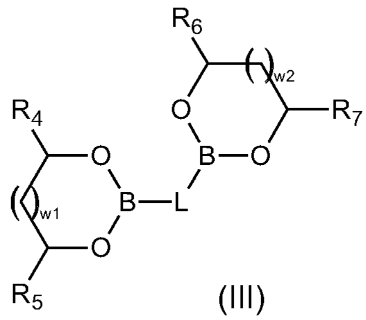

(III)

in which:

- $w_1$ and $w_2$, which may be identical or different, are integers chosen between 0 and 1;
- $R_4$, $R_5$, $R_6$ and $R_7$, which may be identical or different, are chosen from the group formed by hydrogen and a hydrocarbon-based group comprising from 1 to 30 carbon atoms, optionally substituted with one or more groups chosen from: a hydroxyl, a group
- OJ or -C(O)-O-J with J being a hydrocarbon-based group comprising from 1 to 24 carbon atoms;
- L is a divalent bonding group chosen from the group formed by a $C_6$-$C_{18}$ aryl, a $C_6$-$C_{18}$ aralkyl and a $C_2$-$C_{24}$ hydrocarbon-based chain.

**12.** The composition as claimed in any one of claims 1 to 10, in which compound A2 is a random copolymer resulting from the copolymerization

- of at least one monomer M4 of formula (IV):

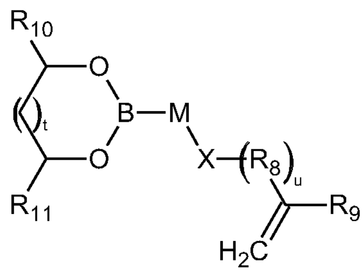

(IV)

in which:

- t is an integer equal to 0 or 1;
- u is an integer equal to 0 or 1;
- M and $R_8$ are identical or different divalent bonding groups, chosen from the group formed by $C_6$-$C_{18}$ aryl, a $C_7$-$C_{24}$ aralkyl and a $C_2$-$C_{24}$ alkyl, preferably a $C_6$-$C_{18}$ aryl,
- X is a function chosen from the group formed by -O-C(O)-, -C(O)-O-, -C(O)-N(H)-, -N(H)-C(O)-, -S-, -N(H)-, -N(R'$_4$)- and -O- with R'$_4$ being a hydrocarbon-based chain comprising from 1 to 15 carbon atoms;
- $R_9$ is chosen from the group formed by -H, $-CH_3$ and $-CH_2-CH_3$;
- $R_{10}$ and $R_{11}$, which may be identical or different, are chosen from the group formed by hydrogen and a hydrocarbon-based group having from 1 to 30 carbon atoms, optionally substituted with one or more groups chosen from: a hydroxyl, a group
- OJ or -C(O)-O-J with J being a hydrocarbon-based group comprising from 1 to 24 carbon atoms;

- with at least one second monomer M5 of general formula (V):

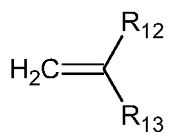

(V)

in which:

- $R_{12}$ is chosen from the group formed by -H, $-CH_3$ and $-CH_2-CH_3$;
- $R_{13}$ is chosen from the group formed by a $C_6$-$C_{18}$ aryl, a $C_6$-$C_{18}$ aryl substituted with a group $R'_{13}$, -C(O)-O-$R'_{13}$; -O-$R'_{13}$, -S-$R'_{13}$ and -C(O)-N(H)-$R'_{13}$ with $R'_{13}$ being a $C_1$-$C_{30}$ alkyl group.

**13.** The composition as claimed in claim 12, in which copolymer A2 has a number-average degree of polymerization ranging from 50 to 1500 and preferably from 50 to 800 and a polydispersity index (Ip) ranging from 1.04 to 3.54, preferably ranging from 1.10 to 3.10.

**14.** A lubricant composition resulting from the mixing of at least:

- one lubricant oil; and
- one composition as defined in any one of claims 1 to 13.

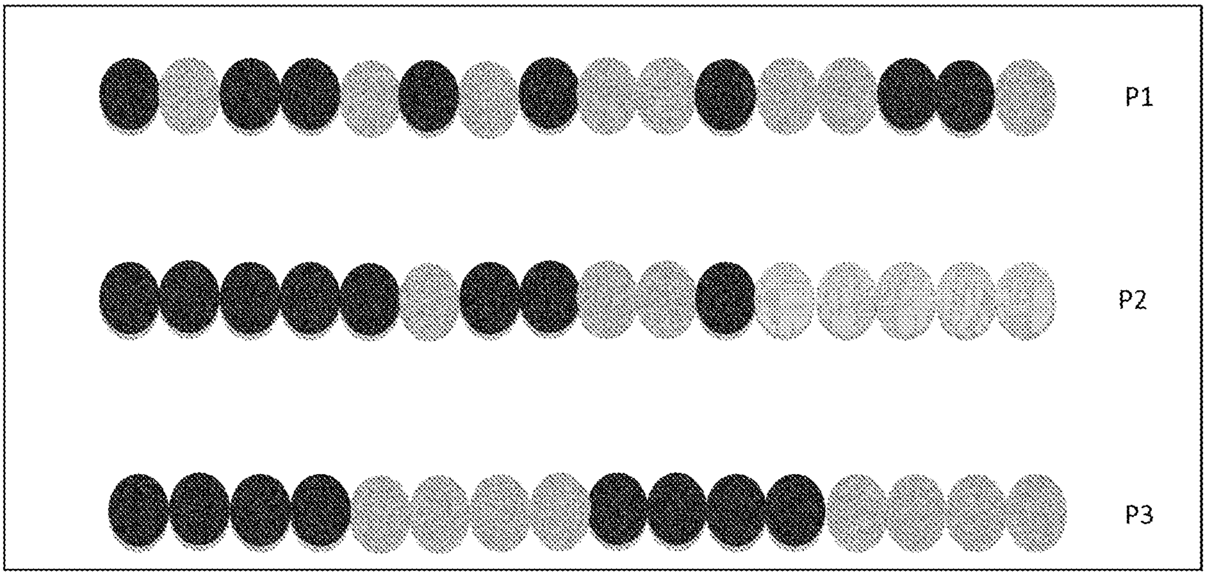


Figure 1

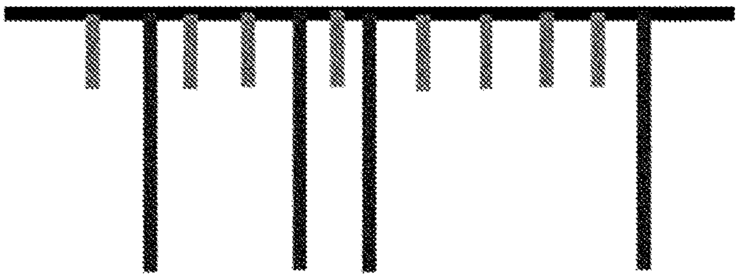

Figure 2

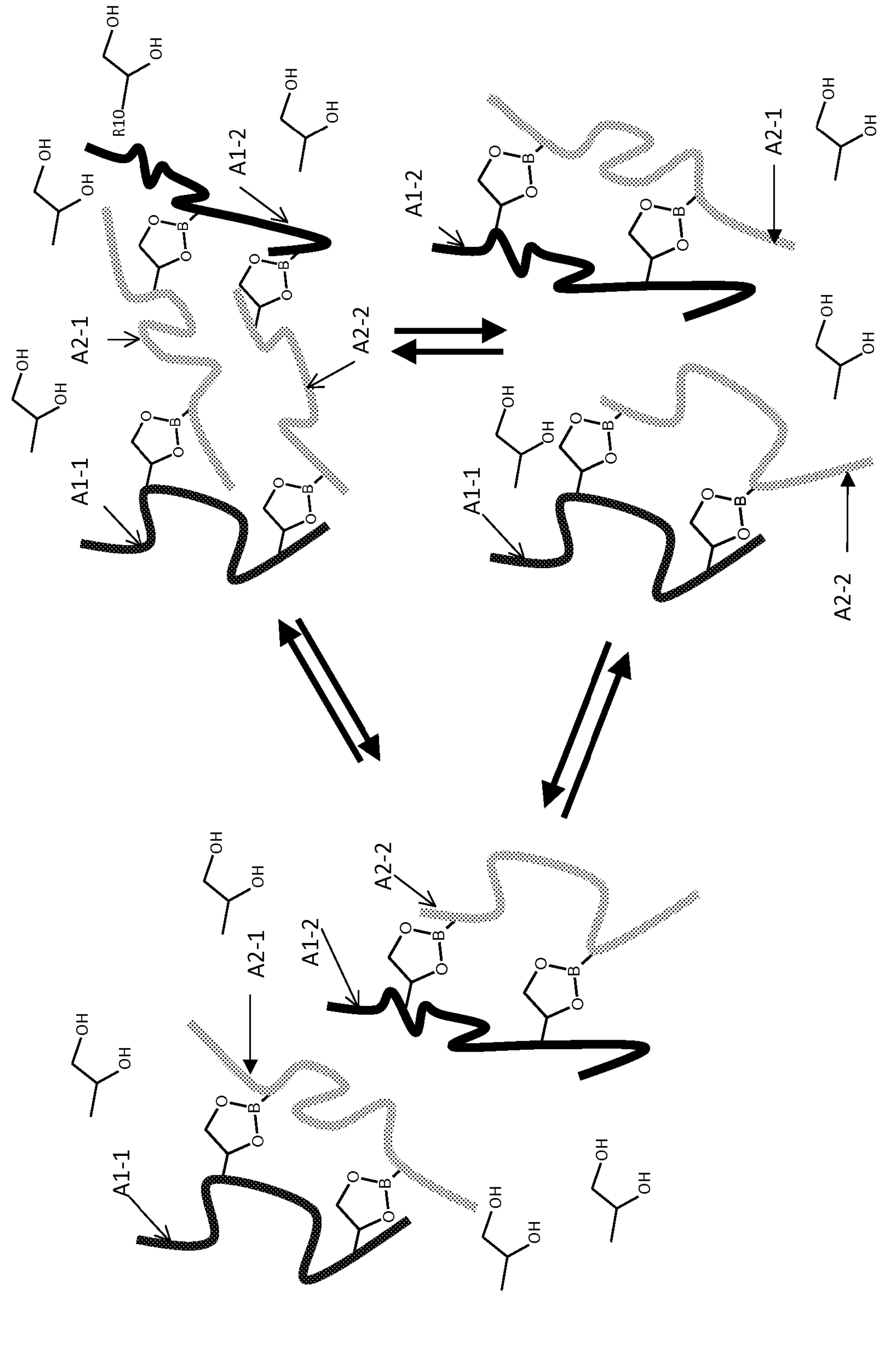

Figure 3

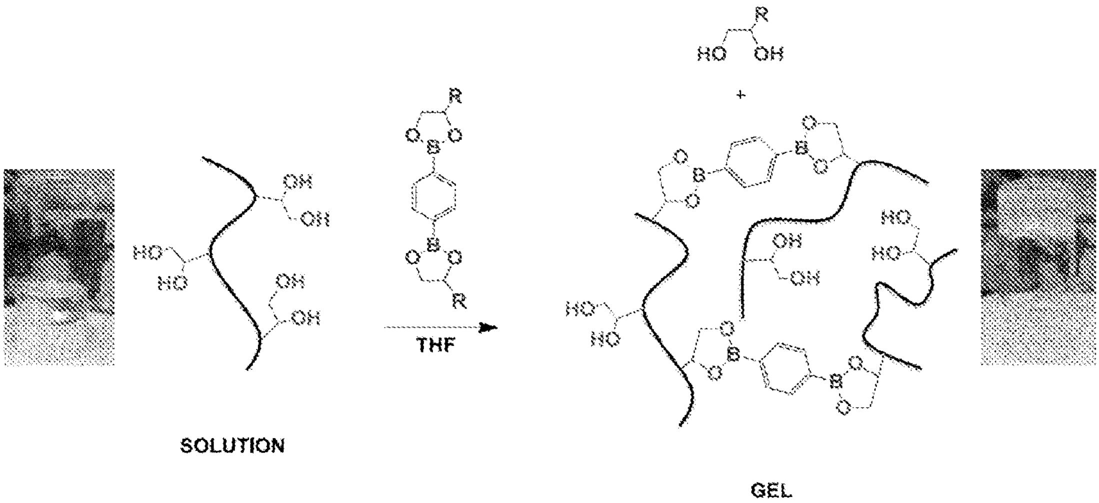


Figure 4

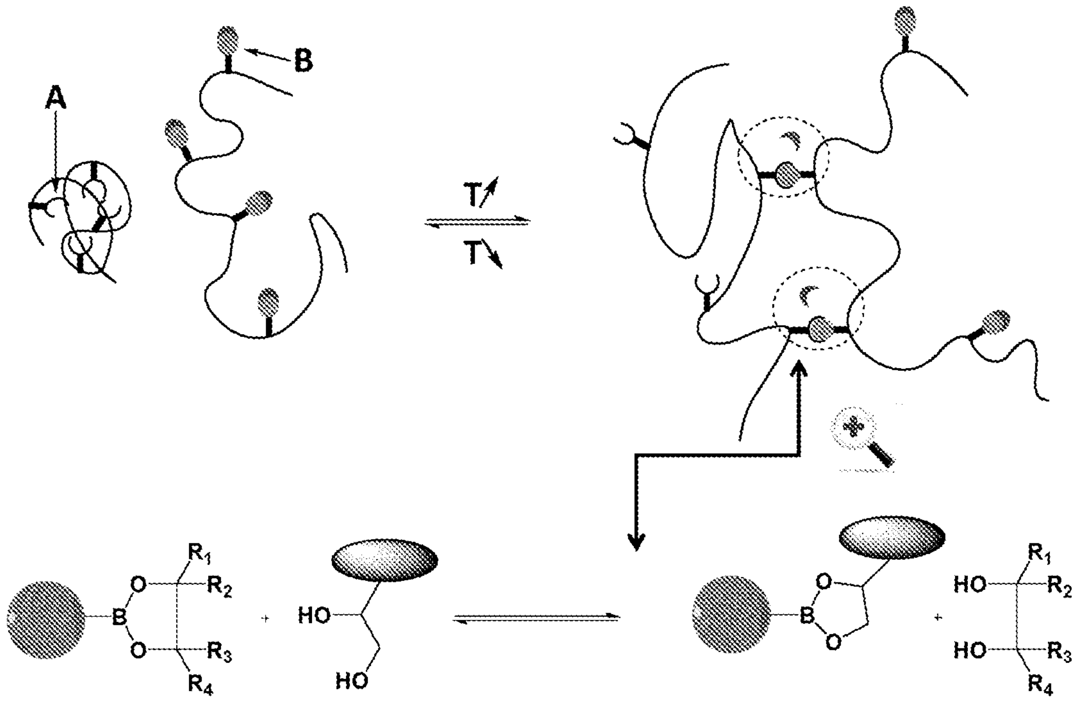


1 2 3

Figure 5

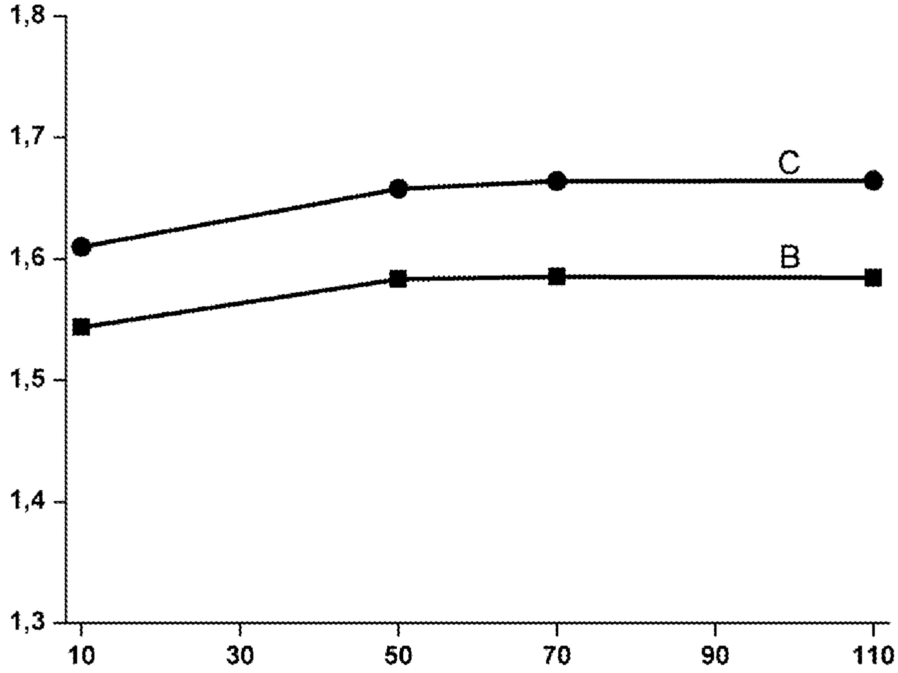


Figure 6

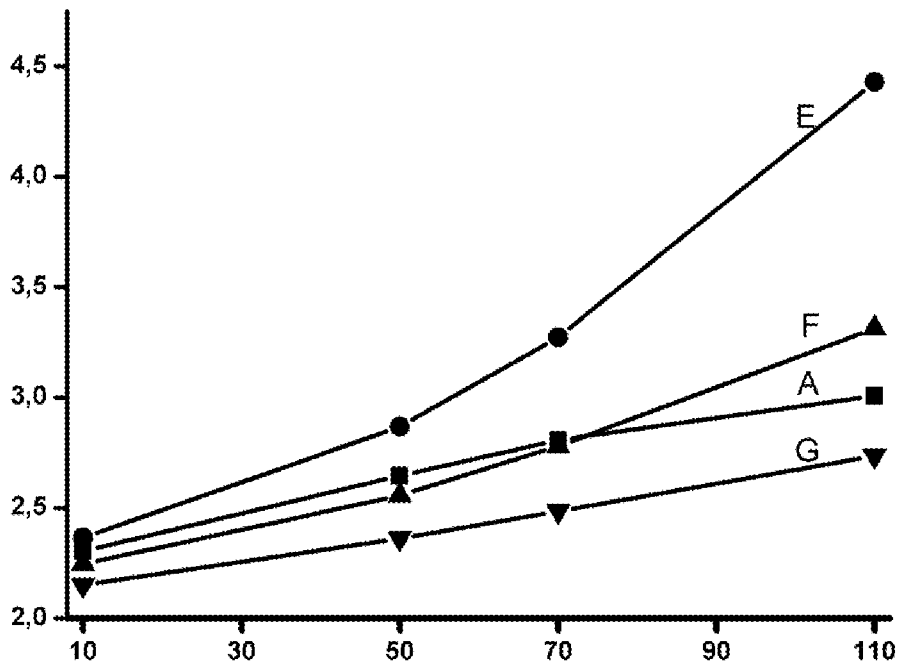


Figure 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2015110642 A
- WO 2015110643 A
- WO 2016113229 A


### Littérature non-brevet citée dans la description


- **MOAD, G. ; SOLOMON, D. H.** The Chemistry of Radical Polymerization. Elsevier Ltd, 2006, 639
- **MATYASZEWSKI, K. ; DAVIS, T. P.** Handbook of Radical Polymerization. Wiley-Interscience, 2002, 936
- **FONTANILLE, M. ; GNANOU, Y.** Chimie et physico-chimie des polymères. 2010, 546